# EUROPEAN PATENT APPLICATION

(11) **EP 1 645 286 A1**
(43) Date of publication of application: **12.04.2006**
(21) Application number: 04728057.3
(22) Date of filing: 16.04.2004
(51) Int. Cl.: A61K 45/00, A61K 31/404, A61K 31/4045, A61K 31/407, A61K 31/553, C07D 403/04, C07D 403/14, C07D 498/22, A61P 25/28, A61P 25/08, A61P 25/22, A61P 25/18, A61P 25/24, C07D 487/14

(54) **DRUG FOR NERVE REGENERATION**

(30) Priority: 18.04.2003 JP 2003114579
(71) Applicant: KYOWA HAKKO KOGYO CO., LTD., Chiyoda-ku, Tokyo 100-8185 (JP)
(72) Inventor: MORISHITA, Tsuyoshi c/o BioFrontier Laboratories, Machida-shi, Tokyo 194-8533 (JP); SAKURADA, Kazuhiro, Kanagawa 225-0016 (JP); SUZUKI, Keiko BioFrontier Laboratories,, Machida-shi, Tokyo 194-8533 (JP); IKEDA, Shun-ichi 1367-21, Minami-oya,, Tokyo 194-0031 (JP)
(74) Representative: Vossius & Partner
(86) International application number: PCT/JP2004/005503
(87) International publication number: WO 2004/091663

(57) **Abstract**

An object of the present invention is to provide a nerve regenerating drug, an agent for the promotion of neuropoiesis of a neural stem cell, a neuron obtained by culturing a neural stem cell in the presence of the agent for the promotion of neuropoiesis, and a method of the manufacture of the neuron.

In order to achieve the object, the invention provides a nerve regenerating drug comprising a substance that inhibits the activity of glycogen synthase kinase-3, as an active ingredient; an agent for the promotion of neuropoiesis of a neural stem cell comprising the substance as an active ingredient; a neuron obtained by culturing a neural stem cell in the presence of the agent for the promotion of neuropoiesis; and a method of the manufacture of the neuron.

The medical drug according to the invention is useful as a therapeutic drug for neurological diseases such as Parkinson's disease, Alzheimer's disease, Down's disease, cerebrovascular disorder, cerebral stroke, spinal cord injury, Huntington's chorea, multiple sclerosis, amyotrophic lateral sclerosis, epilepsy, anxiety disorder, schizophrenia, depression and manic depressive psychosis.

## Description

### TECHNICAL FIELD

The present invention relates to a nerve regenerating drug comprising a substance that inhibits the activity of glycogen synthase kinase-3 (hereinafter, abbreviated as GSK-3), as an active ingredient; an agent for the promotion of neuropoiesis comprising a substance that inhibits the activity of GSK-3, as an active ingredient; a neuron obtained by culturing a neural stem cell in the presence of the agent for the promotion of neuropoiesis; and a method of the manufacture of the neuron.

### BACKGROUND ART

Neurological diseases collectively refer to neurodegenerative diseases in which a brain or a peripheral neuron is injured due to a genetic factor, an environmental factor, an aging factor or the like; depression and manic depressive psychosis not accompanied by degeneration of a nerve; and the like. Specific examples of the neurodegenerative disease include Parkinson's disease, Alzheimer's disease, polyglutamic acid disease, amyotrophic lateral sclerosis, polyneuropathy, spinal cord injury, cerebrovascular disorder and the like. Although general therapeutic methods of these neurodegenerative diseases are therapies in which a neurotransmitter which was lost due to the injury of a neuron is supplied, diseases of which symptoms may be ameliorated by the therapy are limited to Parkinson's disease, Alzheimer's disease and the like. Further, progress of the nerve cell death can not be interrupted by the supplementary therapy of a neurotransmitter.

Although regenerative medical treatments for regenerating a central nervous system have been studied as a therapeutic method for positively recovering the functions of a dopaminergic neuron which was lost in Parkinson's disease, they involve a variety of problems, because they are methods in which the brain of an aborted fetus is used. Therefore, they have not been applied for general utilization.

Moreover, a therapeutic method in which neural stem cells obtained from the brain of an aborted fetus or ES cells obtained from a human fertilized egg are subjected to large scale culture followed by use in transplantation after converting into an intended neuron has also been studied, however, a technique for allowing accurate differentiation into the intended neuron has not been established, and is also involved problems resulting from the method in which neural stem cells derived from a fetus or human ES cells are used. Thus, clinical applications have not been advanced.

On the other hand, a therapeutic method of neurodegenerative diseases in which neural stem cells endogenously existing in the brain of a patient are stimulated by an agent or the like to induce regeneration has been studied on the basis of reports on separation of neural stem cells from an adult brain, suggesting the occurrence of neogenesis of a neuron also in a human adult brain during a lifetime.

Promotion of neuropoiesis in hippocampus or olfactory bulb by an intracranial administration of a cytokine or by a disease model has been reported such as: insulin like growth factor-1 [J. Neuroscience, 20, 2896-2903 (2000)], a fibroblast growthfactor-2 [Pro. Nat. Acad. Sci. USA, 98, 5874-5879 (2001)], a stem cell factor [J. Clin. Invest., 110, 311-319 (2002)], erythropoietin [J. Neuroscience, 21, 9733-9743 (2001)], whole brain ischaemia [J. Neuroscience, 18, 7768-7778 (1998)] and stimulus epilepticus [J. Neuroscience, 22, 3174-3188 (2002)]. Further, it is also reported that neogenesis of a dopaminergic neuron occurs in corpus striatum by intracranially administering a tumor growth factor-α to ameliorate the symptom of Parkinson's disease [Pro. Nat. Acad. Sci. USA, 97, 14686-14691 (2000)]. Additionally, it is also reported that 40% of CA1 pyramidal cells lost due to ischemic injury in hippocampus are completely recovered by intracranially administering a fibroblast growth factor-2 and an epidermal growth factor on day 2 to day 5 post the ischaemia [Cell, 110, 429-441 (2002) ] .

However, any one of the aforementioned methods requires intracerebral administration of a proteinous factor, therefore, applications to general medical treatments have been difficult.

Examples of reported low molecular weight compound that can be peripherally administered and can result in neuropoiesis include antidepressants such as monoamine oxidase inhibitors, serotonin specific transporter inhibitors and phosphodiesterase IV inhibitors [Neuropsychopharmacology, 25, 836-844(2001)]. As mechanisms which may involve intracerebral induction of nerve regeneration by these agents, it has been believed that the agent promotes neuropoiesis around a serotonergic neuron through directly or indirectly acting on a serotonin receptor signal of the serotonergic neuron to produce a neurotrophic factor. Therefore, it is believed that these agents can not be utilized as a nerve regenerating drug for almost all neurological diseases that are not related to degeneration of the serotonergic neuron.

Further, it is reported that lithium that is a mood stabilizing drug protects a neogenetic neuron, which is newly and constitutively generated in hippocampus, from death of the neuron through inducing the expression of a cell death suppressive gene bcl-2, thereby apparently increasing neuropoiesis in hippocampus, [J. Neurochemistry, 75, 1729-1734 (2000)]. In addition, lithium is also reported to induce the expression of a neurotrophic factor BDNF [Neuropharmacology, 43, 1173-1179 (2002)]. However, it has not been reported that lithium promotes neuropoiesis by directly acting on a neural stem cell and promoting neuronal differentiation, and also, the activity of lithium to promote neuropoiesis in a region other than hippocampus has not been reported. Furthermore, it has not been known as to why lithium has a therapeutic effect on depression and manic depressive psychosis not accompanied by degeneration of a nerve.

In connection with Alzheimer' s disease, a hypothesis was proposed stating that glycogen synthase kinase-3 (hereinafter, abbreviated as GSK-3) excessively phosphorylates a tau protein that is a microtubule-associated protein thereby resulting in neurofibrillary alteration to induce nerve cell death [Trends in Molecular Medicine, 8, 126-132 (2002)]. Further, a substance that inhibits the activity of GSK-3 was reported to have an activity to protect mature nerve cells *in vitro* [J. Neurochemistry, 77, 94-102 (2001)]. On the basis of this report, it has been believed that the substance that inhibits the activity of GSK-3 can be used as a therapeutic drug for various neurodegenerative diseases in addition to Alzheimer' s disease (Pamphlet of International Patent Publication No. 00/38675), however, it has not been known whether or not a neurodegenerative disease can be actually treated by protecting a mature neuron, and that there exists a neuropoiesis promoting action of the substance that inhibits the activity of GSK-3.

### DISCLOSURE OF THE INVENTION

An object of the present invention is to provide a nerve regenerating drug comprising a substance that inhibits the activity of GSK-3, as an active ingredient; an agent for the promotion of neuropoiesis of a neural stem cell comprising the substance as an active ingredient; a neuron obtained by culturing a neural stem cell in the presence of the agent for the promotion of neuropoiesis; and a method of the manufacture of the neuron.

The present invention relates to the following items (1) to (41).
(1) A nerve regenerating drug which comprises a substance that inhibits the activity of glycogen synthase kinase-3 (hereinafter, abbreviated as GSK-3), as an active ingredient.
(2) The medical drug according to the above item (1) wherein the nerve regenerating drug is a therapeutic drug for a neurological disease.
(3) The medical drug according to the above item (2) wherein the neurological disease is selected from the group consisting of Parkinson' s disease, Alzheimer' s disease, Down' s disease, cerebrovascular disorder, cerebral stroke, spinal cord injury, Huntington's chorea, multiple sclerosis, amyotrophic lateral sclerosis, epilepsy, anxiety disorder, schizophrenia, depression and manic depressive psychosis.
(4) The medical drug according to any one of the above items (1) to (3) wherein the substance that inhibits the activity of GSK-3 is lithium or a pharmacologically acceptable salt thereof.
(5) The medical drug according to any one of the above items (1) to (3) wherein the substance that inhibits the activity of GSK-3 is a bisindolylmaleimide derivative, a 3-aryl-4-indolylmaleimide derivative, an indolocarbazole derivative, an indolo[3,2-d][1]benzazepin-6(5H)-one derivative or an indirubin derivative, or a pharmacologically acceptable salt thereof.
(6) The medical drug according to any one of the above items (1) to (3) wherein the substance that inhibits the activity of GSK-3 is a compound represented by the formula (I): 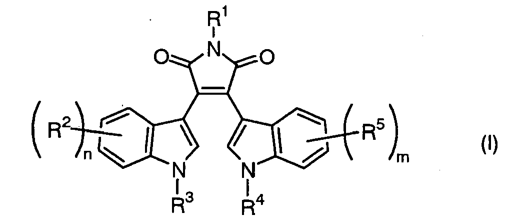 [wherein n and m may be the same or different, and represent an integer of 1 to 3; R¹, R³ and R⁴ may be the same or different, and represent hydrogen, substituted or unsubstituted lower alkyl, substituted or unsubstituted lower alkenyl, -COR⁶ (wherein R⁶ represents hydrogen, substituted or unsubstituted lower alkyl, substituted or unsubstituted lower alkenyl, substituted or unsubstituted aryl or substituted or unsubstituted cycloalkyl), -COOR⁷ (wherein R⁷ represents hydrogen, substituted or unsubstituted lower alkyl, substituted or unsubstituted aryl or substituted or unsubstituted cycloalkyl) or -OR⁸ (wherein R⁸ represents hydrogen, substituted or unsubstituted lower alkyl, substituted or unsubstituted aryl or substituted or unsubstituted cycloalkyl) ; R² and R⁵ may be the same or different, and represent hydrogen, substituted or unsubstituted lower alkyl, a substituted or unsubstituted lower alkenyl, substituted or unsubstituted lower alkoxy, substituted or unsubstituted lower alkoxycarbonyl, substituted or unsubstituted aryl, carboxy, halogen, hydroxy, nitro, amino, or mono- or di-lower alkylamino; when n and m are 2 or 3, each of R² and R⁵ may be the same or different], a compound represented by the formula (II): 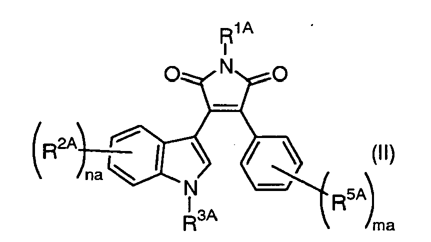 (wherein na, ma, R^{1A}, R^{2A}, R^{3A} and R^{5A} are as defined for the aforementioned n, m, R¹, R², R³ and R⁵, respectively) or a compound represented by the formula (III): 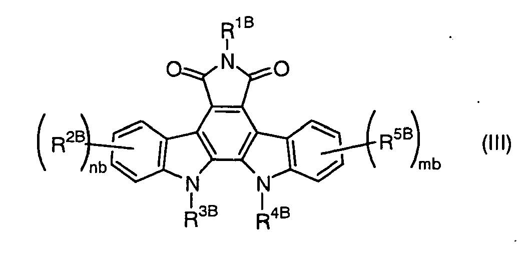 [wherein nb, mb, R^{1B}, R^{2B} and R^{5B} are as defined for the aforementioned n, m, R¹, R² and R⁵, respectively; R^{3B} and R^{4B} maybe the same or different, and represent hydrogen, substituted or unsubstituted lower alkyl, substituted or unsubstituted lower alkenyl, -COR⁶ (wherein R⁶ is as defined above), -COOR⁷ (wherein R⁷ is as defined above) or -OR⁸ (wherein R⁸ is as defined above), or R^{3B} and R^{4B} together form 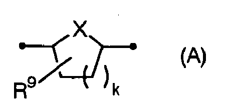 (wherein k represents 1 or 2; X represents CH₂, NH, an oxygen atom or a sulfur atom; R⁹ represents hydroxy, carboxy, carbamoyl or lower alkoxycarbonyl)], or a pharmacologically acceptable salt thereof.
(7) The medical drug according to any one of the above items (1) to (3) wherein the substance that inhibits the activity of GSK-3 is a compound represented by the formula (Ia): 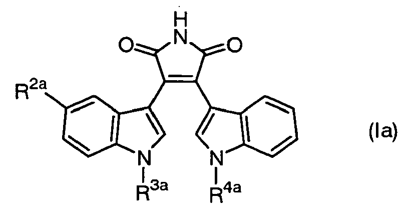 (wherein R^{2a} represents hydrogen, lower alkoxy, lower alkoxycarbonyl, aryl or nitro; R^{3a} and R^{4a} may be the same or different, and represent substituted or unsubstituted lower alkyl), or a pharmacologically acceptable salt thereof.
(8) The medical drug according to any one of the above items (1) to (3) wherein the substance that inhibits the activity of GSK-3 is a compound represented by the formula (IIa): 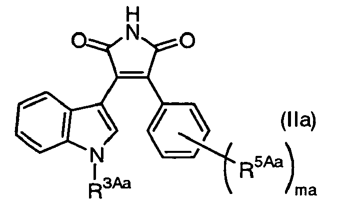 (wherein ma is as defined above; R^{3Aa} represents substituted or unsubstituted lower alkyl; R^{5Aa} represents halogen), or a pharmacologically acceptable salt thereof.
(9) The medical drug according to any one of the above items (1) to (3) wherein the substance that inhibits the activity of GSK-3 is a compound represented by the formula (IIIa): 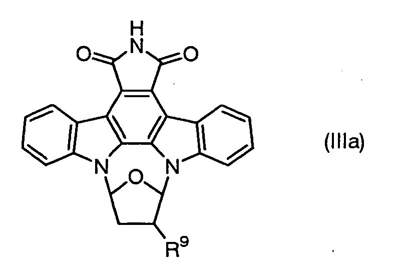 (wherein R⁹ is as defined above), or a pharmacologically acceptable salt thereof.
(10) The medical drug according to any one of the above items (1) to (3) wherein the substance that inhibits the activity of GSK-3 is a compound selected from the group consisting of 3,4-bis(1-methylindole-3-yl)-1H-pyrrole-2,5-dione, 3-(1-methylindole-3-yl)-4-(1-propylindole-3-yl)-1H-pyrrole -2,5-dione, 3-[1-(3-cyanopropyl)indole-3-yl]-4-(1-methylindole-3-yl)-1H-pyrrole-2,5-dione, 3-[1-(3-aminopropyl)-indole-3-yl]-4-(1-methylindole-3-yl)-1H-pyrrole-2,5-dione, 3-[1-(3-carboxypropyl)indole-3-yl]-4-(1-methylindole-3-yl)-1H-pyrrole-2,5-dione, 3-[1-(3-carbamoylpropyl)indole-3-yl]-4-(1-methylindole-3-yl)-1H-pyrrole-2,5 -dione, 3-[1-(3-aminopropyl)indole-3-yl]-4-(1-methyl-5-propyloxyin dole-3-yl)-1H-pyrrole-2,5-dione, 3-[1-(3-hydroxypropyl)indole-3-yl]-4-(1-methyl-5-phenylindole-3-yl)-1H-py rrole-2,5-dione, 3-[1-(3-aminopropyl)indole-3-yl]-4-(1-methyl-5-phenylindol e-3-yl)-1H-pyrrole-2,5-dione, 3-[1-(3-hydroxypropyl)indole-3-yl]-4-(1-methyl-5-methoxycarbonylindole-3-yl)-1H-pyrrole-2,5-dione, 3-[1-(3-hydroxypropyl)indole-3-yl]-4-(1-methyl-5-nitroindole-3-yl)-1H-pyr role-2,5-dione, 3-(1-methylindole-3-yl)-4-[1-(3-hydroxypropyl)-5-nitroindole-3-yl]-1H-pyrrole-2,5-dione, 3-(2-chlorophenyl)-4-(1-methylindole-3-yl)-1H-pyrrole-2,5-dione, 3-(2,4-dichlorophenyl)-4-(1-methylindole-3-yl)-1H-pyrrole-2,5-dione, 3-(2-chlorophenyl)-4-[1-(3-hydroxypropyl)indole-3-yl]-1H-pyrrole-2,5-dione, 4-[1-(3-aminopropyl)indole-3-yl]-3-(2-chlorophenyl)-1H-pyrrole-2,5-dion e and 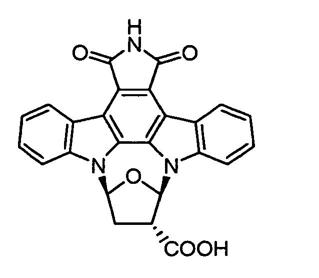 , or a pharmacologically acceptable salt thereof.
(11) The medical drug according to any one of the above items (1) to (3) wherein the substance that inhibits GSK-3 is a compound represented by the formula (IV): 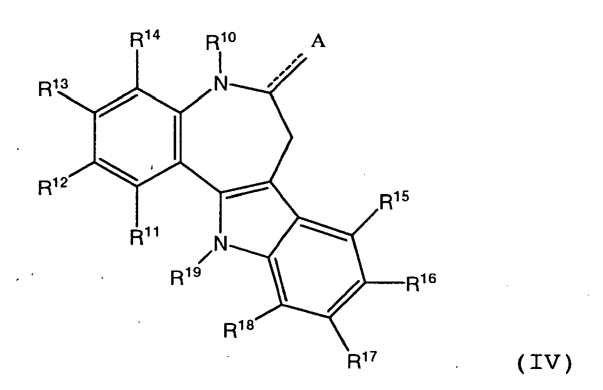 [wherein A is oxygen or sulfur coupled to the right by a single or double bond; R¹⁰ is selected from the group consisting of hydrogen, aryl, lower aliphatic substituents, particularly alkyl and lower alkyl ester; R¹¹-R¹⁴ are independently selected from the group consisting of alkoxy, amino, acyl, aliphatic substituents, particularly alkyl, alkenyl and alkinyl substituents, aliphatic alcohols, particularly alkyl alcohols, aliphatic nitriles, particularly alkyl nitriles, cyano, nitro, carboxyl, halogen, hydrogen, hydroxyl, imino and α,β-unsaturatedketones; R¹⁵-R¹⁸ are independently selected from the group consisting of aliphatic substituents, particularly alkyl, alkenyl and alkinyl substituents, particularly lower aliphatic substituents, alipahatic alcohols, particularly alkyl alcohols, alkoxy, acyl, cyano, nitro, epoxy, haloalkyl groups, halogen, hydrogen and hydroxyl; R¹⁹ is selected from the group consisting of aliphatic groups, particularly lower alkyl groups, aliphatic alcohols, particularly alkyl alcohols, carboxylic acids and hydrogen], or a pharmacologically acceptable salt thereof.
(12) The medical drug according to any one of the above items (1) to (3) wherein the substance that inhibits GSK-3 is a compound selected from the group consisting of 7,12-dihydro-indolo[3,2-d][1]benzazepin-6(5H)-one, 2-bromo-7,12-dihydro-indolo[3,2-d][1]benzazepin-6(5H)-one, 9-bromo-7,12-dihydro-indolo[3,2-d][1]benzazepin-6(5H)-one, 9-chloro-7,12-dihydro-indolo[3,2-d][1]benzazepin-6(5H)-one, 11-chloro-7,12-dihydro-indolo[3,2-d][1]benzazepin-6(5H)-on e, 10-bromo-7,12-dihydro-indolo[3,2-d][1]benzazepin-6(5H)-one, 8-bromo-6,11-dihydro-thieno[3',2':2,3]azepino-[4,5-b]indol-5(4H)-one, 9-bromo-7,12-dihydro-4-methoxy-indolo[3,2-d][1]benzazepin-6(5H)-one, 9-bromo-7,12-dihydro-4-hydroxy-indolo[3,2-d][1]benzazepin-6(5H)-one, 7,12-dihydro-4-methoxy-indolo[3,2-d][1]benzazepin-6(5H)-on e, 9-bromo-7,12-dihydro-2,3-dimethoxy-indolo[3,2-d][1]benzaze pin-6(5H)-one, 9-bromo-7,12-dihydro-2,3-di-hydroxy-indolo[3,2-d][1]benzazepin-6(5H)-one, 7,12-dihydro-2,3-dimethoxy-indolo[3,2-d][1]benzazepin-6(5H)-one, 7,12-dihydro-9-trifluormethyl-indolo[3,2-d][1]benzazepin-6 (5H)-one, 7,12-dihydro-2,3-dimethoxy-9-trifluoromethyl-indolo[3,2-d] [1]benzazepin-6(5H)-one, 2-bromo-7,12-dihydro-9-trifluoromethyl-indolo[3,2-d][1]benzazepin-6(5H)-o ne, 9-bromo-7,12-dihydro-indolo[3,2d][1]benzazepin-6(5H)-thion e,9-bromo-5,12-bis-(t-butyloxycarbonyl)-7,12-dihydro-indolo[ 3,2-d][1]benzazepin-6(5H)-one, 9-bromo-12-(t-butyloxycarbonyl)-7,12-dihydro-indolo[3,2-d] [1]benzazepin-6(5H)-one, 9-bromo-5,7-bis-(t-butyloxycarbonyl)-7,12-dihydro-indolo[3 ,2-d][1]benzazepin-6(5H)-one, 9-bromo-5,7,12-tri-(t-butyloxycarbonyl)-7,12-dihydro-indol o[3,2-d][1]benzazepin-6(5H)-one, 9-bromo-7,12-dihydro-5-methyloxycarbonylmethyl-indolo[3,2-d][1]benzazepin-6(5H)-one, 9-bromo-7,12-dihydro-12-methyloxycarbonylmethyl-indolo[3,2 -d][1]benzazepin-6(5H)-one, 9-bromo-7,12-dihydro-12-(2-hydroxyethyl)-indolo[3,2-d][1]benzazepin-6-(5H)-one, 2,9-dibromo-7,12-dihydro-indolo[3,2-d][1]benzazepin-6(5H)-one, 8,10-dichloro-7,12-dihydro-indolo-[3,2d][1]benzazepin-6(5H)-one, 9-cyano-7,12-dihydro-indolo[3,2-d][1]benzazepin-6(5H)-one, 9-bromo-7,12-dihydro-5-methyl-indolo[3,2-d][1]benzazepin-6 (5H)-one, 5-benzyl-9-bromo-7,12-dihydro-5-methyl-indolo[3,2-d][1]-benzazepin-6(5H)-one, 9-bromo-7,12-dihydro-12-methyl-indolo-[3,2-d][1]benzazepin-6(5H)-one, 9-bromo-12-ethyl-7,12-dihydro-indolo[3,2-d][1]benzazepin-6 (5H)-one, 9-bromo-7,12-dihydro-12-(2-propenyl)-indolo[3,2-d][1]benzazepin-6(5H)-one, 7,12-dihydro-9-methyl-indolo[3,2-d][1]-benzazepin-6(5H)-one, 7,12-dihydro-9-methoxy-indolo[3,2-d][1]benzazepin-6(5H)-on e, 9-fluoro-7,12-dihydro-12-(2-propenyl)-indolo[3,2-d][1]benz azepin-6(5H)-one, 11-bromo-7,12-dihydro-indolo[3,2d][1]benzazepin-6(5H)-one, 9-bromo-7,12-dihydro-2-(methyliminoamine)-indolo[3,2-d][1] benzazepin-6(5H)-one, 9-bromo-7,12-dihydro-2-(carboxylic acid)-indolo[3,2-d][1]benzazepin-6(5H)-one, 9-bromo-7,12-dihydro-10-hydroxy-indolo[3,2-d][1]benzazepin -6(5H)-one, 9-bromo-7,12-dihydro-11-hydroxymethyl-indolo[3,2-d][1]-benzazepin-6(5H)-one, 7,12-dihydro-4-hydroxy-indolo[3,2-d][1]benzazepin-6(5H)-on e, 7,12-dihydro-2,3-dihydroxy-indolo[3,2-d][1]benzazepin-6(5H )-one, 2,3-dimethoxy-9-nitro-7,12-dihydro-indolo.[3,2d][.1]benzazep in-6(5H)-one, 9-cyano-7,12-dihydro-indolo[3,2-d][1]benzazepin-6(5H)-one; 2,3-dimethoxy-9-cyano-7,12-dihydro-indolo[3,2-d][1]benzazepin-6(5H)-one, 9-nitro-7,12-dihydro-indolo[3,2-d][1]benzazepin-6(5H)-one, 3-(6-oxo-9-trifluoromethyl-5,6,7,12-tetrahydro-indolo[3,2-d][1]benzazepin-2-yl)-propionitrile, 2-bromo-9-nitro-7,12-dihydro-indolo[3,2-d][1]benzazepin-6( 5H)-one, 3-(6-oxo-9-trifluoromethyl-5,6,7,12-tetrahydro-indolo[3,2-d][1]benzazepin-2-yl)acrylonitrile, 2-(3-hydroxy-1-propinyl)-9-trifluoromethyl-7,12-dihydro-in dolo[3,2-d][1]benzazepin-6(5H)-one, 2-iodo-9-bromo-7,12-dihydro-indolo[3,2-d][1]benzazepin-6(5 H)-one, 2-(3-oxo-1-butenyl)-9-trifluoromethyl-7,12-tetrahydro-indo lo[3,2-d][1]benzazepin-6(5H)-one, 8-chloro-6,11-dihydro-thieno[3',2':2,3]azepino[4,5-b]indol -5(4H)-one, 2-iodo-9-trifluoromethyl-7,12-dihydro-indolo[3,2-d][1]-benzazepin-6(5H)-one, 7,12-dihydro-pyrido[3',2':4,5]-pyrrolo[3,2-d] [1]benzazepin-6(5H)-one, 11-methyl-7,12-dihydro-indolo[3,2-d][1]-benzazepin-6(5H)-o ne, 2-[2-(1-hydroxycyclohexyl)-ethinyl]-9-trifluoromethyl-7,12-dihydro-indolo-[3,2-d][1]benzazepin-6(5H)-one, 2-cyano-7,12-dihydro-indolo[3,2-d][1]benzazepin-6(5H)-one, 2-iodo-7,12-dihydro-indolo[3,2-d][1]benzazepin-6(5H)-one, 11-ethyl-7,12-dihydro-indolo[3,2-d][1]benzazepin-6(5H)-one, 8-methyl-6,11-dihydro-thieno[3',2':2,3]azepino[4,5-b]indol -5(4H)-one and 3-(6-oxo-9-trifluoromethyl-5,6,7,12-tetrahydro-indolo[3,2-d][1]benzazepin-2-yl)acrylic acid methyl ester, or a pharmacologically acceptable salt thereof.
(13) The medical drug according to any one of the above items (1) to (3) wherein the substance that inhibits GSK-3 is a compound selected from the group consisting of 9-cyano-7,12-dihydro-indolo[3,2-d][1]benzazepin-6(5H)-one, 9-bromo-7,12-dihydro-2,3-dimethoxy-indolo[3,2-d][1]benzazepin-6(5H)-one, 2-bromo-7,12-dihydro-9-trifluoromethyl-indolo[3,2-d][1]ben zazepin-6(5H)-one, 7,12-dihydro-2,3-dimethoxy-9-trifluoromethyl-indolo[3,2-d] [1]benzazepin-6(5H)-one, 2,9-dibromo-7,12-dihydro-indolo[3,2-d][1]-benzazepin-6(5H)-one, 7,12-dihydro-9-trifluoromethyl-indolo-[3,2-d][1]benzazepin-6(5H)-one, 9-chloro-7,12-dihydro-indolo[3,2-d][1]benzazepin-6(5H)-one, 8-bromo-6,11-dihydro-thieno[3',2':2,3]azepino[4,5-b]indole -5(4H)-one, 7,12-dihydro-9-methoxy-indolo[3,2-d][1]benzazepin-6(5H)-on e, 10-bromo-7,12-dihydro-indolo[3,2-d][1]benzazepin-6(5H)-one, 11-bromo-7,12-dihydro-indolo[3,2-d][1]-benzazepin-6(5H)-one, 11-chloro-7,12-dihydro-indolo[3,2-d]-[1]benzazepin-6(5H)-one, 9-fluoro-7,12-dihydro-indolo-[3,2-d][1]benzazepin-6(5H)-one, 9-methyl-7,12-dihydro-indolo[3,2-d][1]benzazepin-6(5H)-one, 9-bromo-7,12-dihydro-indolo[3,2-d][1]benzazepin-6(5H)-thione, 8,10-di-chloro-7,12-dihydro-indolo[3,2-d][1]benzazepin-6(5H)-one, 9-bromo-7,12-dihydro-12-(2-hydroxyethyl)-indolo[3,2-d][1]-benzazepin-6(5H)-one, 9-bromo-7,12-dihydro-2,3-di-hydroxy-indolo[3,2-d][1]benzazepin-6(5H)-one, 2-bromo-7,12-dihydro-indolo[3,2-d][1]benzazepin-6(5H)-one, 7,12-dihydro-2,3-dimethoxy-indolo[3,2-d][1]benzazepin-6(5H )-one, 9-bromo-7,12-dihydro-12-methyl-indolo[3,2-d][1]benzazepin-6(5H)-one, 9-bromo-7,12-dihydro-5-methyloxycarbonylmethyl-indolo[3,2-d][1]benzazepin-6(5H)-one and 7,12-dihydro-indolo[3,2-d][1]benzazepin-6(5H)-one, or a pharmacologically acceptable salt thereof.
(14) The medical drug according to any one of the above items (1) to (3) wherein the substance that inhibits GSK-3 is a compound selected from the group consisting of 9-cyano-7,12-dihydro-indolo[3,2-d][1]benzazepin-6(5H)-one, 9-bromo-7,12-dihydro-2,3-dimethoxy-indolo[3,2-d][1]benzazepin-6(5H)-one, 2-bromo-7,12-dihydro-9-trifluoromethyl-indolo[3,2-d][1]ben zazepin-6(5H)-one, 7,12-dihydro-2,3-dimethoxy-9-trifluoromethyl-indolo[3,2-d] [1]benzazepin-6(5H)-one, 2,9-dibromo-7,12-dihydro-indolo[3,2-d][1]-benzazepin-6(5H)-one, 7,12-dihydro-9-trifluormethyl-indolo-[3,2-d][1]benzazepin-6(5H)-one, 9-chloro-7,12-dihydro-indolo[3,2-d][1]benzazepin-6(5H)-one, 8-bromo-6,11-dihydro-thieno[3',2':2,3]azepino[4,5-b]indol-5(4H)-one and 7,12-dihydro-9-methoxy-indolo[3,2-d][1]benzazepin-6(5H)-on e, or a pharmacologically acceptable salt thereof.
(15) The medical drug according to any one of the above items (1) to (3) wherein the substance that inhibits GSK-3 is 9-bromo-7,12-dihydro-indolo[3,2-d] [1]benzazepin-6(5H)-one or a pharmacologically acceptable salt thereof.
(16) The medical drug according to any one of the above items (1) to (3) wherein the substance that inhibits GSK-3 is a compound represented by the formula (V): 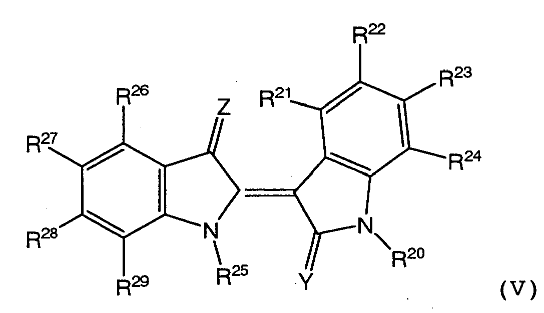 [wherein R²⁰ and R²⁵ which may be the same or different represent hydrogen; halogen; a hydroxy group; a methylene hydroxy group; a straight chain or branched C₁ to C₁₈-alkyl or alkoxy or methylenealkoxy group; a cycloalkyl group having 3 to 7 carbon atoms, and including one or more heteroatoms as needed; a substituted or unsubstituted aryl, aralkyl or aryloxy group having one or more heteroatoms as needed; a mono-, di- or trialkylsilyl group each independently having 1 to 6 carbon atoms within the straight chain or branched alkyl group; a mono-, di- or triarylsilyl group each independently having a substituted or unsubstituted aryl group; a trifluoromethyl group; -COM; -COOM; or a -CH₂COOM group (wherein M represents hydrogen, a straight chain or branched C₁ to C₁₈-alkyl group substituted with one or more hydroxy and/or amino groups if necessary, or an aryl group, which may be substituted with one or more halogen, alkyl groups or alkoxy groups, having one or more heteroatoms if necessary); an -NR³⁰R³¹ group (wherein R³⁰ and R³¹ which may be the same or different represent hydrogen, a C₁ to C₁₈ straight chain or branched alkyl group additionally substituted with one or more hydroxy and/or amino groups if necessary, a substituted or unsubstituted aryl group including one or more heteroatoms if necessary); an acyl group; a -CH₂-NR³⁰R³¹ methyleneamino group (wherein R³⁰ and R³¹ have the meanings as defined above); a benzyl group having one or more heteroatoms in the benzene ring if necessary; a methylenecycloalkyl group having 3 to 7 carbon atoms, and including one or more heteroatoms if necessary; a physiological amino acid group coupled to a nitrogen atom as an amide; an O-glycoside or N-glycoside of which glycoside being selected from monosaccharides or disaccharides; or a methylenesulfonate group; R²¹, R²², R²³, R²⁴, R²⁶, R²⁷, R²⁸ and R²⁹ which may be the same or different represent hydrogen; halogen; a hydroxy group; a nitroso group; a nitro group; an alkoxy group; a straight chain or branched C₁ to C₁₈ alkyl group substituted with one or more hydroxy and/or amino groups if necessary; a substituted or unsubstituted aryl group having one or more heteroatoms if necessary; a substituted or unsubstituted aralkyl group having one or more heteroatoms if necessary; a substituted or unsubstituted aryloxy group having one or more heteroatoms if necessary; a substituted or unsubstituted methylenearyloxy group having one or more heteroatoms if necessary; a cycloalkyl group having 3 to 7 carbon atoms, and including one or more heteroatoms if necessary; a methylenecycloalkyl group having 3 to 7 carbon atoms, and including one or more heteroatoms if necessary; a trifluoromethyl group; -COM; -COOM; or a CH₂COOM group (wherein M represents hydrogen, a straight chain or branched C₁ to C₁₈-alkyl group additionally substituted with one or more hydroxy and/or amino groups if necessary, or an aryl group, which may be substituted with one or more halogen atoms, alkyl groups or alkoxy groups, having one or more heteroatoms if necessary); an -NR³⁰R³¹ group (wherein R³⁰ and R³¹ which may be the same or different represent hydrogen, a straight chain or branched C₁ to C₁₈-alkyl group additionally substituted with one or more hydroxy and/or amino groups if necessary, a substituted or unsubstituted aryl group including one or more heteroatoms if necessary, an acyl group; or form a part of cycloalkyl having 3 to 7 carbon atoms with the nitrogen atom including one or more heteroatoms if necessary); a -CONR³⁰R³¹ group (wherein R³⁰ and R³¹ have the meanings as defined above) ; a hydroxylamino group; a phosphate group; a phosphonate group; a sulfate group; a sulfonate group; a sulfonamide group; an -SO₂NR³⁰R³¹ group (wherein R³⁰ and R³¹ have the meanings as defined above); an -N=N-R³² azo group (wherein R³² represents an aromatic group substituted with one or more carboxyl, phosphoryl or sulfonate groups if necessary, or an O-glycoside or N-glycoside group of which glycoside being selected from monosaccharides or disaccharides); or R²⁰ and R²⁴, and R²⁵ and R²⁹ together form a ring having one to four CH₂ groups each independently substituted if necessary, respectively; Y and Z which may be the same or different represent an oxygen; sulfur; selenium; tellurium atom; an NR³³ group (wherein R³³ represents hydrogen, a straight chain or branched C₁ to C₁₈ alkyl group substituted with one or more carboxyl, phosphoryl or sulfonate groups if necessary, a substituted or unsubstituted aryl group including one or more heteroatoms if necessary, an aralkyl group or a sulfonate group); or -NOR³³ (wherein R³³ group have the meanings as defined above)], or a pharmacologically acceptable salt thereof.
(17) The medical drug according to any one of the above items (1) to (3) wherein the substance that inhibits GSK-3 is a compound selected from the group consisting of indirubin, 5-iodo-indirubin, 5-bromo-indirubin, 5-chloro-indirubin, 5-fluoro-indirubin, 5-methyl-indirubin, 5-nitro-indirubin, 5-SO₃H-indirubin, 5'-bromo-indirubin,5-5'-dibromo-indirubin and 5' -bromo-indirubin 5-sulfonic acid, or a pharmacologically acceptable salt thereof.
(18) The medical drug according to any one of the above items (1) to (3) wherein the substance that inhibits GSK-3 is a compound selected from the group consisting of indirubin-3'-monooxime, 5-iodo-indirubin-3'-monooxime and 5-SO₃Na-indirubin-3'-monooxime, or a pharmacologically acceptable salt thereof.
(19) The medical drug according to any one of the above items (1) to (3) wherein the substance that inhibits GSK-3 is indirubin-3' -monooxime or a pharmacologically acceptable salt thereof.
(20) An agent for the promotion of neuropoiesis of a neural stem cell which comprises a substance that inhibits the activity of GSK-3, as an active ingredient.
(21) The agent for the promotion of neuropoiesis according to the above item (20) wherein the substance that inhibits the activity of GSK-3 is lithiumor a pharmacologically acceptable salt thereof.
(22) The agent for the promotion of neuropoiesis according to the above item (20) wherein the substance that inhibits the activity of GSK-3 is a bisindolylmaleimide derivative, a 3-aryl-4-indolylmaleimide derivative, an indolocarbazole derivative, an indolo[3,2-d][1]benzazepin-6(5H)-one derivative or an indirubin derivative, or a pharmacologically acceptable salt thereof.
(23) The agent for the promotion of neuropoiesis according to the above item (20) wherein the substance that inhibits the activity of GSK-3 is a compound represented by the formula (I): 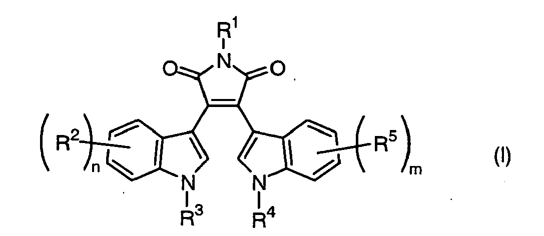 [wherein n and m may be the same or different, and represent an integer of 1 to 3; R¹, R³ and R⁴ may be the same or different, and represent hydrogen, substituted or unsubstituted lower alkyl, substituted or unsubstituted lower alkenyl, -COR⁶ (wherein R⁶ represents hydrogen, substituted or unsubstituted lower alkyl, substituted or unsubstituted lower alkenyl, substituted or unsubstituted aryl or substituted or unsubstituted cycloalkyl), -COOR⁷ (wherein R⁷ represents hydrogen, substituted or unsubstituted lower alkyl, substituted or unsubstituted aryl or substituted or unsubstituted cycloalkyl) or -OR⁸ (wherein R⁸ represents hydrogen, substituted or unsubstituted lower alkyl, substituted or unsubstituted aryl or substituted or unsubstituted cycloalkyl); R² and R⁵ may be the same or different, and represent hydrogen, substituted or unsubstituted lower alkyl, a substituted or unsubstituted lower alkenyl, substituted or unsubstituted lower alkoxy, substituted or unsubstituted lower alkoxycarbonyl, substituted or unsubstituted aryl, carboxy, halogen, hydroxy, nitro, amino, or mono- or di-lower alkylamino; when n and m are 2 or 3, each of R² and R^{S} may be the same or different], a compound represented by the formula (II): 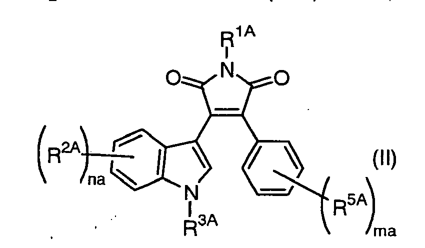 (wherein na, ma, R^{1A}, R^{2A}, R^{3A} and R^{5A} are as defined for the aforementioned n, m, R¹, R², R³ and R⁵, respectively) or a compound represented by the formula (III): 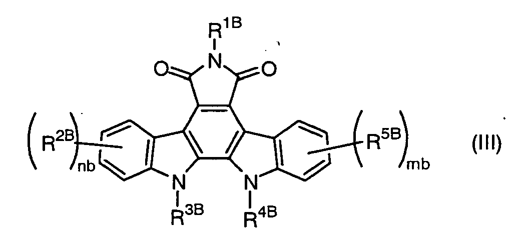 [wherein nb, mb, R^{1B}, R^{2B} and R^{5B} are as defined for the aforementioned n, m, R¹, R² and R⁵, respectively; R^{3B} and R^{4B} may be the same or different, and represent hydrogen, substituted or unsubstituted lower alkyl, substituted or unsubstituted lower alkenyl, -COR⁶ (wherein R⁶ is as defined above), -COOR⁷ (wherein R⁷ is as defined above) or -OR⁸ (wherein R⁸ is as defined above), or R^{3B} and R^{4B} together form 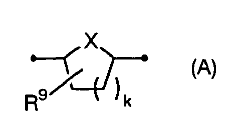 (wherein k represents 1 or 2; X represents CH₂, NH, an oxygen atom or a sulfur atom; R⁹ represents hydroxy, carboxy, carbamoyl or lower alkoxycarbonyl)], or a pharmacologically acceptable salt thereof.
(24) The agent for the promotion of neuropoiesis according to the above items (20) wherein the substance that inhibits the activity of GSK-3 is a compound represented by the formula (Ia): 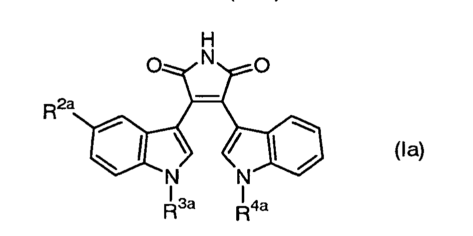 (wherein R^{2a} represents hydrogen, lower alkoxy, lower alkoxycarbonyl, aryl or nitro; R^{3a} and R^{4a} may be the same or different, and represent substituted or unsubstituted lower alkyl), or a pharmacologically acceptable salt thereof.
(25) The agent for the promotion of neuropoiesis according to the above item (20) wherein the substance that inhibits the activity of GSK-3 is a compound represented by the formula (IIa): 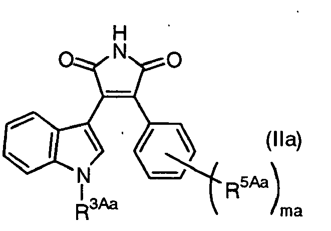 (wherein ma is as defined above; R^{3Aa} represents substituted or unsubstituted lower alkyl; R^{5Aa} represents halogen), or a pharmacologically acceptable salt thereof.
(26) The agent for the promotion of neuropoiesis according to the above item (20) wherein the substance that inhibits the activity of GSK-3 is a compound represented by the formula (IIIa): 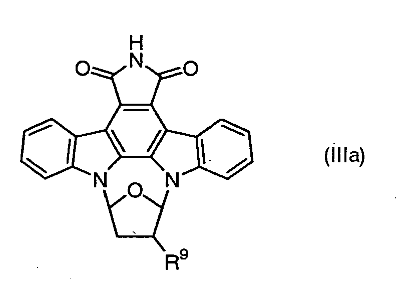 (wherein R⁹ is as defined above) or a pharmacologically acceptable salt thereof.
(27) The agent for the promotion of neuropoiesis according to the above item (20) wherein the substance that inhibits the activity of GSK-3 is a compound selected from the group consisting of 3,4-bis(1-methylindole-3-yl)-1H-pyrrole-2,5-dione, 3-(1-methylindole-3-yl)-4-(1-propyl-indole-3-yl)-1H-pyrrole-2,5-dione, 3-[1-(3-cyanopropyl)-indole-3-yl)-4-(1-methylindole-3-yl)-1H-pyrrole-2,5-dione, 3-[1-(3-aminopropyl)indole-3-yl]-4-(1-methylindole-3-yl)-1 H-pyrrole-2,5-dione, 3-[1-(3-carboxypropyl)indole-3-yl]-4-(1-methylindole-3-yl) -1H-pyrrole-2,5-dione, 3-[1-(3-carbamoylpropyl)indole-3-yl]-4-(1-methylindole-3-y 1)-1H-pyrrole-2,5-dione, 3-[1-(3-aminopropyl)indole-3-yl]-4-(1-methyl-5-propyloxyin dole-3-yl)-1H-pyrrole-2,5-dione, 3-[1-(3-hydroxypropyl)indole-3-yl]-4-(1-methyl-5-phenylind ole-3-yl)-1H-pyrrole-2,5-dione, 3-[1-(3-aminopropyl)indole-3-yl]-4-(1-methyl-5-phenylindol e-3-yl)-1H-pyrrole-2,5-dione, 3-[1-(3-hydroxypropyl)indole-3-yl]-4-(1-methyl-5-methoxy-carbonylindole-3-yl)-1H-pyrrole-2,5-dione, 3-[1-(3-hydroxypropyl)indole-3-yl]-4-(1-methyl-5-nitroindole-3-yl)-1H-pyr role-2,5-dione, 3-(1-methylindole-3-yl)-4-[1-(3-hydroxypropyl)-5-nitroindo le-3-yl]-1H-pyrrole-2,5-dione, 3-(2-chlorophenyl)-4-(1-methylindole-3-yl)-1H-pyrrole-2,5-dione, 3-(2,4-dichlorophenyl)-4-(1-methylindole-3-yl)-1H-pyrrole-2,5-dione, 3-(2-chlorophenyl)-4-[1-(3-hydroxypropyl)-indole-3-yl]-1H-pyrrole-2,5-dione, 4-[1-(3-aminopropyl)-indole-3-yl]-3-(2-chlorophenyl)-1H-pyrrole-2,5-dione and 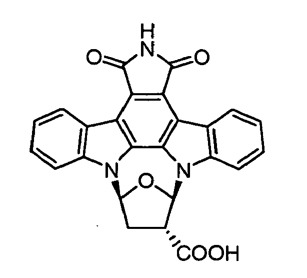 , or a pharmacologically acceptable salt thereof.
(28) The agent for the promotion of neuropoiesis according to the above item (20) wherein the substance that inhibits GSK-3 is a compound represented by the formula (IV) : 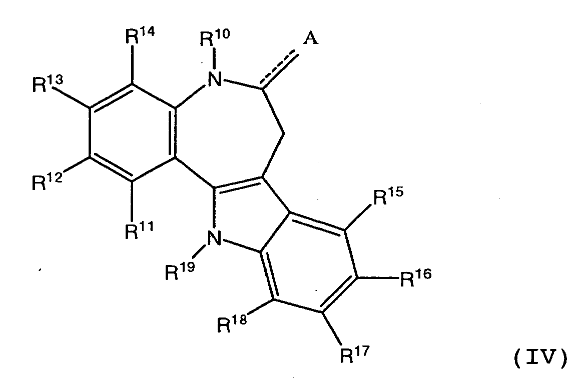 [wherein A is oxygen or sulfur coupled to the right by a single or double bond; R¹⁰ is selected from the group consisting of hydrogen, aryl, lower aliphatic substituents, particularly alkyl and lower alkyl ester; R¹¹-R¹⁴ are independently selected from the group consisting of alkoxy, amino, acyl, aliphatic substituents, particularly alkyl, alkenyl and alkinyl substituents, aliphatic alcohols, particularly alkyl alcohols, aliphaticnitriles, particularly alkyl nitriles, cyano, nitro, carboxyl, halogen, hydrogen, hydroxyl, imino and α,β-unsaturated ketones; R¹⁵-R¹⁸ are independently selected from the group consisting of aliphatic substituents, particularly alkyl, alkenyl and alkinyl substituents, particularly lower aliphatic substituents, alipahatic alcohols, particularly alkyl alcohols, alkoxy, acyl, cyano, nitro, epoxy, haloalkyl groups, halogen, hydrogen and hydroxyl; R¹⁹ is selected from the group consisting of aliphatic groups, particularly lower alkyl groups, aliphatic alcohols, particularly alkyl alcohols, carboxylic acids and hydrogen], or a pharmacologically acceptable salt thereof.
(29) The agent for the promotion of neuropoiesis according to the above item (20) wherein the substance that inhibits GSK-3 is a compound selected from the group consisting of 7,12-dihydro-indolo[3,2-d][1]benzazepin-6(5H)-one, 2-bromo-7,12-dihydro-indolo[3,2-d][1]benzazepin-6(5H)-one, 9-bromo-7,12-dihydro-indolo[3,2-d][1]benzazepin-6(5H)-one, 9-chloro-7,12-dihydro-indolo[3,2-d][1]benzazepin-6(5H)-one, 11-chloro-7,12-dihydro-indolo[3,2-d][1]benzazepin-6(5H)-on e, 10-bromo-7,12-dihydro-indolo[3,2-d][1]benzazepin-6(5H)-one, 8-bromo-6,11-dihydro-thieno[3',2':2,3]azepino-[4,5-b]indol-5(4H)-one, 9-bromo-7,12-dihydro-4-methoxy-indolo[3,2-d][1]benzazepin-6(5H)-one, 9-bromo-7,12-dihydro-4-hydroxy-indolo[3,2-d] [1]benzazepin-6(5H)-one, 7,12-dihydro-4-methoxy-indolo[3,2-d][1]benzazepin-6(5H)-on e, 9-bromo-7,12-dihydro-2,3-dimethoxy-indolo[3,2-d]-[1]benzazepin-6(5H)-one, 9-bromo-7,12-dihydro-2,3-di-hydroxy-indolo[3,2-d] [1]benzazepin-6(5H)-one, 7,12-dihydro-2,3-dimethoxy-indolo[3,2-d] [1]benzazepin-6(5H)-one, 7,12-dihydro-9-trifluormethyl-indolo[3,2-d] [1]benzazepin-6 (5H)-one, 7,12-dihydro-2,3-dimethoxy-9-trifluoromethyl-indolo[3,2-d] [1]benzazepin-6(5H)-one, 2-bromo-7,12-dihydro-9-trifluoromethyl-indolo[3,2-d][1]benzazepin-6(5H)-o ne, 9-bromo-7,12-dihydro-indolo[3,2d][1]benzazepin-6(5H)-thion e, 9-bromo-5,12-bis-(t-butyloxycarbonyl)-7,12-dihydro-indolo[3,2-d][1]benzazepin-6(5H)-one, 9-bromo-12-(t-butyloxycarbonyl)-7,12-dihydro-indolo[3,2-d] [1]benzazepin-6(5H)-one, 9-bromo-5,7-bis-(t-butyloxycarbonyl)-7,12-dihydro-indolo[3,2-d][1]benzazepin-6(5H)-one, 9-bromo-5,7,12-tri-(t-butyloxycarbonyl)-7,12-dihydro-indol o[3,2-d][1]benzazepin-6(5H)-one, 9-bromo-7,12-dihydro-5-methyloxycarbonylmethyl-indolo[3,2-d][1]benzazepin-6(5H)-one, 9-bromo-7,12-dihydro-12-methyloxycarbonylmethyl-indolo-[3,2-d][1]benzazepin-6(5H)-one, 9-bromo-7,12-dihydro-12-(2-hydroxyethyl)-indolo[3,2-d][1]benzazepin-6-(5H)-one, 2,9-dibromo-7,12-dihydro-indolo[3,2-d] [1]-benzazepin-6(5H)-one, 8,10-dichloro-7,12-dihydro-indolo-[3,2d] [1]benzazepin-6(5H)-one, 9-cyano-7,12-dihydro-indolo[3,2-d] [1]benzazepin-6(5H)-one, 9-bromo-7,12-dihydro-5-methyl-indolo[3,2-d] [1]benzazepin-6(5H)-one, 5-benzyl-9-bromo-7,12-dihydro-5-methyl-indolo[3,2-d] [1]-benzazepin-6(5H)-one, 9-bromo-7,12-dihydro-12-methyl-indolo[3,2-d][1]benzazepin-6(5H)-one, 9-bromo-12-ethyl-7,12-dihydro-indolo[3,2-d][1]benzazepin-6 (5H)-one, 9-bromo-7,12-dihydro-12-(2-propenyl)-indolo[3,2-d][1]benzazepin-6(5H)-one, 7,12-dihydro-9-methyl-indolo[3,2-d][1]-benzazepin-6(5H)-one, 7,12-dihydro-9-methoxy-indolo[3,2-d]-[1]benzazepin-6(5H)-one, 9-fluoro-7,12-dihydro-12-(2-propenyl)-indolo[3,2-d][1]benz azepin-6(5H)-one, 11-bromo-7,12-dihydro-indolo[3,2d][1]benzazepin-6(5H)-one, 9-bromo-7,12-dihydro-2-(methyliminoamine)-indolo[3,2-d]-[1]benzazepin-6(5H)-one,9-bromo-7,12-dihydro-2-(carboxylic acid)-indolo[3,2-d][1]benzazepin-6(5H)-one, 9-bromo-7,12-dihydro-10-hydroxy-indolo[3,2-d][1]benzazepin -6(5H)-one, 9-bromo-7,12-dihydro-11-hydroxymethyl-indolo[3,2-d][1]-benzazepin-6(5H)-one, 7,12-dihydro-4-hydroxy-indolo[3,2-d]-[1]benzazepin-6(5H)-one, 7,12-dihydro-2,3-dihydroxy-indolo[3,2-d][1]benzazepin-6(5H )-one, 2,3-dimethoxy-9-nitro-7,12-dihydro-indolo[3,2d][1]benzazep in-6(5H)-one, 9-cyano-7,12-dihydro-indolo[3,2-d][1]benzazepin-6(5H)-one, 2,3-dimethoxy-9-cyano-7,12-dihydro-indolo[3,2-d][1]benzazepin-6(5H)-one, 9-nitro-7,12-dihydro-indolo[3,2-d][1]-benzazepin-6(5H)-one, 3-(6-oxo-9-trifluoromethyl-5,6,7,12-tetrahydro-indolo[3,2-d][1]benzazepin-2-yl)-propionitrile, 2-bromo-9-nitro-7,12-dihydro-indolo[3,2-d][1]benzazepin-6-(5H)-one, 3-(6-oxo-9-trifluoromethyl-5,6,7,12-tetrahydro-indolo[3,2-d][1]benzazepin-2-yl)acrylonitrile, 2-(3-hydroxy-1-propinyl)-9-trifluoromethyl-7,12-dihydro-in dolo[3,2-d][1]benzazepin-6(5H)-one, 2-iodo-9-bromo-7,12-dihydro-indolo[3,2-d][1]benzazepin-6(5 H)-one, 2-(3-oxo-1-butenyl)-9-trifluoromethyl-7,12-tetrahydro-indo lo[3,2-d][1]benzazepin-6(5H)-one, 8-chloro-6,11-dihydro-thieno-[3',2':2,3]azepino[4,5-b]indol-5(4H)-one, 2-iodo-9-trifluoromethyl-7,12-dihydro-indolo[3,2-d][1]benzazepin-6-(5H)-one, 7,12-dihydro-pyrido[3',2':4,5]pyrrolo[3,2-d] [1]-benzazepin-6(5H)-one, 11-methyl-7,12-dihydro-indolo-[3,2-d][1]-benzazepin-6(5H)-one, 2-[2-(1-hydroxycyclohexyl)ethinyl]-9-trifluoromethyl-7,12-dihydro-indolo[3,2-d]-[1]benzazepin-6(5H)-one, 2-cyano-7,12-dihydro-indolo-[3,2-d][1]benzazepin-6(5H)-one, 2-iodo-7,12-dihydro-indolo[3,2-d][1]benzazepin-6(5H)-one, 11-ethyl-7,12-dihydro-indolo[3,2-d][1]benzazepin-6(5H)-one, 8-methyl-6,11-dihydro-thieno[3',2':2,3]azepino[4,5-b]indol -5(4H)-one and 3-(6-oxo-9-trifluoromethyl-5,6,7,12-tetrahydro-indolo[3,2-d][1]benzazepin-2-yl)acrylic acid, methyl ester, or a pharmacologically acceptable salt thereof.
(30) The agent for the promotion of neuropoiesis according to the above item (20) wherein the substance that inhibits GSK-3 is a compound selected from the group consisting of 9-cyano-7,12-dihydro-indolo[3,2-d] [1]benzazepin-6(5H)-one, 9-bromo-7,12-dihydro-2,3-dimethoxy-indolo[3,2-d][1]benzazepin-6(5H)-one, 2-bromo-7,12-dihydro-9-trifluoromethyl-indolo[3,2-d][1]benzazepin-6(5H)-one, 7,12-dihydro-2,3-dimethoxy-9-trifluoromethyl-indolo[3,2-d] [1]benzazepin-6(5H)-one, 2,9-dibromo-7,12-dihydro-indolo[3,2-d][1]-benzazepin-6(5H)-one, 7,12-dihydro-9-trifluoromethyl-indolo[3,2-d][1]benzazepin-6(5H)-one, 9-chloro-7,12-dihydro-indolo[3,2-d][1]benzazepin-6(5H)-one, 8-bromo-6,11-dihydro-thieno[3',2':2,3]azepino[4,5-b]indole -5(4H)-one, 7,12-dihydro-9-methoxy-indolo[3,2-d][1]benzazepin-6(5H)-on e, 10-bromo-7,12-dihydro-indolo[3,2-d][1]benzazepin-6(5H)-one, 11-bromo-7,12-dihydro-indolo[3,2-d][1]benzazepin-6(5H)-one, 11-chloro-7,12-dihydro-indolo[3,2-d][1]benzazepin-6(5H)-one, 9-fluoro-7,12-dihydro-indolo[3,2-d]-[1]benzazepin-6(5H)-one, 9-methyl-7,12-dihydro-indolo-[3,2-d][1]benzazepin-6(5H)-one, 9-bromo-7,12-dihydro-indolo[3,2-d][1]benzazepin-6(5H)-thione, 8,10-dichloro-7,12-dihydro-indolo[3,2-d][1]benzazepin-6(5H)-one, 9-bromo-7,12-dihydro-12-(2-hydroxyethyl)-indolo[3,2-d][1]-benzazepin-6(5H)-one, 9-bromo-7,12-dihydro-2,3-dihydroxy-indolo[3,2-d][1]benzaze pin-6(5H)-one, 2-bromo-7,12-dihydro-indolo[3,2-d][1]benzazepin-6(5H)-one, 7,12-dihydro-2,3-dimethoxy-indolo[3,2-d][1]benzazepin-6(5H )-one, 9-bromo-7,12-dihydro-12-methyl-indolo[3,2-d][1]benzazepin-6(5H)-one, 9-bromo-7,12-dihydro-5-methyloxycarbonylmethyl-indolo[3,2-d][1]benzazepin-6(5H)-one and 7,12-dihydro-indolo[3,2-d][1]benzazepin-6(5H)-one, or a pharmacologically acceptable salt thereof.
(31) The agent for the promotion of neuropoiesis according to the above item (20) wherein the substance that inhibits GSK-3 is a compound selected from the group consisting of 9-cyano-7,12-dihydro-indolo[3,2-d][1]benzazepin-6(5H)-one, 9-bromo-7,12-dihydro-2,3-dimethoxy-indolo[3,2-d][1]-benzazepin-6(5H)-one, 2-bromo-7,12-dihydro-9-trifluoromethyl-indolo[3,2-d][1]benzazepin-6(5H)-one, 7,12-dihydro-2,3-dimethoxy-9-trifluoromethyl-indolo[3,2-d] [1]benzazepin-6(5H)-one, 2,9-dibromo-7,12-dihydro-indolo[3,2-d][1]-benzazepin-6(5H)-one, 7,12-dihydro-9-trifluormethyl-indolo[3,2-d][1]benzazepin-6 (5H)-one, 9-chloro-7,12-dihydro-indolo[3,2-d][1]benzazepin-6(5H)-one, 8-bromo-6,11-dihydro-thieno[3',2':2,3]azepino[4,5-b]indol-5(4H)-one and 7,12-dihydro-9-methoxy-indolo[3,2-d][1]benzazepin-6(5H)-on e, or a pharmacologically acceptable salt thereof.
(32) The agent for the promotion of neuropoiesis according to the above item (20) wherein the substance that inhibits GSK-3 is 9-bromo-7,12-dihydro-indolo[3,2-d][1]-benzazepin-6(5H)-one or a pharmacologically acceptable salt thereof.
(33) The agent for the promotion of neuropoiesis according to the above item (20) wherein the substance that inhibits the activity of GSK-3 is a compound represented by the formula (V): 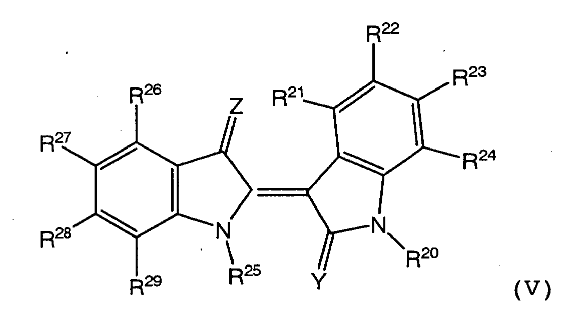 [wherein R²⁰ and R²⁵ which may be the same or different represent hydrogen; halogen; a hydroxy group; a methylene hydroxy group; a straight chain or branched C₁ to C₁₈-alkyl or alkoxy or methylenealkoxy group; a cycloalkyl group having 3 to 7 carbon atoms, and including one or more heteroatoms as needed; a substituted or unsubstituted aryl, aralkyl or aryloxy group having one or more heteroatoms as needed; a mono-, di- or trialkylsilyl group each independently having 1 to 6 carbon atoms within the straight chain or branched alkyl group; a mono-, di- or triarylsilyl group each independently having a substituted or unsubstituted aryl group; a trifluoromethyl group; -COM; -COOM; or a -CH₂COOM group (wherein M represents hydrogen, a straight chain or branched C₁ to C₁₈-alkyl group substituted with one or more hydroxy and/or amino groups if necessary, or an aryl group, which may be substituted with one or more halogen, alkyl groups or alkoxy groups, having one or more heteroatoms if necessary); an -NR³⁰R³¹ group (wherein R³⁰ and R³¹ which may be the same or different represent hydrogen, a C₁ to C₁₈ straight chain or branched alkyl group additionally substituted with one or more hydroxy and/or amino groups if necessary, a substituted or unsubstituted aryl group including one or more heteroatoms if necessary); an acyl group; a -CH₂-NR³⁰R³¹ methyleneamino group (wherein R³⁰ and R³¹ have the meanings as defined above); a benzyl group having one or more heteroatoms in the benzene ring if necessary; a methylenecycloalkyl group having 3 to 7 carbon atoms, and including one or more heteroatoms if necessary; a physiological amino acid group coupled to a nitrogen atom as an amide; an O-glycoside or N-glycoside of which glycoside being selected frommonosaccharides or disaccharides; or a methylenesulfonate group; R²¹, R²², R²³, R²⁴, R²⁶, R²⁷, R²⁸ and R²⁹ which may be the same or different represent hydrogen; halogen; a hydroxy group; a nitroso group; a nitro group; an alkoxy group; a straight chain or branched C₁ to C₁₈ alkyl group substituted with one or more hydroxy and/or amino groups if necessary; a substituted or unsubstituted aryl group having one or more heteroatoms if necessary; a substituted or unsubstituted aralkyl group having one or more heteroatoms if necessary; a substituted or unsubstituted aryloxy group having one or more heteroatoms if necessary; a substituted or unsubstituted methylenearyloxy group having one or more heteroatoms if necessary; a cycloalkyl group having 3 to 7 carbon atoms, and including one or more heteroatoms if necessary; a methylenecycloalkyl group having 3 to 7 carbon atoms, and including one or more heteroatoms if necessary; a trifluoromethyl group; -COM; -COOM; or a CH₂COOM group (wherein M represents hydrogen, a straight chain or branched C₁ to C₁₈-alkyl group additionally substituted with one or more hydroxy and/or amino groups if necessary, or an aryl group, which may be substituted with one or more halogen atoms, alkyl groups or alkoxy groups, having one or more heteroatoms if necessary); an -NR³⁰R³¹ group (wherein R³⁰ and R³¹ which may be the same or different represent hydrogen, a straight chain or branched C₁ to C₁₈-alkyl group additionally substituted with one or more hydroxy and/or amino groups if necessary, a substituted or unsubstituted aryl group including one or more heteroatoms if necessary, an acyl group; or form a part of cycloalkyl having 3 to 7 carbon atoms with the nitrogen atom including one or more heteroatoms if necessary); a -CONR³⁰R³¹ group (wherein R³⁰ and R³¹ have the meanings as defined above); a hydroxylamino group; a phosphate group; a phosphonate group; a sulfate group; a sulfonate group; a sulfonamide group; an -SO₂NR³⁰R³¹ group (wherein R³⁰ and R³¹ have the meanings as defined above); an -N=N-R³² azo group (wherein R³² represents an aromatic group substituted with one or more carboxyl, phosphoryl or sulfonate groups if necessary, or an O-glycoside or N-glycoside group of which glycoside being selected from monosaccharides or disaccharides); or R²⁰ and R²⁴, and R²⁵ and R²⁹ together form a ring having one to four CH₂ groups each independently substituted if necessary, respectively; Y and Z which may be the same or different represent an oxygen; sulfur; selenium; tellurium atom; an NR³³ group (wherein R³³ represents hydrogen, a straight chain or branched C₁ to C₁₈ alkyl group substituted with one or more carboxyl, phosphoryl or sulfonate groups if necessary, a substituted or unsubstituted aryl group including one or more heteroatoms if necessary, an aralkyl group or a sulfonate group); or -NOR³³ (wherein R³³ group have the meanings as defined above)], or a pharmacologically acceptable salt thereof.
(34) The agent for the promotion of neuropoiesis according to the above item (20) wherein the substance that inhibits GSK-3 is a compound selected from the group consisting of indirubin, 5-iodo-indirubin, 5-bromo-indirubin, 5-chloro-indirubin, 5-fluoro-indirubin, 5-methyl-indirubin, 5-nitro-indirubin, 5-SO₃H-indirubin, 5'-bromo-indirubin, 5-5' -dibromo-indirubin and 5' -bromo-indirubin 5-sulfonic acid, or a pharmacologically acceptable salt thereof.
(35) The agent for the promotion of neuropoiesis according to the above item (20) wherein the substance that inhibits GSK-3 is a compound selected from the group consisting of indirubin-3' -monooxime, 5-iodo-indirubin-3' -monooxime and 5-SO₃Na-indirubin-3'-monooxime, or a pharmacologically acceptable salt thereof.
(36) The agent for the promotion of neuropoiesis according to the above item (20) wherein the substance that inhibits GSK-3 is indirubin-3'-monooxime or a pharmacologically acceptable salt thereof.
(37) A neuron obtained by culturing a neural stem cell in the presence of the agent for the promotion of neuropoiesis according to any one of the above items (20) to (36).
(38) A method of the manufacture of a neuron which comprises culturing a neural stem cell in the presence of the agent for the promotion of neuropoiesis according to any one of the above items (20) to (36) to allow neogenesis of the neuron, and collecting the neuron from the culture.
(39) A method of the regeneration of a nerve which comprises administering a substance that inhibits GSK-3.
(40) Use of a substance that inhibits GSK-3 for the manufacture of a nerve regenerating drug.
(41) Use of a substance that inhibits GSK-3 for the manufacture of an agent for the promotion of neuropoiesis of a neural stem cell.

Hereinafter, details of the present invention are explained.
1. A substance that inhibits the activity of GSK-3 included in the nerve regenerating drug and the agent for the promotion of neuropoiesis of a neural stem cell of the invention
   The substance that inhibits the activity of GSK-3 may be any one as long as it is a compound that inhibits the activity of GSK-3, and examples thereof include e.g., lithium, bisindolylmaleimide derivatives, 3-aryl-4-indolylmaleimide derivatives, indolocarbazole derivatives, indolo[3,2-d][1]benzazepin-6(5H)-one derivatives, indirubin derivatives and the like.
   Specific examples of the bisindolylmaleimide derivative, 3-aryl-4-indolylmaleimide derivative, indolocarbazole derivative include e.g., compounds represented by the formulae (I) to (III). Among them, 3,4-bis (1-methylindole-3-yl)-1H-pyrrole-2,5-dione, 3-(1-methylindole-3-yl)-4-(1-propyl-indole-3-yl)-1H-pyrrole-2,5-dione, 3-[1-(3-cyanopropyl)-indole-3-yl]-4-(1-methylindole-3-yl)-1H-pyrrole-2,5-dione, 3-[1-(3-aminopropyl)indole-3-yl]-4-(1-methylindole-3-yl)-1 H-pyrrole-2,5-dione, 3-[1-(3-carboxypropyl)indole-3-yl]-4-(1-methylindole-3-yl) -1H-pyrrole-2,5-dione, 3-[1-(3-carbamoylpropyl)indole-3-yl]-4-(1-methylindole-3-y 1)-1H-pyrrole-2,5-dione, 3-[1-(3-aminopropyl)indole-3-yl]-4-(1-methyl-5-propyloxyin dole-3-yl)-1H-pyrrole-2,5-dione, 3-[1-(3-hydroxypropyl)indole-3-yl]-4-(1-methyl-5-phenylind ole-3-yl)-1H-pyrrole-2,5-dione, 3-[1-(3-aminopropyl)indole-3-yl]-4-(1-methyl-5-phenylindol e-3-yl)-1H-pyrrole-2,5-dione, 3-[1-(3-hydroxypropyl)indole-3-yl]-4-(1-methyl-5-methoxy-carbonylindole-3-yl)-1H-pyrrole-2,5-dione, 3-[1-(3-hydroxy-propyl)indole-3-yl]-4-(1-methyl-5-nitroindole-3-yl)-1H-pyr role-2,5-dione, 3-(1-methylindole-3-yl)-4-[1-(3-hydroxypropyl)-5-nitroindo le-3-yl]-1H-pyrrole-2,5-dione, 3-(2-chlorophenyl)-4-(1-methylindole-3-yl)-1H-pyrrole-2,5-dione, 3-(2,4-dichlorophenyl)-4-(1-methylindole-3-yl)-1H-pyrrole-2,5-dione, 3-(2-chlorophenyl)-4-[1-(3-hydroxypropyl)indole-3-yl]-1H-pyrrole-2,5-dione, 3-[1-(3-aminopropyl)indole-3-yl]-4-(2-chlorophenyl)-1H-pyrrole-2,5-dion e, 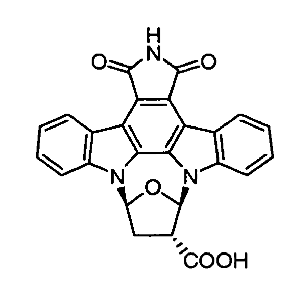 and the like are preferred.
   Specific examples of the indolo[3,2-d][1]benzazepin-6(5H)-one derivative include e.g., compounds represented by the formula (IV). Among them, 7,12-dihydro-indolo[3,2-d][1]benzazepin-6(5H)-one, 2-bromo-7,12-dihydro-indolo[3,2-d][1]benzazepin-6(5H)-one, 9-bromo-7,12-dihydro-indolo[3,2-d][1]benzazepin-6(5H)-one, 9-chloro-7,12-dihydro-indolo[3,2-d][1]benzazepin-6(5H)-one, 11-chloro-7,12-dihydro-indolo[3,2-d][1]benzazepin-6(5H)-on e, 10-bromo-7,12-dihydro-indolo[3,2-d][1]benzazepin-6(5H)-one, 8-bromo-6,11-dihydro-thieno[3',2':2,3]azepino[4,5-b]-indole-5(4H)-one, 9-bromo-7,12-dihydro-4-methoxy-indolo-[3,2-d][1]benzazepin-6(5H)-one, 9-bromo-7,12-dihydro-4-hydroxy-indolo[3,2-d][1]benzazepin-6(5H)-one, 7,12-dihydro-4-methoxy-indolo[3,2-d][1]benzazepin-6(5H)-on e, 9-bromo-7,12-dihydro-2,3-dimethoxy-indolo[3,2-d][1]-benzazepin-6(5H)-one, 9-bromo-7,12-dihydro-2,3-dihydroxy-indolo-[3,2-d][1]benzazepin-6(5H)-one, 7,12-dihydro-2,3-dimethoxy-indolo[3,2-d] [1]benzazepin-6(5H )-one, 7,12-dihydro-9-trifluoromethyl-indolo[3,2-d][1]benzazepin-6(5H)-one, 7,12-dihydro-2,3-dimethoxy-9-trifluoromethyl-indolo[3,2-d] [1]benzazepin-6(5H)-one, 2-bromo-7,12-dihydro-9-trifluoromethyl-indolo[3,2-d][1]ben zazepin-6(5H)-one, 9-bromo-7,12-dihydro-indolo[3,2-d][1]-benzazepin-6(5H)-thione, 9-bromo-5,12-bis-(t-butyloxycarbonyl)-7,12-dihydro-indolo[3,2-d][1]benzazepin-6(5H)-on e, 9-bromo-12-(t-butyloxycarbonyl)-7,12-dihydro-indolo-[3,2-d][1]benzazepin-6(5H)-one, 9-bromo-5,7-bis-(t-butyloxycarbonyl)-7,12-dihydro-indolo[3,2-d][1]benzazepin-6-(5H)-one, 9-bromo-5,7,12-tri-(t-butyloxycarbonyl)-7,12-dihydro-indol O[3,2-d][1]benzazepin-6(5H)-one, 9-bromo-7,12-dihydro-5-methyloxycarbonylmethyl-indolo[3,2-d][1]benzazepin-6(5H)-one, 9-bromo-7,12-dihydro-12-methyl-oxycarbonylmethyl-indolo[3,2-d][1]benzazepin-6(5H)-one, 9-bromo-7,12-dihydro-12-(2-hydroxyethyl)-indolo[3,2-d][1]-benzazepin-6(5H)-one, 2,9-dibromo-7,12-dihydro-indolo[3,2-d]-[1]benzazepin-6(5H)-one, 8,10-dichloro-7,12-dihydro-indolo-[3,2-d][1]benzazepin-6(5H)-one, 9-cyano-7,12-dihydro-indolo[3,2-d][1]benzazepin-6(5H)-one, 9-bromo-7,12-dihydro-5-methyl-indolo[3,2-d][1]benzazepin-6 (5H)-one, 5-benzyl-9-bromo-7,12-dihydro-5-methyl-indolo[3,2-d][1]-benzazepin-6(5H)-one, 9-bromo-7,12-dihydro-12-methyl-indolo[3,2-d][1]benzazepin-6(5H)-one, 9-bromo-12-ethyl-7,12-dihydro-indolo[3,2-d][1]benzazepin-6 (5H)-one, 9-bromo-7,12-dihydro-12-(2-propenyl)-indolo[3,2-d][1]benzazepin-6(5H)-one, 7,12-dihydro-9-methyl-indolo[3,2-d][1]-benzazepin-6(5H)-one, 7,12-dihydro-9-methoxy-indolo[3,2-d]-[1]benzazepin-6(5H)-one, 9-fluoro-7,12-dihydro-12-(2-propenyl)-indolo[3,2-d][1]benz azepin-6(5H)-one, 11-bromo-7,12-dihydro-indolo[3,2-d][1]benzazepin-6(5H)-one, 9-bromo-7,12-dihydro-2-(methyliminoamine)-indolo[3,2-d][1] benzazepin-6(5H)-one, 9-bromo-7,12-dihydro-2-(carboxylic acid)-indolo[3,2-d][1]benzazepin-6(5H)-one, 9-bromo-7,12-dihydro-10-hydroxy-indolo[3,2-d][1]benzazepin -6(5H)-one, 9-bromo-7,12-dihydro-11-hydroxymethyl-indolo[3,2-d][1]-benzazepin-6(5H)-one, 7,12-dihydro-4-hydroxy-indolo[3,2-d][1]benzazepin-6(5H)-on e, 7,12-dihydro-2,3-dihydroxy-indolo-[3,2-d][1]benzazepin-6(5H)-one, 2,3-dimethoxy-9-nitro-7,12-dihydro-indolo[3,2-d][1]benzaze pin-6(5H)-one, 9-cyano-7,12-dihydro-indolo[3,2-d][1]benzazepin-6(5H)-one, 2,3-dimethoxy-9-cyano-7,12-dihydro-indolo[3,2-d][1]benzazepin-6(5H)-one, 9-nitro-7,12-dihydro-indolo[3,2-d][1]-benzazepin-6(5H)-one, 3-(6-oxo-9-trifluoromethyl-5,6,7,12-tetrahydro-indolo[3,2-d][1]benzazepin-2-yl)propionitrile, 2-bromo-9-nitro-7,12-dihydro-indolo[3,2-d][1]benzazepin-6( 5H)-one, 3-(6-oxo-9-trifluoromethyl-5,6,7,12-tetrahydro-indolo[3,2-d][1]benzazepin-2-yl)acrylonitrile, 2-(3-hydroxy-1-propinyl)-9-trifluoromethyl-7,12-dihydro-in dolo[3,2-d][1]benzazepin-6(5H)-one, 2-iodo-9-bromo-7,12-dihydro-indolo[3,2-d][1]benzazepin-6(5 H)-one, 2-(3-oxo-1-butenyl)-9-trifluoromethyl-7,12-tetrahydro-indo lo[3,2-d][1]benzazepin-6(5H)-one, 8-chloro-6,11-dihydro-thieno[3',2':2,3]-azepino[4,5-b]indole-5(4H)-one, 2-iodo-9-trifluoromethyl-7,12-dihydro-indolo[3,2-d][1]benzazepin-6(5H)-one, 7,12-dihydro-pyrido[3',2':4,5]pyrrolo[3,2-d][1]-benzazepin-6(5H)-one, 11-methyl-7,12-dihydro-indolo-[3,2-d][1]benzazepin-6(5H)-one, 2-[2-(1-hydroxycyclohexyl)ethinyl] -9-trifluoromethyl-7,12-dihydro-indolo-[3,2-d] [1]benzazepin-6(5H)-one, 2-cyano-7,12-dihydro-indolo[3,2-d][1]benzazepin-6(5H)-one, 2-iodo-7,12-dihydro-indolo[3,2-d] [1]benzazepin-6(5H)-one, 11-ethyl-7,12-dihydro-indolo[3,2-d][1]benzazepin-6(5H)-one, 8-methyl-6,11-dihydro-thieno[3',2':2,3]azepino[4,5-b]indol e-5(4H)-one, 3-(6-oxo-9-trifluoromethyl-5,6,7,12-tetrahydro-indolo-[3,2-d][1]benzazepin-2-yl)acrylic acid, methyl ester and the like are preferred.
   Specific examples of the indirubin derivative include e.g., compounds represented by the formula (V). Among them, indirubin, 5-iodo-indirubin, 5-bromo-indirubin, 5-chloro-indirubin, 5-fluoro-indirubin, 5-methyl-indirubin, 5-nitro-indirubin, 5-SO₃H-indirubin, 5'-bromo-indirubin, 5-5'-dibromo-indirubin, 5'-bromo-indirubin 5-sulfonic acid, indirubin-3'-monooxime, 5-iodo-indirubin-3'-monooxime, 5-SO₃Na-indirubin-3'-monooxime and the like are preferred.
   Hereinafter, compounds represented by the formulae (I) to (V) are referred to as compounds (I) to (V), respectively. The same is applicable to compounds represented by other number of the formula.
   In the definition of each group in the compounds (I) to (III) and the compounds (Ia) to (IIIa), followings are illustrated.
   (i) Examples of lower alkyl moiety of the lower alkyl, lower alkoxy and lower alkoxycarbonyl include e.g., straight chain or branched alkyl having 1 to 10 carbon atoms, and specific examples thereof include methyl, ethyl, propyl, isopropyl, butyl, isobutyl, sec-butyl, tert-butyl, pentyl, isopentyl, neopentyl, hexyl, heptyl, octyl, 6-methylheptyl, isooctyl, nonyl, decyl and the like.
   (ii) Examples of the cycloalkyl include e.g., cycloalkyl having 3 to 8 carbon atoms, and specific examples thereof include cyclopropyl,cyclobutyl,cyclopentyl,cyclohexyl,cycloheptyl, cyclooctyl and the like.
   (iii) Examples of the lower alkenyl include e.g., straight chain, branched or cyclic alkenyl having 2 to 8 carbon atoms, and specific examples thereof include vinyl, allyl, 1-propenyl, butenyl, pentenyl, hexenyl, heptenyl, octenyl, cyclohexenyl, 2,6-octadienyl and the like.
   (iv) Examples of lower alkyl moiety of the mono- or di-lower alkylamino are as defined for the aforementioned lower alkyl, and two lower alkyl moieties of a dilower alkylamino may be the same or different.
   (v) The halogen represents each atom of fluorine, chlorine, bromine and iodine.
   (vi) Examples of the aryl include e.g., monocyclic, bicyclic or tricyclic aryl having 6 to 14 carbon atoms, and specific examples thereof include phenyl, naphthyl, indenyl, anthranil and the like.
   (vii) The substituents in the substituted lower alkyl, substituted lower alkenyl, substituted lower alkoxy and substituted lower alkoxycarbonyl may be the same or different, and examples thereof include halogen, cyano, nitro, hydroxy, carboxy, carbamoyl, amino, mono- or di-lower alkylamino, cycloalkyl, lower alkanoyl, lower alkoxy, aryl, substituted aryl, aryloxy, substituted aryloxy, lower alkoxycarbonyl, lower alkanoyloxy and the like with the number of substitution being 1 to 3.
      The illustrated halogen, mono- or di-lower alkylamino, cycloalkyl, aryl moiety of the aryl and aryloxy, and the lower alkyl moiety of the lower alkoxy, lower alkoxycarbonyl, lower alkanoyl and lower alkanoyloxy herein are as defined for the aforementioned halogen (v), mono- or di-lower alkylamino (iv), cycloalkyl (ii), aryl (vi) and lower alkyl (i).
      Further, the illustrated substituents in the substituted aryl and substituted aryloxy herein maybe the same or different, and examples thereof include lower alkyl, lower alkoxy, lower alkoxycarbonyl, halogen, cyano, nitro, hydroxy, carboxy, amino and the like with the number of substitution being 1 to 3.
   The illustrated halogen, and the lower alkyl moiety of the lower alkyl, lower alkoxy and lower alkoxycarbonyl herein are as defined for the aforementioned halogen (v) and lower alkyl (i).
   (viii) Examples of the substituent in the substituted aryl and substituted cycloalkyl include e.g., lower alkyl, substituted lower alkyl and the like in addition to the groups included in the definition of the substituent in the aforementioned substituted lower alkyl (vii).
      The illustrated substituents in the substituted lower alkyl herein may be the same or different, and examples thereof include e.g., lower alkoxy, lower alkoxycarbonyl, halogen, cyano, nitro, hydroxy, carboxy, amino and the like with the number of substitution being 1 to 3.
      The illustrated halogen and the lower alkyl moiety of the lower alkoxy and lower alkoxycarbonyl herein are as defined for the aforementioned halogen (v) and lower alkyl (i).
      Further, the illustrated lower alkyl herein is as defined for the aforementioned lower alkyl (i).
      Pharmacologically acceptable salts of the bisindolylmaleimide derivative, 3-aryl-4-indolylmaleimide derivative, indolocarbazole derivative, indolo[3,2-d][1]-benzazepin-6(5H)-one derivative, indirubin derivative, compounds (I) to (V) and compounds (Ia) to (IIIa) are preferably nontoxic and water soluble, and examples thereof include e.g., inorganic acid salts such as hydrochlorides, sulfates, nitrates and phosphates, and organic acid salts such as acetates, maleates, fumarates and citrates; examples of pharmacologically acceptable metal salt include alkalinemetal salts such as sodium salts and potassium salts, alkaline earth metal salts such as magnesium salts and calcium salts, aluminum salts, zinc salts and the like; examples of pharmacologically acceptable ammonium salt include salts such as ammonium and tetramethylammonium; examples of pharmacologically acceptable organic amine salt include addition salts of morpholine, piperidine and the like; and the like.
      The bisindolylmaleimide derivative, 3-aryl-4-indolylmaleimide derivative, indolocarbazole derivative, indolo[3,2-d][1]benzazepin-6(5H)-one derivative, indirubin derivative, compounds (I) to (V) and compounds (Ia) to (IIIa) can be produced by the method described in EP 470490, WO 93/18766, WO 93/18765, EP 397060, WO 98/11105, WO 98/11103, WO 98/11102, WO 98/04552, WO 98/04551, DE 4243321, DE 4005970, DE 4217964, DE 4005970, DE 3914764, WO 96/04906, WO 95/07910, DE 42179464, US 5856517, US 5891901, WO 99/42100, EP 328026, EP 384349, EP 540956, DE 4005969, EP 508792, WO 99/65910, WO 01/037819 and the like, or the method according to the same.
      Moreover, as the substance that inhibits the activity of GSK-3, a short interference RNA (siRNA) can be also used. The siRNA for GSK-3 suppresses the expression of GSK-3 by the RNAi activity thereof, and consequently, it can inhibit the activity of GSK-3. The siRNA can inhibit the activity of GSK-3 through introducing itself within a cell, and in addition, the inhibition also becomes possible by introducing a vector that expresses the siRNA into a cell.
      For producing an effective siRNA on human GSK-3, it is important to select a target sequence that is highly effective. A variety of methods have been known as an algorithm for the determination of the sequence, however, for example, a highly effective target sequence can be selected among 19 arbitrary sequences in a messenger RNA sequence of human GSK-3, by selecting a sequence that fulfills more of the respective conditions of: content of guanine and cytosine being 30 to 52%; three or more bases being adenine or uridine among 5 bases at 3' end; melting temperature being less than 20°C; the third base being adenine; the tenth base being uridine; the 13th base being other than glycine; the 19th base being adenine; the 19th base not being glycine and cytosine. An effective siRNA for human GSK-3 can be produced by allowing hybridization of both of a sense stranded oligo RNA prepared by adding nucleotides of two bases at 3' end of an oligo RNA having the target sequence, and an antisense stranded oligo RNA prepared by adding nucleotides of two bases at 3' end of an oligo RNA having a sequence that is complementary to the target sequence. Although synthesis, purification and hybridization of the siRNA can be executed by any one of various methods, for example, they can be performed using *Silencer* siRNA Construction Kit (manufactured by Ambion, Inc.) according to the attached protocol. The vector for expressing the siRNA can be any one of various kinds of plasmid vectors, and various kinds of viral vectors such as retroviral vectors, lentiviral vectors and adenoviral vectors. For example, it can be produced by incorporating the oligo DNA having the target sequence selected by the aforementioned method into piGENE hU6 Vector (manufactured by iGENE Therapeutics, Inc.) according to the attached protocol. Although introduction of the siRNA or the vector that expresses an siRNA into a cell can be executed by any one of a variety of methods, for example, it can be performed using Nucleofector Device (manufactured by Amaxa) according to the attached protocol.
2. Method of searching a substance that inhibits the activity of GSK-3
   Exemplary method of searching a substance that inhibits the activity of GSK-3 includes a method which comprises:
   [i] permitting coexistence of GSK-3, a peptide phosphorylated by GSK-3, and ATP, in the presence of a subject substance,
   [ii] permitting coexistence of GSK-3, a peptide phosphorylated by GSK-3, and ATP as in the above [i] in the absence of a subject substance,
   [iii] measuring and comparing the amount of thus phosphorylated peptides in both cases [i] and [ii], and
   [iv] selecting a substance yielding a smaller amount of the phosphorylated peptide in the presence of the subject substance, in comparison with the absence of the subject substance.

   Although the subject substance is not particularly limited, examples thereof include e.g., peptides; proteins; cell extracts and purified products derived from the cell extract; cell culture supernatants and purified products derived from the supernatant; biological samples such as a serum and purified products derived from the biological sample; somatic extracts of a microorganism and purified products derived from the somatic extract; microorganism culture supernatants and purified products derived from the supernatant; compounds; compounds synthesized using combinatorial chemistry; and the like.
   GSK-3 is not particularly limited as long as it has the activity of GSK-3, but examples thereof include GSK-3α or β derivedfrompreferablyamammal, andmorepreferablyrat, mouse, monkey or human, and specific examples include a protein having an amino acid sequence set out in SEQ ID NO: 1.
   GSK-3 can be obtained by a method which comprises introducing an expression vector having a gene encoding GSK-3 into an animal cell, and culturing the animal cell, and the like. The gene encoding GSK-3 is not particularly limited as long as it encodes GSK-3, but examples thereof include genes encoding GSK-3α or β derived from preferably a mammal, and more preferably rat, mouse, monkey or human, and specific examples include a gene having a base sequence set out in SEQ ID NO: 2.
   Examples of the peptide phosphorylated by GSK-3 include glycogen synthase, and examples of the glycogen synthase include e.g., a peptide having an amino acid sequence set out in SEQ ID NO: 3.
   The glycogen synthase can be obtained by a method which comprises introducing an expression vector having a gene encoding glycogen synthase into an animal cell, and culturing the animal cell, and the like. The gene encoding glycogen synthase is not particularly limited as long as it is a gene encoding glycogen synthase, but examples thereof include genes encoding glycogen synthase derived from preferably a mammal, and more preferably rat, mouse, monkey or human, and specific examples include a gene having a base sequence set out in SEQ ID NO: 4.
   Furthermore, a peptide having an amino acid sequence including a site which is phosphorylated by GSK-3 can be also used as the peptide phosphorylated by GSK-3 in the amino acid sequence of an eukaryotic initiation factor 2B (eIF2B) protein which participates in translation of a protein, and specifically, examples thereof include a peptide having an amino acid sequence set out in SEQ ID NO: 5.
   Exemplary method of the measurement of the activity of GSK-3 may include e.g., a method which comprises subjecting glycogen synthase or a peptide including a phosphorylation site of the enzyme to a phosphorylation reaction by GSK-3 using [γ-³³P]ATP as ATP that may be a donor of phosphoric acid in the presence of a subject substance, or in the absence of the subject substance; and measuring the amount of ³³P incorporated into the enzyme or peptide using a liquid scintillation counter or the like.
3. Nerve regenerating drug
   A nerve regenerating drug refers to an agent exhibiting an action to increase an intracerebral neuron by promoting neuropoiesis through directly acting on an intracerebral neural stem cell of a human or an animal.
   The nerve regenerating drug can be used as a therapeutic drug for neurological diseases accompanied by degeneration or damage of a nerve.
   Examples of the neurological disease include Parkinson' s disease, Alzheimer' s disease, Down' s disease, cerebrovascular disorder, cerebral stroke, spinal cord injury, Huntington's chorea, multiple sclerosis, amyotrophic lateral sclerosis, epilepsy, anxiety disorder, schizophrenia, depression, manic depressive psychosis and the like.
   The substance that inhibits the activity of GSK-3, or a pharmacologically acceptable salt thereof can be administered neat alone as a nerve regenerating drug, however, it is desired that to provide as any one of various medical drug formulations, in general. Furthermore, those medical drug formulations are used for animals and humans.
   The nerve regenerating drug of the invention may contain, as an active ingredient, a substance that inhibits the activity of GSK-3 or a pharmacologically acceptable salt thereof alone, or in a mixture with other arbitrary active ingredient for therapy. Further, those medical drug formulations are manufactured by an arbitrary method which has been well known in the technical field of physical pharmacy through mixing the active ingredient with one or more pharmacologically acceptable carriers.
   Route of administration for use is desirably a route which is the most effective for the therapy, and examples thereof include oral or parenteral such as e.g., intravenous route.
   Examples of dosage form include tablet, powder, granule, syrup, injection and the like.
   Liquid preparations such as e.g., syrups suited for oral administration can be produced using water, a saccharide such as sucrose, sorbit or fructose, a glycol such as polyethylene glycol or propylene glycol an oil such as sesame oil, olive oil or soybean oil, a preservative such as p-hydroxybenzoate ester, a flavor such as strawberry flavor or peppermint. Furthermore, the tablet, powder granule and the like can be produced using an excipient such as lactose, glucose, sucrose or mannit, a disintegrant such as starch or sodium alginate, a lubricant such as magnesium stearate or talc, a binder such as polyvinyl alcohol, hydroxypropyl cellulose or gelatin, a surfactant such as fatty acid ester, a plasticizer such as glycerin or the like.
   Formulation which is suitable for the parenteral administration preferably comprises a sterile aqueous agent containing an active compound that is isotonic with the blood of a recipient. For example, in cases of an injection, a solution for injection is prepared using a carrier comprising a saline solution, a glucose solution or a mixture of a saline solution and a glucose solution, and the like.
   Moreover, also in cases of these parenteral formulations, one or more auxiliary components as illustrated for the oral formulation selected from diluents, preservatives, flavors, excipients, disintegrants, lubricants, binders, surfactants, plasticizers and the like can be added.
   Amount of administration and frequency of administration of the substance that inhibits the activity of GSK-3 or a pharmacologically acceptable salt thereof may vary depending on the dosage form, age, body weight of the patient, nature or severity of the symptom to be treated, however, in cases of oral administration, 0.01 mg to 1 g, preferably 0.05 to 50 mg per one adult is generally administered once or several times per day. In cases of parenteral administration such as intravenous administration, 0.001 to 100 mg, preferably 0.01 to 10 mg per one adult is generally administered once or several times per day.
4. Agent for the promotion of neuropoiesis of neural stem cell
   An agent for the promotion of neuropoiesis of a neural stem cell refers to an agent that promotes neuropoiesis of a neural stem cell when it is brought into contact with a neural stem cell *in vivo* or *in vitro.*
   The neural stem cell is not particularly limited as long as it is a neural stem cell, but cerebral adult neural stem cells are preferred.
   The brain may be a brain of any animals, however, examples thereof include brains of preferably mammals, and more preferably, rat, mouse, monkey, human and the like.
   Although the substance that inhibits the activity of GSK-3 or a pharmacologically acceptable salt thereof can be used neat alone as an agent for the promotion of neuropoiesis of a neural stem cell, it is usually desired that the substance is provided as any kind of medical formulations. Further, those medical formulations are used for animals as well as humans.
   The agent for the promotion of neuropoiesis of a neural stem cell of the invention may contain the substance that inhibits the activity of GSK-3 or a pharmacologically acceptable salt thereof alone, as an active ingredient, or as a mixture with other arbitrary active ingredient for the therapy. Those medical formulations can be manufactured by a similar method to that for the formulation of the aforementioned nerve regeneration drug, and can be administered according to any method of administration which is similar thereto.
   The agent for the promotion of neuropoiesis of a neural stem cell of the invention can be used for a method of the manufacture of a neuron which comprises bringing the agent into contact with a neural stem cell *in vitro,* culturing the nerve cell to permit neogenesis of a neuron and collecting the neuron from the culture. When the agent for the promotion of neuropoiesis of a neural stem cell of the invention is used *in vitro,* it is preferred that the substance that inhibits the activity of GSK-3 or a pharmacologically acceptable salt thereof is used after dissolving in a solution capable of dissolving the substance or the salt. Examples of the solution include water, DMSO and the like.
5. Neuron obtained by culturing a neural stem cell in the presence of the agent for the promotion of neuropoiesis of the invention
   Through culturing an animal neural stem cell in the presence of the agent for the promotion of neuropoiesis of the invention, neuropoiesis of the neural stem cell can be actively promoted. The animal neural stem cell may be a neural stem cell of any animal, and examples thereof include neural stem cells derived from preferably mammals, more preferably rat, mouse, monkey and human. The neural stem cell may be a neural stem cell derived from a brain. Although the neural stem cell may be a cell derived from an animal with any week old or year old, however, preferably, it may be an adult neural stem cell.
   Examples of the process for obtaining an adult neural stem cell from an animal include a process which comprises excising a brain from an adult animal by a surgical procedure, preparing a brain cell crude extract, and concentrating adult stem cells from the crude extract, according to the process described in J. Neuroscinece, 19, 8487 (1999) and Genes & Develop., 10, 3129 (1996).
   Further, examples of the process for obtaining an adult neural stem cell from a human include a process which comprises collecting a tissue from lateral ventricle wall of a patient suffering from a neurological disease by biopsy, preparing a brain cell crude extract, and concentrating adult stem cells from the extract, according to the method described in Experimental Cell Research, 289, 378 (2003).
   When the adult neural stem cell is cultured in the presence of the agent for the promotion of neuropoiesis of the invention, it is preferred that the agent for the promotion of neuropoiesis is brought into action at a concentration of 100 nmol/l to 100 µmol/l per the adult neural stem cell at a density of approximately 1.8 x 10⁵ cells/cm². However, the lithium or a pharmacologically acceptable salt thereof is preferably brought into action at a concentration of 100 µmol/l to 10 mmol/l. Neuropoiesis can be promoted through bringing the adult neural stem cell into contact with the agent for the promotion of neuropoiesis of the invention, and allowing static culture at 37°C in an atmosphere of 5% CO₂ for 4 to 14 days while conducting medium replacement of total amount or partial amount every three days.
   The medium for use in the culture of the adult neural stem cell may be any medium as long as it does not prevent the promotion of neuropoiesis, however, it is preferred that a DMEM/F12 medium (manufactured by Invitrogen Corporation) containing a 1% N2 supplement (manufactured by Invitrogen Corporation) or the like is used.
   The neuron obtained by the aforementioned culture can be used for the therapy of the neurological disease by recovering from the culture medium and transplanting to an impaired part of a patient suffering from the neurological disease by a surgical procedure. Examples of the neurological disease include Parkinson's disease, Alzheimer's disease, Down's disease, cerebrovascular disorder, cerebral stroke, spinal cord injury, Huntington's chorea, multiple sclerosis, amyotrophic lateral sclerosis, epilepsy, anxiety disorder, schizophrenia, depression, manic depressive psychosis and the like.
6. Method of evaluation of the nerve regenerating drug of the invention
   Possible therapy of a neurological disease by the nerve regenerating drug of the invention through regeneration of a neuron *in vivo* can be verified by the following method.
   The aforementioned nerve regenerating drug of the invention is administered to an experimental animal such as rat or monkey. Although the experimental animal may be a healthy animal without injury, an animal with a brain injured by a method such as ischaemia, administration of 6-hydroxydopamine (6-OHDA) or administration of kainic acid is preferred because neuropoiesis can be effectively observed by inflicting an ischemic injury of hippocampus [Cell, 110, 429 (2002)]. The route of administration may be oral, intraoral, subcutaneous, intramuscular, intravenous or intraventricular route. In connection with amount of administration and method of administration, for example, administration in an amount of 100 µg to 10 mg, preferably 500 µg to 500 ng per 1 kg of the body weight, preferably once or several times per day is conducted. In the case of parenteral administration such as intravenous administration, administration in an amount of 10 µg to 1 mg, preferably 100 µg to 100 ng per 1 kg of the body weight, preferably once or several times per day is conducted.
   The neuron yielded by neogenesis can be detected by the following method.
   After administering a retroviral vector capable of expressing a gene having an ability to label a cell such as Green Fluorescent Protein (GFP) or beta galactosidase, or bromodeoxyuridine (BrdU) which can label proliferating cells to the experimental animal concurrently with, prior to or following the initial administration of the substance, the substance is administered once or several times per day, and the animal is kept for 10 to 20 days. Thereafter, brain of the experimental animal is excised, and the cerebral frozen section is prepared. The section is observed using a fluorescence microscope, and for example, in the case where BrdU is used as the agent that labels proliferating cells, number of BrdU positive cells and ratio of the number of BrdU positive cells to the number of Tuj1 positive cells which is a neuron marker are compared to those for a negative control per unit area.
   According to the method as described above, the nerve regenerating drug of the invention can be evaluated for the neuropoiesis promoting action and therapeutic effect for a neurological disease.
7. Method of evaluation of the agent for the promotion of neuropoiesis of the invention
   ANSC-7 cells which can be obtained by the method described in Reference Example 2 are plated at 1.8 x 10⁵ cells per well in a 12-well culture dish which was coated with polyornithine and laminin and was filled with a DMEM/F12 medium containing 1 ml of a 1% N2 supplement (manufactured by Invitrogen Corporation) and 2 0 ng/ml FGF-2 (manufacturedbyPeproTech Inc.), and incubated overnight. Entire culture fluid is changed into a DMEM/F12 medium containing 0.5% fetal bovine serum and a 1% N2 supplement without including FGF2 (manufactured by Invitrogen Corporation, hereinafter, referred to as differentiation inducing medium) to induce differentiation. Upon the induction, the substance that inhibits the activity of GSK-3 serially diluted in PBS (manufactured by Invitrogen Corporation) or DMSO in the range of from 0.01 mmol/l to 5 mol/l is added thereto in 1/1000 volume. As a negative control, the same volume of PBS or DMSO is added.

After changing the culture fluid into the differentiation inducing medium supplemented with each substance that inhibits the activity of GSK-3 every three days, and inducing differentiation for 6 days, it is replaced with a 15% neutral buffered formalin solution (Wako Pure Chemical Industries, Ltd.) to allow fixation for 20 min. Thereafter, washing with PBS containing 0.3% TritonX-100 (manufacturedbyNacalai Tesque, Inc.) for 5 min is repeated three times. Next, after blocking the cells using a 10% goat fetal serum diluted in PBS (manufactured by DAKO Corporation) for 2 hrs, a reaction is allowed with a mouse anti-Tuj1 (β tubulin isotype III) antibody (manufactured by Sigma-Aldrich Corporation) diluted 1000 times in PBS as a primary antibody at 4°C for 16 hrs. Thereafter, washing with PBS containing 0.3% Triton X-100 for 5 min is repeated three times.

Next, a reaction is allowed with Alexa Fluor 488 conjugate goat anti-mouse IgG antibody (manufactured by Molecular Probes, Inc.) diluted 1000 times as a secondary antibody at room temperature for 2 hrs. At the same time, Bisbenzimide H 33342 Fluorochrome, Trihydrochloride (manufactured by CALBIOCHEM, hereinafter, described as H33342) is added thereto to give the final concentration of 1 µg/ml to stain the nucleus. After immersing in PBS, observation with an inverted fluorescence microscope (manufactured by Nikon Corporation) is conducted, and the number of Tuj1 positive cells per 2.44 mm² is counted.

Hereinafter, Experimental Example relating to a neuropoiesis promoting action of the agent for the promotion of neuropoiesis of the invention is demonstrated. Experimental Example 1: Promotion of neuropoiesis with lithium chloride (1)

According to the method described in the above section 7, when the differentiation of the ANSC-7 cells is induced, lithium chloride or sodium chloride (both manufactured by Nacalai Tesque, Inc.) dissolved in PBS to give 0.01, 0.1, 1 or 3 mol/l is added to the medium containing the ANSC-7 cells in 1/1000 volume of the culture fluid, and the number of neurons on day 6 following the induction of differentiation was analyzed. As a consequence, the number of Tuj1 positive neurons became 1.1, 1.3, 1.8 and 2.1 times with lithium chloride at the final concentration of 0.01, 0.1, 1 and 3 mmol/l, respectively (significantly different with lithium chloride of 1 mmol/l or greater), exhibiting increase depending on the concentration of lithium chloride. Furthermore, total number of H33342 positive cells with 3 mmol/l lithium chloride was 1.1 times compared to the control without lithium chloride, exhibiting no significant difference. As in the foregoing, it was revealed that lithium chloride exhibits a neuropoiesis promoting action of an ANSC-7 cell. Moreover, in the case of sodium chloride that is a negative control, the number of Tuj1 positive neurons is 1.0, 1.1, 1.2, 1.2 times at the final concentration of 0.01, 0.1, 1, 3 mmol/1, respectively, all of which not being significantly different. Thus, increase in the number of neogenetic neurons is believed not being the effect due to salt concentration or chloride ion, but being the effect of lithium. Experimental Example 2: Promotion of neuropoiesis by lithium chloride (2)

In order to elucidate if the neuropoiesis promoting action on the ANSC-7 cell results from the induction of differentiation of BDNF and Bcl-2, whether the expression of BDNF and Bcl-2 is promoted by lithium or not was analyzed with semiquantitative RT-PCR.

The ANSC-7 cells were plated in 7 wells in total of a 6-well culture dish which had been coated with polyornithine and laminin and had been filled with a DMEM/F12 medium containing 2 ml of a 1% N2 supplement and 20 ng/ml FGF-2 to give 4.5 x 10⁵ cells per well, and incubated overnight. Total RNA was obtained from cells in one well using RNeasy mini kit (manufactured by QIAGEN), according to the accompanying protocol. Entire culture medium in the remaining 6 wells was changed into the differentiation inducing medium to induce the differentiation. To two wells among those was added 3 mol/l of lithium chloride in 1/1000 volume of the medium, and to other two wells was added 1 mol/l of lithium chloride in 1/1000 volume of the medium. Equal volume of PBS wad added to the remaining two wells as a control.

After 24 hrs passed from initiation of the induction of differentiation, cells were harvested from each well to which lithium chloride at each concentration was added. Total RNA was obtained from the cells according to a similar method to that described above, and from the remaining cells was obtained total RNA on day 6 following initiation of the induction of differentiation. To the total RNA each in,an amount of 5 µg obtained as described above were added 10 µl of 5 x DNAse buffer, 0.5 µl of an RNase inhibitor (40 U/µl), 2.5 µl of RNase-free DNaseI (1. U/µl) (all of which manufactured by Promega Corporation), respectively, and sterile water was added thereto to give the total volume of 50 µl. After allowing a reaction at 37°C for 30 min, the mixture was subjected to a phenol/chloroformtreatment followed by ethanol precipitation.

To the total RNA each in an amount of 1 µg which had been subjected to the DNase treatment was added 1 µl of 0.5 µg/µl oligo(dT)12-18 primer, and sterile water was added thereto to give the total volume of 12 µl. After heating at 65°C for 10 min, the mixture was rapidly cooled on ice, and thereto were added 4 µl of 5 x synthesis buffer (manufactured by Invitrogen Corporation), 1 µl of 10 mmol/l dNTP mix, 2 µl of 0.1 mol/l DTT, 1 µl of 200 U/µl Superscript II RT (manufactured by Invitrogen Corporation) to allow a reaction at 42°C for 50 min. After heating the mixture at 90°C for 5 min, it was left to stand on ice for 10 min.

Next, 1 µl of RNaseH (2 U/µl) (manufactured by Invitrogen Corporation) was added thereto, and a reaction was allowed at 37°C for 20 min. Accordingly, a cDNA was produced by adding sterile water thereto to give the total volume of 200 µl.

In a similar manner, a cDNA was prepared from rat cerebral total RNA for use as a positive control. To 1 µl of the cDNA were added 2 µl of a 10 µmol/l primer set, 1 µl of DMSO (manufactured by Nacalai Tesque, Inc.), 2 µl of 10 x ExTaq buffer, 1.6 µl of dNTPmix, 0 .1 µl of ExTaq (all of which manufactured by TAKARA BIO INC.), and each cDNA fragment was amplified after treating at 94°C for 1 min, through repeating 27 cycles of PCR for Bcl-2 amplification and 35 cycles of PCR for BDNF amplification of: at 94°C for 1 min, at 60°C for 1 min, and at 74°C for 1 min using a thermal cycler. For the amplification of Bcl-2, synthetic DNAs consisting of the base sequence set out in SEQ ID NOs : 6 and 7 were used; and for amplification of BDNF, synthetic DNAs consisting of the base sequence set out in SEQ ID NOs: 8 and 9 were used as a primer set.

The amplified DNA was electrophoresed on a 1.8% agarose (manufactured by Nacalai Tesque, Inc.) gel, and after staining with ethidium bromide (Manufactured by Nacalai Tesque, Inc.), detection was executed with a transilluminator (manufactured by Toyobo Co., Ltd.). Strength of the Bcl-2 band did not vary depending on the difference in concentration of lithium chloride both 24 hrs and 6 days following initiation of the differentiation. Expression of BDNF was not found regardless of the difference in concentration of lithium chloride.

From the results described above, it was suggested that the neuropoiesis promoting activity by lithium chloride was not the result of suppression of cell death via Bcl-2 and BDNF, but lithium chloride actively promoted neuropoiesis.

### Experimental Example 3: Promotion of neuropoiesis by lithium chloride (3)

In order to elucidate if the neuropoiesis promoting action by lithium chloride results from the increase in newly generated cells due to suppression of apoptosis, or from the active induction of differentiation of a neuron, the effect of apoptosis suppression against ANSC-7 cells by lithium was analyzed.

According to the method described in the above section 7, lithium chloride was added to give the final concentration of 3 mmol/l into the medium containing ANSC-7 cells followed by culture for 6 days. ANSC-7 cells thus cultured after adding lithium chloride, and ANSC-7 cells cultured after adding PBS as a control were allowed to a reaction using an *in situ* cell death detection kit, fluorescein (manufactured by Roche Diagnostics K.K.) according to the attached protocol. The cells were observed using an inverted fluorescence microscope (manufured by Nikon Corporation), and the number of apoptotic cell was counted per 2.44 mm².

Consequently, the number of apoptosis positive cells was 1.0 time through adding lithium chloride, which results in no alteration. Therefore, itwas elucidated that lithium chloride did not suppress the apoptosis of ANSC-7 cells. Accordingly, the neuropoiesis promoting action by lithium was revealed to result from active promotion of neuropoiesis.

### Experimental Example 4: Antagonistic action for neuropoiesis promoting action of insulin and forskolin, and lithium

Antagonistic action for neuropoiesis promoting action of insulin and forskolin known as having a neuropoiesis promoting action, and lithium was analyzed.

According to the method described in the above section 7, insulin was added to give the concentration in the medium of 5 µg/ml or 25 µg/ml into the medium upon the induction of differentiation of the ANSC-7 cells, accompanied by adding lithium chloride to the medium containing each concentration of insulin to give the final concentration of 0, 1 and 3 mmol/l. Then, induction of the differentiation was allowed for 6 days.

Consequently, rate of increase in neurons caused by 3 mmol/l of lithium chloride was 0.70 time lower in the case of coexistence with 25 µg/ml of insulin compared to the case of coexistence with 5 µg/ml of insulin, exhibiting a significant decrease. Therefore, antagonism of insulin and lithium was revealed.

Furthermore, according to a similar method to that described above, forskolin was added to the medium in stead of insulin to give the final concentration of 0, 1 and 5 µmol/l, and the rate of increase in neurons in the case of coexistence with lithium chloride at a final concentration of 0 or 3 mmol/l was calculated.

Consequently, rate of increase in neurons caused by 1, 5 µmol/l of forskolin under the condition without lithium chloride was 1.9, 2.2 times, respectively, whereas in the case of coexistence with 3 mmol/l of lithium chloride, it was 1.2, 1.1 times, leading to no increase. Therefore, lithium chloride and forskolin were revealed to antagonize for the neuropoiesis promoting action.

As a target molecule of lithium, GSK-3, inositol-1-phosphate phosphatase and inositol-polyphosphatase have been known[Nature,417,292-295 (2002)]. Furthermore, insulin and forskolin have been known to indirectly inhibit the activity of GSK-3 [Mol. Cell. Biol., 19, 4989-5000 (1999)]. Because the target molecule of lithium, insulin and forskolin for the neuropoiesis promoting action is assumes to be common, it was suggested that lithium promoted neuropoiesis of a neural stem cell by inhibiting the activity of GSK-3.

### Experimental Example 5: Promotion of neuropoiesis by SB-216763 that is a selective inhibitor of GSK-3

SB-216763 known as a selective inhibitor of GSK-3 synthesized according to the method described in Reference Example 1 [Chem. Biol., 7, 793-803 (2000)] was dissolved in DMSO to give 0.1 and 0.33 mmol/l, which was added to a medium containing ANSC-7 in 3/1000 volume thereof upon induction of the differentiation of ANSC-7 cells, according to the method described in the above section 7. Thus, the number of neurons on day 6 following induction of the differentiation was measured.

Consequently, the number of Tuj1 positive neurons became 1.2 times and 1.8 times by SB-216763 at the final concentration of 0.3 and 1.0 µmol/l, respectively, exhibiting significant increase in a concentration dependent manner. Therefore, it was revealed that neuropoiesis can be promoted by a compound having the activity to selectively inhibit GSK-3.

As in the foregoing, the substance having the activity to selectively inhibit GSK-3 was suggested to be an agent for the promotion of neuropoiesis of a neural stem cell, as well as a medicament for nerve regenerative therapy.

### Experimental Example 6: Promotion of neuropoiesis by 9-bromo-7,12-dihydro-indolo[3,2-d][1]benzazepin-6(5H)-one (Kenpaullone) that is an inhibitor of GSK-3

According to a similarmethod to that shown in Experimental Example 1, Kenpaullone which has been known as an inhibitor of GSK-3 and cyclin-dependent kinase (hereinafter, referred to as CDK) [Biochem. J., 371, 199-204 (2003), manufactured by CALBIOCHEM] was dissolved in DMSO to give 0.5, 2 and 5 mmol/l. The DMSO solution in the 1/1000 volume of the culture fluid was added to the medium containing the ANSC-7 cells. Thus, the number of Tuj 1 positive neurons on day 6 following induction of the differentiation was analyzed. As a negative control, an equal volume of DMSOwas added. Moreover, in a similarmanner, Roscovitine which has been known as a CDK inhibitor and which hardly inhibits GSK-3 [Biochem. J., 371, 199-204 (2003), manufactured by Sigma-Aldrich Corporation] was dissolved to give 2, 5 and 10 mol/l in DMSO. The DMSO solution in the 1/1000 volume of the culture fluid was added to the medium containing ANSC-7 cells, and thus, the number of Tuj1 positive neurons on day 6 following induction of the differentiation was analyzed.

Consequently, the number of Tuj1 positive neurons was 1.3 times with Kenpaullone at the final concentration of 0.5 µmol/l, 2.7 times at 2 µmol/l and 3.7 times at 5 µmol/l, in comparison with the negative control, exhibiting significant increase in a concentration dependent manner of Kenpaullone. On the other hand, it was 1.0 time with Roscovitine at the final concentration of 2 µmol/l, 1.2 times at 5 µmol/l, and 1.1 times at 10 µmol/l, in comparison with the negative control, exhibiting no significant increase, respectively. Thus, no increase in the number of Tuj1 neurons caused by Roscovitine was found. Therefore, it was revealed that Kenpaullone exhibited a neuropoiesis promoting action, and that the action is not caused by an inhibitory activity on CDK of Kenpaullone, but is caused by an inhibitory activity on GSK-3.

As in the foregoing, it was suggested that any compound having the activity to inhibit GSK-3 may be an agent for the promotion of neuropoiesis of a neural stem cell, as well as a medicament for nerve regenerative therapy of a neurological disease, not being limited to lithium and SB-216763.

### Experimental Example 7: Suppression of neuropoiesis by high expression of a GSK-3β gene

Because high expression of GSK-3β is found in the brain of a patient suffering from Alzheimer's disease [J. Neuropathol. Exp. Neurol., 56, 70-78 (1997)], influences of the high expression of GSK-3β on differentiation of a neuron of an adult neural stem cell were studied using a retroviral vector in order to elucidate the relationship between the caue of onset of Alzheimer's disease and GSK-3β.

First, a cDNA encoding a wild type rat GSK-3β gene was prepared as follows using a cDNA derived from a rat brain described in Experimental Example 2 as a template. To 2.5 µl of the template cDNA were added 3 µl of a 10 µmol/l primer set including the sequences set out in SEQ ID NOs: 10 and 11 (manufactured by Proligo LLC), 5 µl of 10 x PCR buffer for KOD -plus-, 5 µl of 2 mmol/l dNTPs, 2 µl of 25 mmol/l MgSO₄, 1 µl of KOD -plus- DNA polymerase (all of which manufactured by Toyobo Co., Ltd.) and 31.5 µl of sterile water, and then amplification of the cDNA fragment was allowed by incubating at 94°C for 2 min followed by repeating 25 cycles of: at 94°C for 15 sec, at 60°C for 30 sec, and at 68°C for 1 minute and 20 sec using a thermal cyler.

On the other hand, a cDNA encoding a mutated rat GSK-3β gene having a mutation of a lysine residue at position 85 into an arginine residue, which mutation may lead to loss of the kinas activityof GSK-3β [Proc. Nat. Acad. Sci. USA, 92, 8498-8502 (1995)] was similarly prepared as follows.

With 2.5 µl of a cDNA encoding a wild type rat GSK-3β gene as a template, amplification of a cDNA having a partial length of 5' end of the mutated rat GSK-3β was allowed using a primer set including the sequence set out in SEQ ID NOs: 10 and 12, while amplification of a cDNA having a partial length of 3' end of the mutated rat GSK-3β was allowed using a primer set including the sequence set out in SEQ ID NOs: 11 and 13. With a mixture of respective cDNAs having the partial length as a template, a cDNA having the full length of the mutated rat GSK-3β was amplifiedusing a primer set including the sequence set out in SEQ ID NOs: 10 and 11.

Each of the amplification reaction fluid of wild type and mutant was subjected to a treatment with phenol/chloroform followed by precipitation through adding ethanol. The product was purified with a QIAquick PCR purification kit (manufactured by QIAGEN) according to the attached protocol.

Subsequently, to 10 µg of a pCLNCX plasmid vector (manufactured by IMGENEX Corporation) were added 10 µl of 10 x M buffer and 5 µl of HindIII (manufactured by TAKARA BIO INC.), and thereto was added sterile water to give 100 µl. The reaction was allowed at 37°C for 12 hrs. After subjecting the reaction fluid to a treatment with phenol/chloroform, the DNA was precipitated by adding ethanol, and was dissolved in 32 µl of sterile water. To the DNA solution were added 4 µl of 10 x Blunting buffer and 4 µl of KOD DNApolymerase to allow a reaction at 72°C for 2 min to achieve blunting. After treating the reaction fluid with phenol/chloroform, thereto was added ethanol to precipitate the DNA. After dissolving the DNA in 43 µl of sterile water, thereto were added 5 µl of 10 x Alkaline Phosphatase buffer and 2 µl of Alkaline Phosphatase (all of which manufactured by TAKARA BIO INC.), and the reaction was allowed at 65°C for 30 min. After treating the reaction fluid with phenol/chloroform, thereto was added ethanol to precipitate the DNA which was dissolved in sterile water.

With 3 µg of thus cleaved pCLNCX plasmid vector was mixed 3 µg of the cDNA encoding the wild type or mutant rat GSK-3β gene prepared as described above, to which sterile water was added to give 4 µl. Thereto was added 4 µl of ligation high (manufactured by Toyobo Co. , Ltd.) and the reaction was allowed at 16°C for 12 hrs. The product was transformed into E. coli DH5α competent cell (manufactured by TAKARA BIO INC.). An ampicillin resistant colony was cultured in a liquid LB medium according to a conventional method, and a pCLNC-GSK3β plasmid DNA and a pCLNC-GSK3β (K85R) plasmid DNA were prepared using a DNA Endofree Plasmid Maxi Kit (manufactured by QIAGEN) according to the attached protocol.

Next, a viral vector was produced as follows, and a function relating to the differentiation of a neuron of ANSC-7 cells was analyzed. First, 15 µg of pCLNC-GSK3β, pCLNC-GSK3β (K85R), or pCLNCX plasmid vector DNA as a negative control, and 5 µg of each pMD.G plasmid vector DNA (supplied from Salk Institute, USA) were dissolved in 2 ml of a D-MEM high glucose medium (manufactured by Invitrogen Corporation), and transfection was carried out using a Transfast transfection reagent (manufactured by Promega Corporation) according to the attached protocol into 293gp cells (supplied from Salk Institute, USA) which had been prepared on the preceding day.

On day 3 following the transfection, the culture supernatant was filtrated through a 0.45 µm filter (manufactured by Millipore Corporation) to recover a solution containing the viral vector. The viral vector solution was transferred into a polyaroma tube (manufactured by Hitachi Koki Co., Ltd.), and centrifuged using an ultracentrifuge (manufactured by Hitachi Koki Co. , Ltd.) at 50,000 x g, 18°C for 90 min. The supernatant was eliminated, and the precipitated viruses were suspended in a DMEM/F12 medium containing a 1% N2 supplement and 20 ng/ml FGF-2 and 8 µg/ml hexadimethrin bromide (manufactured by Sigma-Aldrich Corporation). According to a similar method to that shown in Experimental Example 1, 1.8 x 10⁵ ANSC-7 cells per well were plated on a 12-well culture dish which had been coated with polyornithine and laminin, and left to stand overnight. Culture supernatant was removed from the culture, and thereto was added the viral suspension to allow infection by the culture in an incubator at 37°C, at the concentration of CO₂ of 5% for 2 hrs. Subsequently, the entire culture fluid was changed to a differentiation inducing medium to initiate the induction of differentiation. Similarly to Experimental Example 1, the number of Tuj 1 positive neurons on day 6 following induction of the differentiation was analyzed.

Consequently, compared to ANSC-7 cells infected with the retrovirus which had been produced from pCLNCX as a negative control and allowed differentiation, the number of neogenetic neurons was significantly decreased by 33% in the cells infected with a retrovirus which had been produced from pCLNC-GSK3β to permit high expression of GSK3β and allowed differentiation. On the other hand, in the cells infected with a retrovirus which had been produced from pCLNC-GSK3β (K85R) to permit high expression of GSK3β (K85R) having no kinase activity and allowed differentiation, the number of neogenetic neurons was significantly greater by 34% than in the cells in which high expression of wild type GSK-3β was allowed, which was almost the same level as the case in which the retrovirus which had been produced from pCLNCX as a negative control was infected and allowed differentiation. Therefore, it was revealed that neuropoiesis was suppressed by the kinase activity of GSK-3β.

Accordingly, a possibility of onset of Alzheimer's disease through suppression of neuropoiesis due to promotion of phosphorylation of a target molecule which results from high expression of GSK-3β, thereby causing suppression of cerebral autoregeneration ability, was suggested. Thus, it was indicated that GSK-3 inhibitors can be a therapeutic drug for neurological diseases such as e.g., Alzheimer's disease. Experimental Example 8: Suppression of neuropoiesis by an associated beta amyloid peptide, and relief of suppression by GSK-3 inhibitor (1)

A beta amyloid peptide (hereinafter, referred to as Aβ) is a principal component constituting senile plaques, and it is a substance which has been believed to be a cause of Alzheimer' s disease [Proc. Nat. Acad. Sci., USA, 98, 11039-11041 (2001)]. Aβ [1-40] (manufactured by BIOSOURCE) was dissolved in 0.1% (v/v) trifluoroacetic acid (Manufactured by Nacalai Tesque, Inc.) to give 10 mg/ml, and incubated at 25°C for 1 hour. Thereafter, the solution was diluted in PBS to give 0.5 mg/ml. The diluted solution was incubated at 25°C for 24 hrs to allow the formation of an associated product [J.Biol.Chem., 276, 42027-42034 (2001)]. According to a similar method to that shown in Experimental Example 1, the associated product was added to the medium to give the final concentration of 0.1 mg/ml upon induction of the differentiation of the ANSC-7 cell, while an equal volume of PBS was added for the negative control. On day 2 and day 4 following the induction of differentiation, the entire medium was changed to the differentiation inducing medium alone, respectively, and the number of neurons was measured on day 6 following induction of the differentiation.

Consequently, the number of Tuj1 positive neurons significantly decreased by 78% through the addition of the associated Aβ. Therefore, a possibility of on set of Alzheimer's disease that is one of neurological diseases, through suppression of neuropoiesis due to the associated Aβ, thereby causing suppression of cerebral autoregeneration ability, was suggested. Experimental Example 9: Suppression of neuropoiesis by an associated beta amyloid peptide, and relief of suppression by GSK-3 inhibitor (2)

In order to elucidate if suppression of neuropoiesis by associated Aβ is caused by decrease in the number of neogenetic cells resulting from promotion of apoptosis, or if it is caused by suppression of neuronal differentiation, the effect of promoting apoptosis on an ANSC-7 cell exerted by associated Aβ was analyzed.

According to a similarmethod to that shown in Experimental Example 8, ANSC-7 cells subjected to induction of differentiation for 6 days by a differentiation inducing medium containing the associated Aβ at the final concentration of 0.1 mg/ml, and ANSC-7 cells subjected to induction of differentiation by a medium containing PBS as a control were allowed to react using an *in situ* cell death detection kit, fluorescein (manufactured by Roche Diagnostics K.K.) according to the attached protocol. The cells were observed using an inverted fluorescence microscope (manufactured by Nikon Corporation), and the number of apoptotic cells per 2.44 mm² was counted.

Consequently, the number of apoptotic positive cells increased through the addition of the associated Aβ by 14%, but any significant difference was found. Therefore, it was revealed that decrease in the number of neogenetic neurons due to the associated Aβ was primarily caused by suppression of neuronal differentiation rather than promotion of apoptosis. Experimental Example 10: Suppression of neuropoiesis by an associated beta amyloid peptide, and relief of suppression by GSK-3 inhibitor (3)

According to a similarmethod to that shown in Experimental Example 8, lithium chloride at a final concentration of 3 mmol/l, or Kenpaullone at a final concentration of 2 µmol/l was added to a differentiation inducing medium containing the associated Aβ at a final concentration of 0.1 mg/ml upon inducing differentiation of the ANSC-7 cell, and the number of Tuj1 positive neurons on day 6 following inducing differentiation was analyzed.

As a result, by adding lithium chloride or Kenpaullone, the number of Tuj1 positive neurons significantly increased by 73%, 400%, respectively, in comparison with the case of the associated Aβ alone.

Therefore, it was revealed that a GSK-3 inhibitor exhibited an action to release suppression of neuropoiesis due to the associated Aβ.

As in the foregoing, it was indicated that compounds having the activity to selectively inhibit GSK-3 could be a nerve regenerating drug for neurological diseases such as Alzheimer' s disease, and that the compounds could be agent for the promotion of neuropoiesis of a neural stem cell. °

### Experimental Example 11: Promotion of neuropoiesis by Indirubin-3'-monoxime, an inhibitor of GSK-3

According to a similarmethod to that shown in Experimental Example 1, indirubin-3'-monoxime that has been known as an inhibitor of GSK-3 [J. Biol. Chem., 276, 251-260 (2001), manufactured by Sigma-Aldrich Corporation] was dissolved in DMSO to give 1 mmol/l, and the DMSO solution in the 1/1000 volume of the culture fluid was added to a medium containing ANSC-7 cells. Thus, the number of Tuj1 positive neurons on day 6 following induction of the differentiation was analyzed. As a negative control, an equal volume of DMSO was added. Consequently, under the condition of the addition of indirubin-3'-monoxime at a final concentration of 1 µmol/l, the number of Tuj1 positive neurons significantly increased 1.4 times in comparison with the negative control. Therefore, it was revealed that indirubin-3'-monoxime exhibited a neuropoiesis promoting action.

From the foregoings, compounds having the activity to inhibit GSK-3, not being limited to lithium, SB-216763 and Kenpaullone, can be an agent for the promotion of neuropoiesis of a neural stem cell, as well as a medicament for nerve regenerative therapy of a neurological disease.

### Experimental Example 12 : Promotion of neuropoiesis by specific siRNA for GSK-3

A short interference RNA (siRNA) specifically knocks down a gene [Nature, 411, 494-498 (2001)]. In order to further elucidate possible the capability to promote the neuropoiesis through specifically inhibiting GSK-3 which is expressed in an adult neural stem cell, a chemically synthesized siRNA that is specific for GSK-3β was introduced into an ANSC-7 cell, and studied for effects on neuropoiesis.

First, a knockdown effect of two kinds of chemically synthesized siRNA on GSK-3β in an ANSC-7 cell was studied as follows. Introduction of the siRNA into an ANSC-7 cell was carried out using Rat NSC Nucleofector™ Kit (manufactured by Amaxa) . Double stranded siRNA-B1 having SEQ ID NO: 14 and 15, or double stranded siRNA-B2 having SEQ ID NO: 16 and 17 (manufactured by Dharmacon, Inc.) in an amount of 150 pmol per 1 x 10⁶ of ANSC-7 cells was introduced according to the attached protocol. As a negative control, Non-specific Control Duplex IX (47% GC Content) siRNA (manufactured by Dharmacon, Inc.) was introduced. Immediately after the introduction, they were suspended in 5 ml of a differentiation inducing medium, and seeded on a 6 cm culture dish coated with polyornithine and laminin. At 48 hrs following the seeding, the cells were recovered by dissolving in an aqueous solution containing 1% Nonidet P-40, 50 mM Tris-HCl (pH7. 4), 50 mM NaCl (all of which manufactured by Nacalai Tesque, Inc.), and one tablet of Complete mini, EDTA free (manufactured by Roche Diagnostics K.K.) per 10 ml. The amount of GSK-3β was detected by a Western blotting method after separating by an SDS-PAGE process according to a conventional method. For the detection, an anti-GSK-3 antibody that recognized GSK-3α and β (manufactured by Sigma Corporation) was used. As a result, the cells to which two kinds of siRNAs for GSK-3β were introduced both exhibited a decreased amount of GSK-3β by 90% or greater in comparison with the negative control, but the amount of GSK-3α was unchanged. Therefore, it was revealed that each siRNA could specifically knock down GSK-3β.

Subsequently, after introducing those two kinds of siRNAs into ANSC-7, the cells were immediately seeded at 1.8 x 10⁵ cells on a 12-well culture dish which had been coated with polyornithine and laminin and filled with 1 ml of a differentiation inducing medium to induce the differentiation. According to a similar method to that shown in Experimental Example 1, the number of Tuj1 positive neurons on day 6 following induction of the differentiation was analyzed. As a result, the number of Tuj1 positive neurons increased 2.5 times in cells to which siRNA-B1 was introduced in comparison with the negative control, while in cells to which siRNA-B2 was introduced, the number increased 1.9 times. Therefore, it was revealed that the siRNA for GSK-3β siRNA exhibited a neuropoiesis promoting action.

From the foregoing, it was suggested that compounds having the activity to inhibit GSK-3, and nucleic acids including the siRNA could be an agent for the promotion of neuropoiesis of a neural stem cell, as well as a medicament for nerve regenerative therapy of neurological diseases.

### Reference Example 1: Step 1 of synthesis of 3-(2,4-dichlorophenyl)-4-(1-methylindole-3-yl)-1H-pyrrole-2,5-dione (SB-216763): Synthesis of 3-indole glyoxylic acid, methyl ester

Commercially available 3-indole glyoxylic acid (9.55 g) was suspended in methylene chloride (300 mL), and cooled on ice. Thereafter, to the suspension was added oxalyl chloride (8.8 mL), followed by stirring at 20°C for 20 hrs . The reaction fluid was cooled on ice, and after adding methanol (190 mL) thereto, the reaction fluid was stirred at 25°C for 1 hour. To the reaction fluid were added water and methylene chloride, and thus deposited crystals were filtrated, followed by washing of the crystals with methylene chloride. The crystals were dried under a reduced pressure to obtain 3-indole glyoxylic acid, methyl ester (7.07 g, 69%).

### Step2:Synthesisof2-(1-methylindole-3-yl)-2-oxoacetic acid, methyl ester

The 3-indole glyoxylic acid, methyl ester (5.88 g) obtained in the step 1 was dissolved in N,N-dimethylformamide (180 mL), and thereto was added hydrogenated sodium (dispersed in oil at 60%, 1.4 g) in small portions while stirring at 0°C. After stirring the reaction mixture for 1 hour, methyl iodide (1.2 mL) was added thereto, and stirred at 20°C for 20 hrs. After adding ice-cooled water to the reaction fluid, the pH value was adjusted with 1 mol/L hydrochloric acid to become 5. Thus deposited crystals were filtrated, and washed with water. The crystals were dried under a reduced pressure to obtain 2-(1-methylindole-3-yl)-2-oxoacetic acid, methyl ester (1.96 g, 33%).

### Step 3: 2,4-dichlorophenylacetic acid amide

Commercially available 2,4-dichlorophenylacetic acid (12.4 g) was dissolved in methylene chloride (350 mL), and thereto was added oxalyl chloride (10.6 mL) under ice-cooling, followed by stirring at 20°C for 20 hrs. The reaction fluid was concentrated under a reduced pressure, and the resulting residue was dissolved in methylene chloride (100 mL). This solution was added dropwise to an ice-cooled 28% aqueous ammonia solution (250 mL), and methylene chloride was distilled off under a reduced pressure. Thus deposited crystals were filtrated, and washed with water. The crystals were dried under a reduced pressure to obtain 2,4-dichlorophenylacetic acid amide (11.14 g, 90%).

### Step 4: Synthesis of SB-216763

Tert-butoxy potassium (0.5 g) was dissolved in tetrahydrofuran (35 mL), and thereto were added under ice-cooling the 2-(1-methylindole-3-yl)-2-oxoacetic acid, methyl ester (0.4 g) obtained in the step 2, and then the 2,4-dichlorophenylacetic acid amide (0.3 g) obtained in the step 3, followed by stirring at the same temperature for 3 hrs. After adding water to the reaction fluid, extraction with ethyl acetate was carried out. The organic layer was sequentially washed with a saturated aqueous sodium bicarbonate solution, and saturated salt solution. After drying over anhydrous magnesium sulfate, the crystals were washed with ethanol. The crystals were dried under a reduced pressure to obtain SB-216763 (390 mg, 70%).
¹H-NMR (CDCl₃) δ (ppm): 3.89(s, 3H), 6.41(d, J = 8.1 Hz, 1H), 6.80(t, J=7.1 Hz, 1H), 7.15(t, J = 7.1 Hz, 1H), 7.32-7.38(m, 3H), 7.49(s, 1H), 8.01(s, 1H), 10.94(s,1H)
Elementary analysis: theoretical value (C: 61.5, H: 3.3, N: 7.6), measured value (C: 61.3, H: 3.5, N: 7.4)

### Reference Example 2: Isolation and culture of adult neural stem cell from rat brain

After putting a 7-weeks old Sprague Dawley rat to sleep by etherization, decapitation was performed, and the brain was excised through incision of skull from the calvaria. A tissue which includes a part surrounding the cerebral ventricle was isolated from the excised brain under a microscope using scissors and tweezers for ophthalmic use. After dividing the tissue which includes the part surrounding the cerebral ventricle into pieces of approximately 1 mm³ using scissors and scalpel for ophthalmic use, a digesting reaction was allowed in 5 ml of HBSS buffer (manufactured by Invitrogen Corporation) containing 2.5 U/ml papain, 250 U/ml DNase (both manufactured by Worthington, Freehold, NJ) and 1 U/ml neutral protease (Dispase: manufactured by Boehringer Manheim) at 37°C for 30 min. A mixture of the cells and tissue obtained following the reaction was washed three times with DMEM (manufactured by Invitrogen Corporation) containing 10% fetal calf serum (manufactured by Hyclone), dissolved in DMEM containing 10% fetal calf serum, and then undigested substances were eliminated using a 10⁷ µm nylon mesh.

Thus resulting crude cell extract was cultured overnight on a 10 cm culture dish using a DMEM/F12 medium (manufactured by Invitrogen Corporation) containing 10% fetal calf serum in an incubator at 37°C. On the following day, the medium was replaced with DMEM/F12 containing a 1% N2 supplement (manufactured by Invitrogen Corporation) and 20 ng/ml FGF-2 (manufactured by PeproTech Inc.)to initiate the culture. Every three days, half of the medium was replaced with fresh DMEM/F12 containing a 1% N2 supplement and 20 ng/ml FGF-2, and the culture was continued. When a small colony of small cells was formed, it was treated with 1% trypsin for approximately from 30 sec. to 1 min, and the stripped cells were recovered. Culture of the cells was continued overnight using 10 µg/ml polyornithine (manufactured by Sigma Corporation) at room temperature, and then seeded on a multi-well culture dish (manufactured by Fisher Scientific) which had been coated using 5 µg/ml laminin derived from mouse EHS tumor (manufactured by Becton, Dickinson and Company) at 37°C overnight, followed by continuing the culture. Through keeping on the aforementioned culture, small thick cells having small sized projections were concentrated. The cells were used in the aforementioned experiment as an adult neural stem cell strain ANSC-7.

### BEST MODE FOR CARRYING OUT THE INVENTION

### Example 1: Tablet

According to a conventional method, a tablet having the following composition is prepared.

| (1) Prescription | | |
|---|---|---|
| | SB-216763 | 5 mg |
| | Lactose | 62 mg |
| | Potato starch | 30 mg |
| | Polyvinyl alcohol | 2 mg |
| | Magnesium stearate | 1 mg |
| | | 100 mg |

| | | |
|---|---|---|
| (2) | Kenpaullone | 5 mg |
| | Lactose | 62 mg |
| | Potato starch | 30 mg |
| | Polyvinyl alcohol | 2 mg |
| | Magnesium stearate | 1 mg |
| | | 100 mg |

| | | |
|---|---|---|
| (3) | Indirubin-3'-monoxime | 5 mg |
| | Lactose | 62 mg |
| | Potato starch | 30 mg |
| | Polyvinyl alcohol | 2 mg |
| | Magnesium stearate | 1 mg |
| | | 100 mg |

### Example 2: Agent for the promotion of neuropoiesis of a neural stem cell (1)

According to a conventional method, lithium chloride was dissolved in PBS to give 3 mol/l to prepare an agent for the promotion of neuropoiesis of a neural stem cell containing lithium chloride.

### Example 3: Agent for the promotion of neuropoiesis of a neural stem cell (2)

According to a conventional method, SB-216763, Kenpaullone or indirubin-3' -monoxime was dissolved in DMSO to give 0.1 mmol/l to prepare an agent for the promotion of neuropoiesis of a neural stem cell containing SB-216763, Kenpaullone or indirubin-3'-monoxime.

### INDUSTRIAL APPLICABILITY

According to the present invention, a nerve regenerating drug comprising a substance that inhibits the activity of glycogen synthase kinase-3, as an active ingredient; an agent for the promotion of neuropoiesis of a neural stem cell comprising the substance as an active ingredient; a neuron obtained by culturing a neural stem cell in the presence of the agent for the promotion of neuropoiesis; and a method of the manufacture of the neuron can be provided.

### FREE TEXT OF THE SEQUENCE LISTING

SEQ ID NO: 5-Description of artificial sequence: synthetic protein
SEQ ID NO: 6-Description of artificial sequence: synthetic DNA
SEQ ID NO: 7- Description of artificial sequence: synthetic DNA
SEQ ID NO: 8- Description of artificial sequence: synthetic DNA
SEQ ID NO: 9- Description of artificial sequence: synthetic DNA
SEQ ID NO: 10- Description of artificial sequence: synthetic DNA
SEQ ID NO: 11- Description of artificial sequence: synthetic DNA
SEQ ID NO: 12- Description of artificial sequence: synthetic DNA
SEQ ID NO: 13- Description of artificial sequence: synthetic DNA
SEQ ID NO: 14- Description of artificial sequence: synthetic RNA
SEQ ID NO: 15- Description of artificial sequence: synthetic RNA
SEQ ID NO: 16- Description of artificial sequence: synthetic RNA
SEQ ID NO: 17- Description of artificial sequence: synthetic RNA

## Claims

1. A nerve regenerating drug which comprises a substance that inhibits the activity of glycogen synthase kinase-3 (hereinafter, abbreviated as GSK-3), as an active ingredient.

2. The medical drug according to claim 1 wherein the nerve regenerating drug is a therapeutic drug for a neurological disease.

3. The medical drug according to claim 2 wherein the neurological disease is selected from the group consisting of Parkinson's disease, Alzheimer's disease, Down's disease, cerebrovascular disorder, cerebral stroke, spinal cord injury, Huntington's chorea, multiple sclerosis, amyotrophic lateral sclerosis, epilepsy, anxiety disorder, schizophrenia, depression and manic depressive psychosis.

4. The medical drug according to any one of claims 1 to 3 wherein the substance that inhibits the activity of GSK-3 is lithium or a pharmacologically acceptable salt thereof.

5. The medical drug according to any one of claims 1 to 3 wherein the substance that inhibits the activity of GSK-3 is a bisindolylmaleimide derivative, a 3-aryl-4-indolylmaleimide derivative, an indolocarbazole derivative, an indolo[3,2-d][1]benzazepin-6(5H)-one derivative or an indirubin derivative, or a pharmacologically acceptable salt thereof.

6. The medical drug according to any one of claims 1 to 3 wherein the substance that inhibits the activity of GSK-3 is a compound represented by the formula (I): 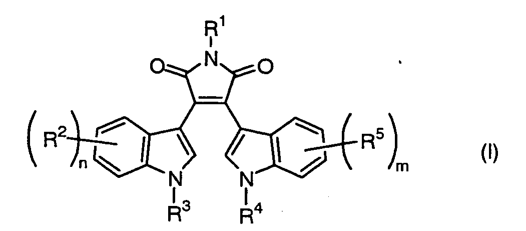 [wherein n and m may be the same or different, and represent an integer of 1 to 3; R¹, R³ and R⁴ may be the same or different, and represent hydrogen, substituted or unsubstituted lower alkyl, substituted or unsubstituted lower alkenyl, -COR⁶ (wherein R⁶ represents hydrogen, substituted or unsubstituted lower alkyl, substituted or unsubstituted lower alkenyl, substituted or unsubstituted aryl or substituted or unsubstituted cycloalkyl), -COOR⁷ (wherein R⁷ represents hydrogen, substituted or unsubstituted lower alkyl, substituted or unsubstituted aryl or substituted or unsubstituted cycloalkyl) or -OR⁸ (wherein R⁸ represents hydrogen, substituted or unsubstituted lower alkyl, substituted or unsubstituted aryl or substituted or unsubstituted cycloalkyl) ; R² and R⁵ may be the same or different, and represent hydrogen, substituted or unsubstituted lower alkyl, a substituted or unsubstituted lower alkenyl, substituted or unsubstituted lower alkoxy, substituted or unsubstituted lower alkoxycarbonyl, substituted or unsubstituted aryl, carboxy, halogen, hydroxy, nitro, amino, or mono- or di-lower alkylamino; when n and m are 2 or 3, each of R² and R⁵ may be the same or different], a compound represented by the formula (II): 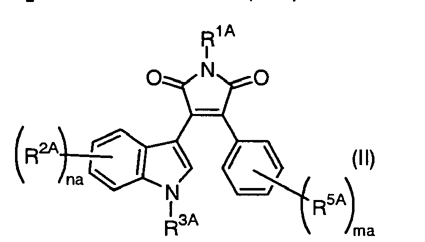 (wherein na, ma, R^{1A}, R^{2A}, R^{3A} and R^{5A} are as defined for the aforementioned n, m, R¹, R², R³ and R⁵, respectively) or a compound represented by the formula (III): 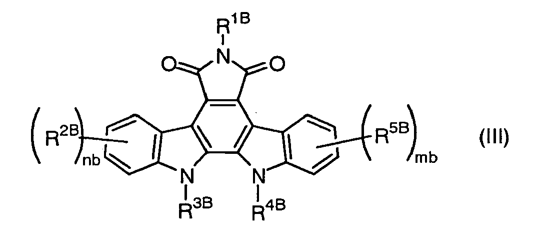 [wherein nb, mb, R^{1B}, R^{2B} and R^{5B} are as defined for the aforementioned n, m, R¹, R² and R⁵, respectively; R^{3B} and R^{4B} maybe the same or different, and represent hydrogen, substituted or unsubstituted lower alkyl, substituted or unsubstituted lower alkenyl, -COR⁶ (wherein R⁶ is as defined above), -COOR⁷ (wherein R⁷ is as defined above) or -OR⁸ (wherein R⁸ is as defined above), or R^{3B} and R^{4B} together form 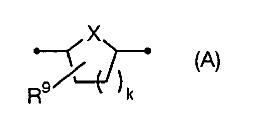 (wherein k represents 1 or 2; X represents CH₂, NH, an oxygen atom or a sulfur atom; R⁹ represents hydroxy, carboxy, carbamoyl or lower alkoxycarbonyl)], or a pharmacologically acceptable salt thereof.

7. The medical drug according to any one of claims 1 to 3 wherein the substance that inhibits the activity of GSK-3 is a compound represented by the formula (Ia): 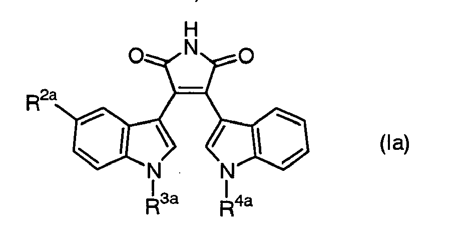 (wherein R^{2a} represents hydrogen, lower alkoxy, lower alkoxycarbonyl, aryl or nitro; R^{3a} and R^{4a} may be the same or different, and represent substituted or unsubstituted lower alkyl), or a pharmacologically acceptable salt thereof.

8. The medical drug according to any one of claims 1 to 3 wherein the substance that inhibits the activity of GSK-3 is a compound represented by the formula (IIa): 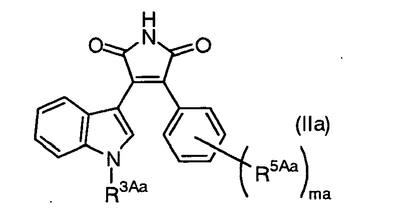 (wherein ma is as defined above; R^{3Aa} represents substituted or unsubstituted lower alkyl; R^{5Aa} represents halogen), or a pharmacologically acceptable salt thereof.

9. The medical drug according to any one of claims 1 to 3 wherein the substance that inhibits the activity of GSK-3 is a compound represented by the formula (IIIa): 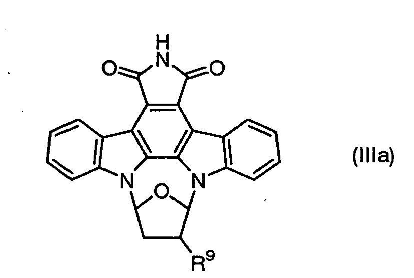 (wherein R⁹ is as defined above) or a pharmacologically acceptable salt thereof.

10. The medical drug according to any one of claims 1 to 3 wherein the substance that inhibits the activity of GSK-3 is a compound selected from the group consisting of 3,4-bis(1-methylindole-3-yl)-1H-pyrrole-2,5-dione, 3-(1-methylindole-3-yl)-4-(1-propylindole-3-yl)-1H-pyrrole -2,5-dione, 3-[1-(3-cyanopropyl)indole-3-yl]-4-(1-methylindole-3-yl)-1 H-pyrrole-2,5-dione, 3-[1-(3-aminopropyl)indole-3-yl]-4-(1-methylindole-3-yl)-1 H-pyrrole-2,5-dione, 3-[1-(3-carboxypropyl)indole-3-yl]-4-(1-methylindole-3-yl) -1H-pyrrole-2,5-dione, 3-[1-(3-carbamoylpropyl)indole-3-yl]-4-(1-methylindole-3-y 1)-1H-pyrrole-2,5-dione, 3'-[1-(3-aminopropyl)indole-3-yl]-4-(1-methyl-5-propyloxyindole-3-yl)-1H -pyrrole-2,5-dione, 3-[1-(3-hydroxypropyl)indole-3-yl]-4-(1-methyl-5-phenylind ole-3-yl)-1H-pyrrole-2,5-dione, 3-[1-(3-aminopropyl)indole-3-yl]-4-(1-methyl-5-phenylindol e-3-yl)-1H-pyrrole-2,5-dione, 3-[1-(3-hydroxypropyl)indole-3-yl]-4-(1-methyl-5-methoxyca rbonylindole-3-yl)-1H-pyrrole-2,5-dione, 3-[1-(3-hydroxypropyl)indole-3-yl]-4-(1-methyl-5-nitroindo le-3-yl)-1H-pyrrole-2,5-dione, 3-(1-methylindole-3-yl)-4-[1-(3-hydroxypropyl)-5-nitroindo le-3-yl]-1H-pyrrole-2,5-dione, 3-(2-chlorophenyl)-4-(1-methylindole-3-yl)-1H-pyrrole-2,5-dione, 3-(2,4-dichlorophenyl)-4-(1-methylindole-3-yl)-1H-pyrrole-2,5-dione, 3-(2-chlorophenyl)-4-[1-(3-hydroxypropyl)indole-3-yl]-lH-pyrrole-2,5-dione, 4-[1-(3-aminopropyl)indole-3-yl]-3-(2-chlorophenyl)-1H-pyrrole-2,5-dione and 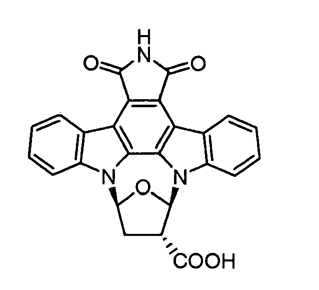 , or a pharmacologically acceptable salt thereof.

11. The medical drug according to any one of claims 1 to 3 wherein the substance that inhibits GSK-3 is a compound represented by the formula (IV): 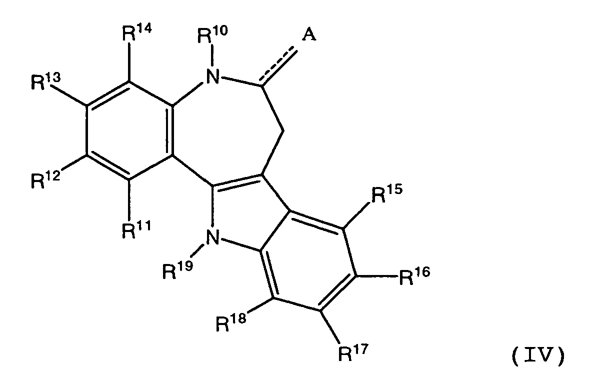 [wherein A is oxygen or sulfur coupled to the right by a single or double bond; R¹⁰ is selected from the group consisting of hydrogen, aryl, lower aliphatic substituents, particularly alkyl and lower alkyl ester; R¹¹-R¹⁴ are independently selected from the group consisting of alkoxy, amino, acyl, aliphatic substituents, particularly alkyl, alkenyl and alkinyl substituents, aliphatic alcohols, particularly alkyl alcohols, aliphatic nitriles, particularly alkyl nitriles, cyano, nitro, carboxyl, halogen, hydrogen, hydroxyl, imino and α, β-unsaturated ketones; R¹⁵- R¹⁸ are independently selected from the group consisting of aliphatic substituents, particularly alkyl, alkenyl and alkinyl substituents, particularly lower aliphatic substituents, alipahatic alcohols, particularly alkyl alcohols, alkoxy, acyl, cyano, nitro, epoxy, haloalkyl groups, halogen, hydrogen and hydroxyl; R¹⁹ is selected from the group consisting of aliphatic groups, particularly lower alkyl groups, aliphatic alcohols, particularly alkyl alcohols, carboxylic acids and hydrogen], or a pharmacologically acceptable salt thereof.

12. The medical drug according to any one of claims 1 to 3 wherein the substance that inhibits GSK-3 is a compound selected from the group consisting of 7,12-dihydro-indolo[3,2-d][1]benzazepin-6(5H)-one, 2-bromo-7,12-dihydro-indolo[3,2-d][1]benzazepin-6(5H)-one, 9-bromo-7,12-dihydro-indolo[3,2-d][1]benzazepin-6(5H)-one, 9-chloro-7,12-dihydro-indolo[3,2-d][1]benzazepin-6(5H)-one, 11-chloro-7,12-dihydro-indolo[3,2-d][1]benzazepin-6(5H)-on e, 10-bromo-7,12-dihydro-indolo[3,2-d][1]benzazepin-6(5H)-one, 8-bromo-6,11-dihydro-thieno[3',2':2,3]azepino[4,5-b]indol-5(4H)-one, 9-bromo-7,12-dihydro-4-methoxy-indolo[3,2-d][1]benzazepin-6(5H)-one, 9-bromo-7,12-dihydro-4-hydroxy-indolo[3,2-d][1]benzazepin-6(5H)-one, 7,12-dihydro-4-methoxy-indolo[3,2-d][1]benzazepin-6(5H)-on e, 9-bromo-7,12-dihydro-2,3-dimethoxy-indolo[3,2-d][1]benzaze pin-6(5H)-one, 9-bromo-7,12-dihydro-2,3-dihydroxy-indolo[3,2-d] [1]benzaze pin-6(5H)-one, 7,12-dihydro-2,3-dimethoxy-indolo[3,2-d][1]benzazepin-6(5H )-one, 7,12-dihydro-9-trifluormethyl-indolo[3,2-d][1]benzazepin-6(5H)-one, 7,12-dihydro-2,3-dimethoxy-9-trifluoromethyl-indolo[3,2-d][1]-benzazepin-6(5H)-one, 2-bromo-7,12-dihydro-9-trifluoromethyl-indolo[3,2-d][1]benzazepin-6(5H)-one, 9-bromo-7,12-dihydro-indolo[3,2d][1]benzazepin-6(5H)-thion e, 9-bromo-5,12-bis-(t-butyloxycarbonyl)-7,12-dihydro-indolo[ 3,2-d] [1]benzazepin-6(5H)-one, 9-bromo-12-(t-butyloxycarbonyl)-7,12-dihydro-indolo[3,2-d] [1]benzazepin-6(5H)-one, 9-bromo-5,7-bis-(t-butyloxycarbonyl)-7,12-dihydro-indolo-[3,2-d][1]benzazepin-6(5H)-one, 9-bromo-5,7,12-tri-(t-butyloxycarbonyl)-7,12-dihydro-indol o[3,2-d][1]benzazepin-6(5H)-one, 9-bromo-7,12-dihydro-5-methyloxycarbonylmethyl-indolo[3,2-d][1]benzazepin-6(5H)-one, 9-bromo-7,12-dihydro-12-methyloxycarbonylmethyl-indolo[3,2-d][1]benzazepin-6(5H)-one, 9-bromo-7,12-dihydro-12-(2-hydroxyethyl)-indolo[3,2-d][1]benzazepin-6(5H)-one, 2,9-dibromo-7,12-dihydro-indolo[3,2-d][1]benzazepin-6(5H)-one, 8,10-dichloro-7,12-dihydro-indolo[3,2d][1]benzazepin-6(5H) -one, 9-cyano-7,12-dihydro-indolo[3,2-d] [1]benzazepin-6(5H)-one, 9-bromo-7,12-dihydro-5-methyl-indolo[3,2-d][1]benzazepin-6 (5H)-one, 5-benzyl-9-bromo-7,12-dihydro-5-methyl-indolo-[3,2-d][1]benzazepin-6(5H)-one, . 9-bromo-7,12-dihydro-12-methyl-indolo[3,2-d][1]benzazepin-6(5H)-one, 9-bromo-12-ethyl-7,12-dihydro-indolo[3,2-d][1]benzazepin-6 (5H)-one, 9-bromo-7,12-dihydro-12-(2-propenyl)-indolo[3,2-d][1]-benzazepin-6(5H)-one, 7,12-dihydro-9-methyl-indolo[3,2-d]-[1]benzazepin-6(5H)-one, 7,12-dihydro-9-methoxy-indolo-[3,2-d][1]benzazepin-6(5H)-one, 9-fluoro-7,12-dihydro-12-(2-propenyl)-indolo[3,2-d][1]benz azepin-6(5H)-one, 11-bromo-7,12-dihydro-indolo[3,2d][1]benzazepin-6(5H)-one, 9-bromo-7,12-dihydro-2-(methyliminoamine)-indolo[3,2-d]-[1]benzazepin-6(5H)-one, 9-bromo-7,12-dihydro-2-(carboxylic acid)-indolo[3,2-d][1]benzazepin-6(5H)-one, 9-bromo-7,12-dihydro-10-hydroxy-indolo[3,2-d][1]benzazepin -6(5H)-one, 9-bromo-7,12-dihydro-11-hydroxymethyl-indolo[3,2-d][1]-benzazepin-6(5H)-one, 7,12-dihydro-4-hydroxy-indolo[3,2-d]-[1]benzazepin-6(5H)-one, 7,12-dihydro-2,3-dihydroxy-indolo[3,2-d][1]benzazepin-6(5H )-one, 2,3-dimethoxy-9-nitro-7,12-dihydro-indolo[3,2d][1]benzazep in-6(5H)-one, 9-cyano-7,12-dihydro-indolo[3,2-d][1]benzazepin-6(5H)-one, 2,3-dimethoxy-9-cyano-7,12-dihydro-indolo[3,2-d][1]benzazepin-6(5H)-one, 9-nitro-7,12-dihydro-indolo[3,2-d][1]-benzazepin-6(5H)-one, 3-(6-oxo-9-trifluoromethyl-5,6,7,12-tetrahydro-indolo[3,2-d][1]benzazepin-2-yl)-propionitrile, 2-bromo-9-nitro-7,12-dihydro-indolo[3,2-d][1]benzazepin-6( 5H)-one, 3-(6-oxo-9-trifluoromethyl-5,6,7,12-tetrahydro-indolo[3,2-d][1]benzazepin-2-yl)acrylonitrile, 2-(3-hydroxy-1-propinyl)-9-trifluoromethyl-7,12-dihydro-in dolo[3,2-d][1]benzazepin-6(5H)-one, 2-iodo-9-bromo-7,12-dihydro-indolo[3,2-d][1]benzazepin-6(5 H)-one, 2-(3-oxo-1-butenyl)-9-trifluoromethyl-7,12-tetrahydro-indo lo[3,2-d][1]benzazepin-6(5H)-one, 8-chloro-6,11-dihydro-thieno-[3',2':2,3]azepino[4,5-b]indol-5(4H)-one, 2-iodo-9-trifluoro-methyl-7,12-dihydro-indolo[3,2-d][1]benzazepin-6(5H)-one, 7,12-dihydro-pyrido [3',2':4,5]pyrrolo[3,2-d]-[1]benzazepin-6(5H)-one, 11-methyl-7,12-dihydro-indolo-[3,2-d][1]-benzazepin-6(5H)-one, 2-[2-(1-hydroxycyclohexyl)ethinyl]-9-trifluoromethyl-7,12-dihydro-indolo[3,2-d ][1]benzazepin-6(5H)-one, 2-cyano-7,12-dihydro-indolo-[3,2-d][1]benzazepin-6(5H)-one, 2-iodo-7,12-dihydro-indolo[3,2-d][1]benzazepin-6(5H)-one, 11-ethyl-7;12-dihydro-indolo[3,2-d][1]benzazepin-6(5H)-one, 8-methyl-6,11-dihydro-thieno[3',2':2,3]azepino[4,5-b]indol -5(4H)-one and 3-(6-oxo-9-trifluoromethyl-5,6,7,12-tetrahydro-indolo[3,2-d][1]benzazepin-2-yl)acrylic acid, methyl ester, or a pharmacologically acceptable salt thereof.

13. The medical drug according to any one of claims 1 to 3 wherein the substance that inhibits GSK-3 is selected from the group consisting of 9-cyano-7,12-dihydro-indolo[3,2-d][1]benzazepin-6(5H)-one, 9-bromo-7,12-dihydro-2,3-dimethoxy-indolo[3,2-d][1]benzazepin-6(5H)-one, 2-bromo-7,12-dihydro-9-trifluoromethyl-indolo[3,2-d][1]-benzazepin-6(5H)-one, 7,12-dihydro-2,3-dimethoxy-9-trifluoromethyl-indolo[3,2-d][1]benzazepin-6(5H)-one, 2,9-dibromo-7,12-dihydro-indolo[3,2-d][1]benzazepin-6(5H)-one, 7,12-dihydro-9-trifluoromethyl-indolo[3,2-d][1]benzazepin-6(5H)-one, 9-chloro-7,12-dihydro-indolo[3,2-d][1]-benzazepin-6(5H)-one, 8-bromo-6,11-dihydro-thieno-[3',2':2,3]azepino[4,5-b]indole-5(4H)-one, 7,12-dihydro-9-methoxy-indolo[3,2-d][1]benzazepin-6(5H)-on e, 10-bromo-7,12-dihydro-indolo[3,2-d][1]benzazepin-6(5H)-one, 11-bromo-7,12-dihydro-indolo[3,2-d][1]benzazepin-6(5H)-one, 11-chloro-7,12-dihydro-indolo[3,2-d][1]benzazepin-6(5H)-on e, 9-fluoro-7,12-dihydro-indolo[3,2-d][1]benzazepin-6(5H)-one, 9-methyl-7,12-dihydro-indolo[3,2-d][1]benzazepin-6(5H)-one, 9-bromo-7,12-dihydro-indolo[3,2-d]-[1]benzazepin-6(5H)-thione, 8,10-dichloro-7,12-dihydro-indolo[3,2-d][1]benzazepin-6(5H )-one, 9-bromo-7,12-dihydro-12-(2-hydroxyethyl)-indolo[3,2-d][1]benzazepin-6(5H) -one, 9-bromo-7,12-dihydro-2,3-dihydroxy-indolo[3,2-d]-[1]benzazepin-6(5H)-one, 2-bromo-7,12-dihydro-indolo-[3,2-d][1]benzazepin-6(5H)-one, 7,12-dihydro-2,3-dimethoxy-indolo[3,2-d][1]benzazepin-6(5H )-one, 9-bromo-7,12-dihydro-12-methyl-indolo[3,2-d][1]benzazepin-6(5H)-one, 9-bromo-7,12-dihydro-5-methyloxycarbonylmethyl-indolo-[3,2-d][1]benzazepin-6(5H)-one and 7,12-dihydro-indolo[3,2-d][1]benzazepin-6(5H)-one.

14. The medical drug according to any one of claims 1 to 3 which is selected from the group consisting of 9-cyano-7,12-dihydro-indolo[3,2-d][1]benzazepin-6(5H)-one, 9-bromo-7,12-dihydro-2,3-dimethoxy-indolo[3,2-d][1]benzazepin-6(5H)-one, 2-bromo-7,12-dihydro-9-trifluoromethyl-indolo[3,2-d][1]ben zazepin-6(5H)-one, 7,12-dihydro-2,3-dimethoxy-9-trifluoromethyl-indolo[3,2-d] [1]benzazepin-6(5H)-one, 2,9-dibromo-7,12-dihydro-indolo[3,2-d][1]-benzazepin-6(5H)-one, 7,12-dihydro-9-trifluormethyl-indolo-[3,2-d][1]benzazepin-6(5H)-one, 9-chloro-7,12-dihydro-indolo[3,2-d][1]benzazepin-6(5H)-one, 8-bromo-6,11-dihydro-thieno[3',2':2,3]azepino[4,5-b]indol-5(4H)-one, 7,12-dihydro-9-methoxy-indolo[3,2-d][1]benzazepin-6(5H)-on e.

15. The medical drug according to any one of claims 1 to 3 which is selected from the group consisting of 9-bromo-7,12-dihydro-indolo[3,2-d][1]benzazepin-6(5H)-one .

16. The medical drug according to any one of claims 1 to 3 wherein the substance that inhibits GSK-3 is a compound represented by the formula (V): 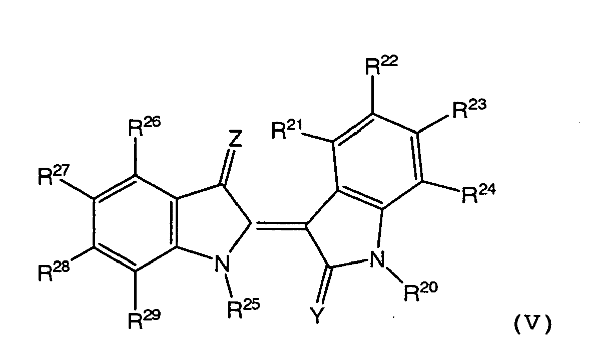 [wherein R²⁰ and R²⁵ which may be the same or different represent hydrogen; halogen; a hydroxy group; a methylene hydroxy group; a straight chain or branched C₁ to C₁₈-alkyl or alkoxy or methylenealkoxy group; a cycloalkyl group having 3 to 7 carbon atoms, and including one or more heteroatoms as needed; a substituted or unsubstituted aryl, aralkyl or aryloxy group having one or more heteroatoms as needed; a mono-, di- or trialkylsilyl group each independently having 1 to 6 carbon atoms within the straight chain or branched alkyl group; a mono-, di- or triarylsilyl group each independently having a substituted or unsubstituted aryl group; a trifluoromethyl group; -COM; -COOM; or a -CH₂COOM group (wherein M represents hydrogen, a straight chain or branched C₁ to C₁₈-alkyl group substituted with one or more hydroxy and/or amino groups if necessary, or an aryl group, which may be substituted with one or more halogen, alkyl groups or alkoxy groups, having one or more heteroatoms if necessary); an -NR³⁰R³¹ group (wherein R³⁰ and R³¹ which may be the same or different represent hydrogen, a C₁ to C₁₈ straight chain or branched alkyl group additionally substituted with one or more hydroxy and/or amino groups if necessary, a substituted or unsubstituted aryl group including one or more heteroatoms if necessary); an acyl group; a -CH₂-NR³⁰R³¹ methyleneamino group (wherein R³⁰ and R³¹ have the meanings as defined above); a benzyl group having one or more heteroatoms in the benzene ring if necessary; a methylenecycloalkyl group having 3 to 7 carbon atoms, and including one or more heteroatoms if necessary; a physiological amino acid group coupled to a nitrogen atom as an amide; an O-glycoside or N-glycoside glycoside of which being selected from monosaccharidesor disaccharides;or a methylenesulfonate group; R²¹, R²², R²³, R²⁴, R²⁶, R²⁷, R²⁸ and R²⁹ which may be the same or different represent hydrogen; halogen; a hydroxy group; a nitroso group; a nitro group; an alkoxy group; a straight chain or branched C₁ to C₁₈ alkyl group substituted with one or more hydroxy and/or amino groups if necessary; a substituted or unsubstituted aryl group having one or more heteroatoms if necessary; a substituted or unsubstituted aralkyl group having one or more heteroatoms if necessary; a substituted or unsubstituted aryloxy group having one or more heteroatoms if necessary; a substituted or unsubstituted methylenearyloxy group having one or more heteroatoms if necessary; a cycloalkyl group having 3 to 7 carbon atoms, and including one or more heteroatoms if necessary; a methylenecycloalkyl group having 3 to 7 carbon atoms, and including one or more heteroatoms if necessary; a trifluoromethyl group; -COM; -COOM; or a CH₂COOM group (wherein M represents hydrogen, a straight chain or branched C₁ to C₁₈-alkyl group additionally substituted with one or more hydroxy and/or amino groups if necessary, or an aryl group, which may be substituted with one or more halogen atoms, alkyl groups or alkoxy groups, having one or more heteroatoms if necessary); an -NR³⁰R³¹ group (wherein R³⁰ and R³¹ which may be the same or different represent hydrogen, a straight chain or branched C₁ to C₁₈-alkyl group additionally substituted with one or more hydroxy and/or amino groups if necessary, a substituted or unsubstituted aryl group including one or more heteroatoms if necessary, an acyl group; or form a part of cycloalkyl having 3 to 7 carbon atoms with the nitrogen atom including one or more heteroatoms if necessary); a -CONR³⁰R³¹ group (wherein R³⁰ and R³¹ have the meanings as defined above) ; a hydroxylamino group; a phosphate group; a phosphonate group; a sulfate group; a sulfonate group; a sulfonamide group; an -SO₂NR³⁰R³¹ group (wherein R³⁰ and R³¹ have the meanings as defined above); an -N=N-R³² azo group (wherein R³² represents an aromatic group substituted with one or more carboxyl, phosphoryl or sulfonate groups if necessary, or an O-glycoside or N-glycoside group glycoside of which being selected from monosaccharides or disaccharides); or R²⁰ and R²⁴, and R²⁵ and R²⁹ together form a ring having one to four CH₂ groups each independently substituted if necessary, respectively; Y and Z which may be the same or different represent an oxygen; sulfur; selenium; tellurium atom; an NR³³ group (wherein R³³ represents hydrogen, a straight chain or branched C₁ to C₁₈ alkyl group substituted with one or more carboxyl, phosphoryl or sulfonate groups if necessary, a substituted or unsubstituted aryl group including one or more heteroatoms if necessary, an aralkyl group or a sulfonate group); or -NOR³³ (wherein R³³ group have the meanings as defined above)], or a pharmacologically acceptable salt thereof.

17. The medical drug according to any one of claims 1 to 3 wherein the substance that inhibits GSK-3 is a compound selected from the group consisting of indirubin, 5-iodo-indirubin, 5-bromo-indirubin, 5-chloro-indirubin, 5-fluoro-indirubin, 5-methyl-indirubin, 5-nitro-indirubin, 5-SO₃H-indirubin, 5'-bromo-indirubin, 5-5'-dibromo-indirubin and 5' -bromo-indirubin 5-sulfonic acid, or a pharmacologically acceptable salt thereof.

18. The medical drug according to any one of claims 1 to 3 wherein the substance that inhibits GSK-3 is a compound selected from the group consisting of indirubin-3' -monooxime, 5-iodo-indirubin-3'-monooxime and 5-SO₃Na-indirubin-3'-monooxime, or a pharmacologically acceptable salt thereof.

19. The medical drug according to any one of claims 1 to 3 wherein the substance that inhibits GSK-3 is indirubin-3'-monooxime or a pharmacologically acceptable salt thereof.

20. An agent for the promotion of neuropoiesis of a neural stem cell which comprises a substance that inhibits the activity of GSK-3, as an active ingredient.

21. The agent for the promotion of neuropoiesis according to claim 20 wherein the substance that inhibits the activity of GSK-3 is lithium or a pharmacologically acceptable salt thereof.

22. The agent for the promotion of neuropoiesis according to claim 20 wherein the substance that inhibits the activity of GSK-3 is a bisindolylmaleimide derivative, a 3-aryl-4-indolylmaleimide derivative, an indolocarbazole derivative or an indolo[3,2-d][1]benzazepin-6(5H)-one derivative, or a pharmacologically acceptable salt thereof.

23. The agent for the promotion of neuropoiesis according to claim 20 wherein the substance that inhibits the activity of GSK-3 is a compound represented by the formula (I) : 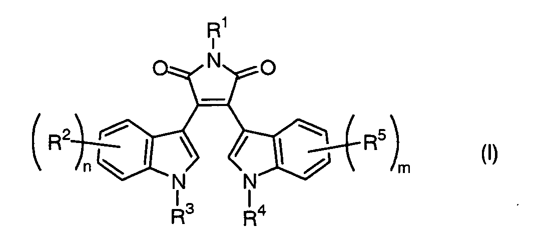 [wherein n and m may be the same or different, and represent an integer of 1 to 3; R¹, R³ and R⁴ may be the same or different, and represent hydrogen, substituted or unsubstituted lower alkyl, substituted or unsubstituted lower alkenyl, -COR⁶ (wherein R⁶ represents hydrogen, substituted or unsubstituted lower alkyl, substituted or unsubstituted lower alkenyl, substituted or unsubstituted aryl or substituted or unsubstituted cycloalkyl), -COOR⁷ (wherein R⁷ represents hydrogen, substituted or unsubstituted lower alkyl, substituted or unsubstituted aryl or substituted or unsubstituted cycloalkyl) or -OR⁸ (wherein R⁸ represents hydrogen, substituted or unsubstituted lower alkyl, substituted or unsubstituted aryl or substituted or unsubstituted cycloalkyl); R² and R⁵ may be the same or different, and represent hydrogen, substituted or unsubstituted lower alkyl, a substituted or unsubstituted lower alkenyl,-substituted or unsubstituted lower alkoxy, substituted or unsubstituted lower alkoxycarbonyl, substituted or unsubstituted aryl, carboxy, halogen, hydroxy, nitro, amino, or mono- or di-lower alkylamino; when n and m are 2 or 3, each of R² and R⁵ may be the same or different], a compound represented by the formula (II): 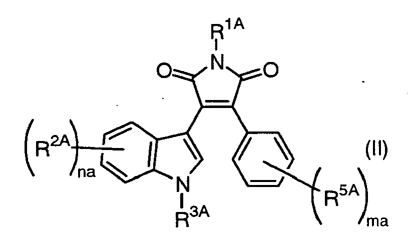 (wherein na, ma, R^{1A}, R^{2A}, R^{3A} and R^{5A} are as defined for the aforementioned n, m, R¹, R², R³ and R⁵, respectively) or a compound represented by the formula (III): 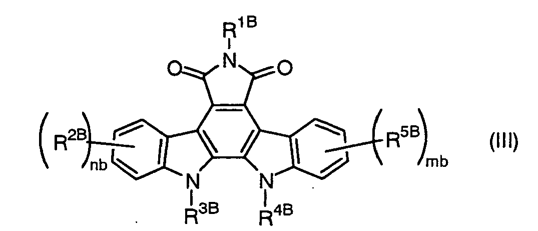 [wherein nb, mb, R^{1B}, R^{2B} and R^{5B} are as defined for the aforementioned n, m, R¹, R² and R⁵, respectively; R^{3B} and R^{4B} maybe the same or dif ferent, and represent hydrogen, substituted or unsubstituted lower alkyl, substituted or unsubstituted lower alkenyl, -COR⁶ (wherein R⁶ is as defined above), -COOR⁷ (wherein R⁷ is as defined above) or -OR⁸ (wherein R⁸ is as defined above), or R^{3B} and R^{4B} together form 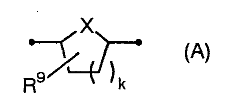 (wherein k represents 1 or 2; X represents CH₂, NH, an oxygen atom or a sulfur atom; R⁹ represents hydroxy, carboxy, carbamoyl or lower alkoxycarbonyl)], or a pharmacologically acceptable salt thereof.

24. The agent for the promotion of neuropoiesis according to claim 20 wherein the substance that inhibits the activity of GSK-3 is a compound represented by the formula (Ia): 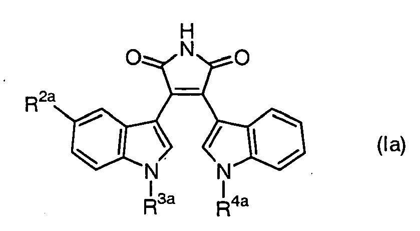 (wherein R^{2a} represents hydrogen, lower alkoxy, lower alkoxycarbonyl, aryl or nitro; R^{3a} and R^{4a} may be the same or different, and represent substituted or unsubstituted lower alkyl), or a pharmacologically acceptable salt thereof.

25. The agent for the promotion of neuropoiesis according to claim 20 wherein the substance that inhibits the activity of GSK-3 is a compound represented by the formula (IIa) : 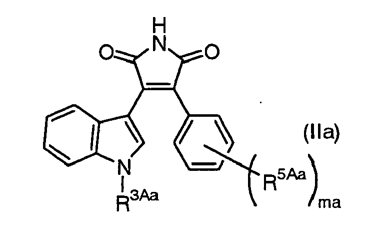 (wherein ma is as defined above; R^{3Aa} represents substituted or unsubstituted lower alkyl; R^{5Aa} represents halogen), or a pharmacologically acceptable salt thereof.

26. The agent for the promotion of neuropoiesis according to claim 20 wherein the substance that inhibits the activity of GSK-3 is a compound represented by the formula (IIIa): 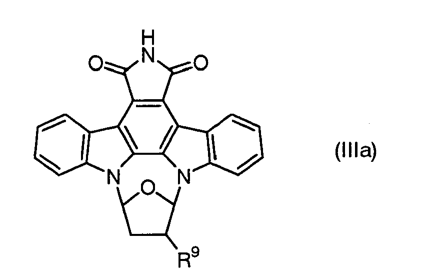 (wherein R⁹ is as defined above), or a pharmacologically acceptable salt thereof.

27. The agent for the promotion of neuropoiesis according to claim 20 wherein the substance that inhibits the activity of GSK-3 is a compound selected from the group consisting of 3,4-bis(1-methylindole-3-yl)-1H-pyrrole-2,5-dione, 3-(1-methylindole-3-yl)-4-(1-propylindole-3-yl)-1H-pyrrole -2,5-dione, 3-[1-(3-cyanopropyl)indole-3-yl]-4-(1-methylindole-3-yl)-1 H-pyrrole-2,5-dione, 3-[1-(3-aminopropyl)indole-3-yl]-4-(1-methylindole-3-yl)-1H-pyrrole-2,5 -dione, 3-[1-(3-carboxypropyl)indole-3-yl]-4-(1-methylindole-3-yl)-1H-pyrrole-2,5-dione, 3-[1-(3-carbamoylpropyl)indole-3-yl]-4-(1-methylindole-3-yl)-1H-pyrrole-2,5 -dione, 3-[1-(3-aminopropyl)indole-3-yl]-4-(1-methyl-5-propyloxyin dole-3-yl)-1H-pyrrole-2,5-dione, 3-[1-(3-hydroxypropyl)indole-3-yl]-4-(1-methyl-5-phenylinol ole-3-yl)-1H-pyrrole-2,5-dione, 3-[1-(3-aminopropyl)indole-3-yl]-4-(1-methyl-5-phenylindol e-3-yl)-1H-pyrrole-2,5-dione, 3-[1-(3-hydroxypropyl)indole-3-yl]-4-(1-methyl-5-methoxy-carbonylindole-3-yl)-1H-pyrrole-2,5-dione, 3-[1-(3-hydroxypropyl)indole-3-yl]-4-(1-methyl-5-nitroindo le-3-yl)-1H-pyrrole-2,5-dione, 3-(1-methylindole-3-yl)-4-[1-(3-hydroxypropyl)-5-nitroindo le-3-yl]-1H-pyrrole-2,5-dione, 3-(2-chlorophenyl)-4-(1-methylindole-3-yl)-1H-pyrrole-2,5-dione, 3-(2,4-dichlorophenyl)-4-(1-methylindole-3-yl)-1H-pyrrole-2,5-dione, 3-(2-chlorophenyl)-4-[1-(3-hydroxypropyl)indole-3-yl]-1H-pyrrole-2,5-dione, 4-[1-(3-aminopropyl)indole-3-yl]-3-(2-chlorophenyl)-1H-pyrrole-2,5-dion e and 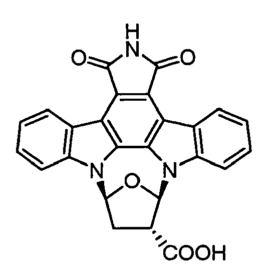 , or a pharmacologically acceptable salt thereof.

28. The agent for the promotion of neuropoiesis according to claim 20 wherein the substance that inhibits GSK-3 is a compound represented by the formula (IV): 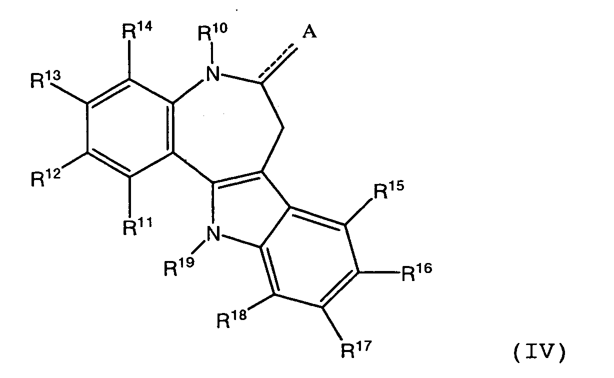 [wherein A is oxygen or sulfur coupled to the right by a single or double bond; R¹⁰ is selected from the group consisting of hydrogen, aryl, lower aliphatic substituents, particularly alkyl and lower alkyl ester; R¹¹-R¹⁴ are independently selected from the group consisting of alkoxy, amino, acyl, aliphatic substituents, particularly alkyl, alkenyl and alkinyl substituents, aliphatic alcohols,particularly alkyl alcohols, aliphatic nitriles, particularly alkyl nitriles, cyano, nitro, carboxyl, halogen, hydrogen, hydroxyl, imino and α,β-unsaturated ketones; R¹⁵-R¹⁸ are independently selected from the group consisting of aliphatic substituents, particularly alkyl, alkenyl and alkinyl substituents, particularly lower aliphatic substituents, alipahatic alcohols, particularly alkyl alcohols, alkoxy, acyl, cyano, nitro, epoxy, haloalkyl groups, halogen, hydrogen and hydroxyl; R¹⁹ is selected from the group consisting of aliphatic groups, particularly lower alkyl groups, aliphatic alcohols, particularly alkyl alcohols, carboxylic acids and hydrogen], or a pharmacologically acceptable salt thereof.

29. The agent for the promotion of neuropoiesis according to claim 20 which is selected from the group cons isting of 7,12-dihydro-indolo[3,2-d][1]benzazepin-6(5H)-one, 2-bromo-7,12-dihydro-indolo[3,2-d][1]benzazepin-6(5H)-one, 9-bromo-7,12-dihydro-indolo[3,2-d][1]benzazepin-6(5H)-one, 9-chloro-7,12-dihydro-indolo[3,2-d][1]benzazepin-6(5H)-one, 11-chloro-7,12-dihydro-indolo-[3,2-d][1]benzazepin-6(5H)-one, 10-bromo-7,12-dihydro-indolo[3,2-d][1]benzazepin-6(5H)-one, 8-bromo-6,11-dihydro-thieno[3' ,2' :2,3]azepino[4,5-b]indol-5(4H)-one, 9-bromo-7,12-dihydro-4-methoxy-indolo[3,2-d][1]benzazepin-6(5H)-one, 9-bromo-7,12-dihydro-4-hydroxy-indolo[3,2-d]-[1]benzazepin-6(5H)-one, 7,12-dihydro-4-methoxy-indolo-[3,2-d][1]benzazepin-6(5H)-one, 9-bromo-7,12-dihydro-2,3-dimethoxy-indolo[3,2-d][1]benzaze pin-6(5H)-one, 9-bromo-7,12-dihydro-2,3-dihydroxy-indolo[3,2-d] [1]benzaze pin-6(5H)-one, 7,12-dihydro-2,3-dimethoxy-indolo[3,2-d] [1]-benzazepin-6(5H)-one, 7,12-dihydro-9-trifluormethyl-indolo-[3,2-d] [1]benzazepin-6(5H)-one, 7,12-dihydro-2,3-dimethoxy-9-trifluoromethyl-indolo[3,2-d] [1]benzazepin-6(5H)-one, 2-bromo-7,12-dihydro-9-trifluoromethyl-indolo-[3,2-d] [1]benzazepin-6(5H)-one, 9-bromo-7,12-dihydro-indolo[3,2d][1]benzazepin-6(5H)-thion e, 9-bromo-5,12-bis-(t-butyloxycarbonyl)-7,12-dihydro-indolo[ 3,2-d][1]benzazepin-6(5H)-one, 9-bromo-12-(t-butyloxycarbonyl)-7,12-dihydro-indolo[3,2-d] [1]benzazepin-6(5H)-one, 9-bromo-5,7-bis-(t-butyloxycarbonyl)-7,12-dihydro-indolo[3 ,2-d][1]benzazepin-6(5H)-one, 9-bromo-5,7,12-tri-(t-butyloxycarbonyl)-7,12-dihydro-indolo[3,2-d][1]benzazepin-6(5H) -one, 9-bromo-7,12-dihydro-5-methyloxycarbonylmethyl-indolo[3,2-d][1]benzazepin-6(5H)-one, 9-bromo-7,12-dihydro-12-methyloxycarbonylmethyl-indolo[3,2-d][1]benzazepin-6(5H)-one, 9-bromo-7,12-dihydro-12-(2-hydroxyethyl)-indolo[3,2-d][1]benzazepin-6(5H)-one, 2,9-dibromo-7,12-dihydro-indolo[3,2-d][1]benzazepin-6(5H)-one, 8,10-dichloro-7,12-dihydro-indolo[3,2d][1]benzazepin-6(5H) -one, 9-cyano-7,12-dihydro-indolo[3,2-d][1]-benzazepin-6(5H)-one, 9-bromo-7,12-dihydro-5-methyl-indolo[3,2-d] [1]benzazepin-6 (5H)-one, 5-benzyl-9-bromo-7,12-dihydro-5-methyl-indolo[3,2-d][1]ben zazepin-6(5H)-one, 9-bromo-7,12-dihydro-12-methyl-indolo[3,2-d] [1]benzazepin-6(5H)-one, 9-bromo-12-ethyl-7,12-dihydro-indolo[3,2-d][1]benzazepin-6 (5H)-one, 9-bromo-7,12-dihydro-12-(2-propenyl)-indolo[3,2-d][1]benza zepin-6(5H)-one, 7,12-dihydro-9-methyl-indolo[3,2-d][1]benzazepin-6(5H)-one, 7,12-dihydro-9-methoxy-indolo[3,2-d][1]benzazepin-6(5H)-on e, 9-fluoro-7,12-dihydro-12-(2-propenyl)-indolo[3,2-d][1]benz azepin-6(5H)-one, 11-bromo-7,12-dihydro-indolo[3,2d][1]-benzazepin-6(5H)-one, 9-bromo-7,12-dihydro-2-(methylimino-amine)-indolo[3,2-d][1]benzazepin-6(5H)-one, 9-bromo-7,12-dihydro-2-(carboxylic acid)-indolo[3,2-d][1]-benzazepin-6(5H)-one, 9-bromo-7,12-dihydro-10-hydroxy-indolo-[3,2-d][1]benzazepin-6(5H)-one, 9-bromo-7,12-dihydro-11-hydroxymethyl-indolo[3,2-d][1]benz azepin-6(5H)-one, 7,12-dihydro-4-hydroxy-indolo[3,2-d][1]benzazepin-6(5H)-on e, 7,12-dihydro-2,3-dihydroxy-indolo[3,2-d][1]-benzazepin-6(5H)-one, 2,3-dimethoxy-9-nitro-7,12-dihydro-indolo[3,2d][1]benzazep in-6(5H)-one, 9-cyano-7,12-dihydro-indolo[3,2-d][1]benzazepin-6(5H)-one, 2,3-dimethoxy-9-cyano-7,12-dihydro-indolo[3,2-d][1]benzaze pin-6(5H)-one, 9-nitro-7,12-dihydro-indolo[3,2-d][1]benzazepin-6(5H)-one, 3-(6-oxo-9-trifluoromethyl-5,6,7,12-tetrahydro-indolo[3,2-d][1]benzazepin-2-yl)-propionitrile, 2-bromo-9-nitro-7,12-dihydro-indolo[3,2-d][1]benzazepin-6( 5H)-one, 3-(6-oxo-9-trifluoromethyl-5,6,7,12-tetrahydro-indolo[3,2-d] [1]benzazepin-2-yl)acrylonitrile, 2-(3-hydroxy-1-propinyl)-9-trifluoromethyl-7,12-dihydro-in dolo[3,2-d] [1]benzazepin-6(5H)-one, 2-iodo-9-bromo-7,12-dihydro-indolo[3,2-d][1]-benzazepin-6(5H)-one, 2-(3-oxo-1-butenyl)-9-trifluoromethyl-7,12-tetrahydro-indolo[3,2-d][1]benzazepin-6(5H)-on e, 8-chloro-6,11-dihydro-thieno[3',2':2,3]azepino[4,5-b]indol -5(4H)-one, 2-iodo-9-trifluoromethyl-7,12-dihydro-indolo[3,2-d][1]benz azepin-6(5H)-one, 7,12-dihydro-pyrido-[3',2':4,5]pyrrolo[3,2-d] [1]benzazepin-6(5H)-one, 11-methyl-7,12-dihydro-indolo[3,2-d][1]-benzazepin-6(5H)-o ne, 2-[2-(1-hydroxycyclohexyl)ethinyl]-9-trifluoromethyl-7,12-dihydro-indolo[3,2-d] [1]benzazepin-6(5H)-one, 2-cyano-7,12-dihydro-indolo[3,2-d][1]benzazepin-6(5H)-one, 2-iodo-7,12-dihydro-indolo[3,2-d][1]benzazepin-6(5H)-one, 11-ethyl-7,12-dihydro-indolo[3,2-d] [1]benzazepin-6(5H)-one, 8-methyl-6,11-dihydro-thieno[3',2':2,3]azepino[4,5-b]-indol-5(4H)-one and 3-(6-oxo-9-trifluoromethyl-5,6,7,12-tetrahydro-indolo[3,2-d][1]benzazepin-2-yl)acrylic acid, methyl ester.

30. The agent for the promotion of neuropoiesis according to claim 20 which is selected from the group consisting of 9-cyano-7,12-dihydro-indolo[3,2-d][1]-benzazepin-6(5H)-one, 9-bromo-7,12-dihydro-2,3-dimethoxy-indolo-[3,2-d][1]benzazepin-6(5H)-one, 2-bromo-7,12-dihydro-9-trifluoromethyl-indolo[3,2-d][1]ben zazepin-6(5H)-one, 7,12-dihydro-2,3-dimethoxy-9-trifluoromethyl-indolo[3,2-d] [1]benzazepin-6(5H)-one, 2,9-dibromo-7,12-dihydro-indolo[3,2-d][1]benzazepin-6(5H)-one, 7,12-dihydro-9-trifluoromethyl-indolo[3,2-d][1]benzazepin-6(5H)-one, 9-chloro-7,12-dihydro-indolo[3,2-d][1]-benzazepin-6(5H)-one, 8-bromo-6,11-dihydro-thieno-[3',2':2,3]azepino[4,5-b]indole-5(4H)-one, 7,12-dihydro-9-methoxy-indolo[3,2-d][1]benzazepin-6(5H)-on e, 10-bromo-7,12-dihydro-indolo[3,2-d] [1]benzazepin-6(5H)-one, 11-bromo-7,12-dihydro-indolo[3,2-d] [1]benzazepin-6(5H)-one, 11-chloro-7,12-dihydro-indolo[3,2-d][1]benzazepin-6(5H)-on e, 9-fluoro-7,12-dihydro-indolo[3,2-d][1]benzazepin-6(5H)-one, 9-methyl-7,12-dihydro-indolo[3,2-d][1]benzazepin-6(5H)-one, 9-bromo-7,12-dihydro-indolo[3,2-d][1]-benzazepin-6(5H)-thione, 8,10-dichloro-7,12-dihydro-indolo-[3,2-d][1]benzazepin-6(5H)-one, 9-bromo-7,12-dihydro-12-(2-hydroxyethyl)-indolo[3,2-d][1]b enzazepin-6(5H)-one, 9-bromo-7,12-dihydro-2,3-dihydroxy-indolo[3,2-d][1]benzaze pin-6(5H)-one, 2-bromo-7,12-dihydro-indolo-[3,2-d][1]benzazepin-6(5H)-one, 7,12-dihydro-2,3-dimethoxy-indolo[3,2-d][1]benzazepin-6(5H )-one, 9-bromo-7,12-dihydro-12-methyl-indolo[3,2-d][1]benzazepin-6(5H)-one, 9-bromo-7,12-dihydro-5-methyloxycarbonylmethyl-indolo-[3,2-d][1]benzazepin-6(5H)-one and 7,12-dihydro-indolo[3,2-d][1]benzazepin-6(5H)-one.

31. The agent for the promotion of neuropoiesis according to claim 20 which is selected from the group cons isting of 9-cyano-7,12-dihydro-indolo[3,2-d][1]-benzazepin-6(5H)-one, 9-bromo-7,12-dihydro-2,3-dimethoxy-indolo[3,2-d][1]benzaze pin-6(5H)-one, 2-bromo-7,12-dihydro-9-trifluoromethyl-indolo[3,2-d][1]benzazepin-6(5H)-o ne, 7,12-dihydro-2,3-dimethoxy-9-trifluoromethyl-indolo-[3,2-d][1]benzazepin-6(5H)-one, 2,9-dibromo-7,12-dihydro-indolo[3,2-d][1]benzazepin-6(5H)-one, 7,12-dihydro-9-trifluoromethyl-indolo[3,2-d][1]benzazepin-6(5H)-one, 9-chloro-7,12-dihydro-indolo[3,2-d][1]benzazepin-6(5H)-one, 8-bromo-6,11-dihydro-thieno[3',2':2,3]azepino[4,5-b]indol-5(4H)-one, 7,12-dihydro-9-methoxy-indolo[3,2-d][1]benzazepin-6(5H)-one.

32. The agent for the promotion of neuropoiesis according to claim 20 which is selected from the group consisting of 9-bromo-7,12-dihydro-indolo[3,2-d][1]-benzazepin-6(5H)-one.

33. The agent for the promotion of neuropoiesis according to claim 20 wherein the substance that inhibits GSK-3 is a compound represented by the formula (V): 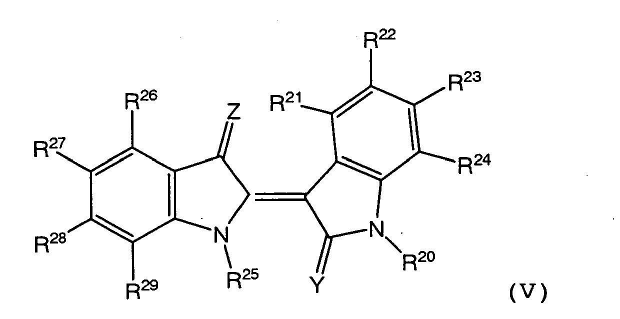 [wherein R²⁰ and R²⁵ which may be the same or different represent hydrogen; halogen; a hydroxy group; a methylene hydroxy group; a straight chain or branched C₁ to C₁₈-alkyl or alkoxy or methylenealkoxy group; a cycloalkyl group having 3 to 7 carbon atoms, and including one or more heteroatoms as needed; a substituted or unsubstituted aryl, aralkyl or aryloxy group having one or more heteroatoms as needed; a mono-, di- or trialkylsilyl group each independently having 1 to 6 carbon atoms within the straight chain or branched alkyl group; a mono-, di- or triarylsilyl group each independently having a substituted or unsubstituted aryl group; a trifluoromethyl group; -COM; -COOM; or a -CH₂COOM group (wherein M represents hydrogen, a straight chain or branched C₁ to C₁₈-alkyl group substituted with one or more hydroxy and/or amino groups if necessary, or an aryl group, which may be substituted with one or more halogen, alkyl groups or alkoxy groups, having one or more heteroatoms if necessary); an -NR³⁰R³¹ group (wherein R³⁰ and R³¹ which may be the same or different represent hydrogen, a C₁ to C₁₈ straight chain or branched alkyl group additionally substituted with one or more hydroxy and/or amino groups if necessary, a substituted or unsubstituted aryl group including one or more heteroatoms if necessary); an acyl group; a -CH₂-NR³⁰R³¹ methyleneamino group (wherein R³⁰ and R³¹ have the meanings as defined above); a benzyl group having one or more heteroatoms in the benzene ring if necessary; a methylenecycloalkyl group having 3 to 7 carbon atoms, and including one or more heteroatoms if necessary; a physiological amino acid group coupled to a nitrogen atom as an amide; an O-glycoside or N-glycoside glycoside of which being selected from monosaccharides or disaccharides; or a methylenesulfonate group; R²¹, R²², R²³, R²⁴, R²⁶, R²⁷, R²⁸ and R²⁹ which may be the same or different represent hydrogen; halogen; a hydroxy group; a nitroso group; a nitro group; an alkoxy group; a straight chain or branched C₁ to C₁₈ alkyl group substituted with one or more hydroxy and/or amino groups if necessary; a substituted or unsubstituted aryl group having one or more heteroatoms if necessary; a substituted or unsubstituted aralkyl group having one or more heteroatoms if necessary; a substituted or unsubstituted aryloxy group having one or more heteroatoms if necessary; a substituted or unsubstituted methylenearyloxy group having one or more heteroatoms if necessary; a cycloalkyl group having 3 to 7 carbon atoms, and including one or more heteroatoms if necessary; a methylenecycloalkyl group having 3 to 7 carbon atoms, and including one or more heteroatoms if necessary; a trifluoromethyl group; -COM; -COOM; or a CH₂COOM group (wherein M represents hydrogen, a straight chain or branched C₁ to C₁₈-alkyl group additionally substituted with one or more hydroxy and/or amino groups if necessary, or an aryl group, which may be substituted with one or more halogen atoms, alkyl groups or alkoxy groups, having one or more heteroatoms if necessary); an -NR³⁰R³¹ group (wherein R³⁰ and R³¹ which may be the same or different represent hydrogen, a straight chain or branched C₁ to C₁₈-alkyl group additionally substituted with one or more hydroxy and/or amino groups if necessary, a substituted or unsubstituted aryl group including one or more heteroatoms if necessary, an acyl group; or form a part of cycloalkyl having 3 to 7 carbon atoms with the nitrogen atom including one or more heteroatoms if necessary); a -CONR³⁰R³¹ group (wherein R³⁰ and R³¹ have the meanings as defined above); a hydroxylamino group; a phosphate group; a phosphonate group; a sulfate group; a sulfonate group; a sulfonamide group; an -SO₂NR³⁰R³¹ group (wherein R³⁰ and R³¹ have the meanings as defined above); an -N=N-R³² azo group (wherein R³² represents an aromatic group substituted with one or more carboxyl, phosphoryl or sulfonate groups if necessary, or an O-glycoside or N-glycoside group glycoside of which being selected from monosaccharides or disaccharides); or R²⁰ and R²⁴, and R²⁵ and R²⁹ together form a ring having one to four CH₂ groups each independently substituted if necessary, respectively; Y and Z which may be the same or different represent an oxygen; sulfur; selenium; tellurium atom; an NR³³ group (wherein R³³ represents hydrogen, a straight chain or branched C₁ to C₁₈ alkyl group substituted with one or more carboxyl, phosphoryl or sulfonate groups if necessary, a substituted or unsubstituted aryl group including one or more heteroatoms if necessary, an aralkyl group or a sulfonate group); or -NOR³³ (wherein R³³ group have the meanings as defined above)], or a pharmacologically acceptable salt thereof.

34. The agent for the promotion of neuropoiesis according to claim 20 wherein the substance that inhibits GSK-3 is a compound selected from the group consisting of indirubin, 5-iodo-indirubin, 5-bromo-indirubin, 5-chloro-indirubin, 5-fluoro-indirubin, 5-methyl-indirubin, 5-nitro-indirubin, 5-SO₃H-indirubin, 5'-bromo-indirubin, 5-5'-dibromo-indirubin and 5' -bromo-indirubin 5-sulfonic acid, or a pharmacologically acceptable salt thereof.

35. The agent for the promotion of neuropoiesis according to claim 20 wherein the substance that inhibits GSK-3 is a compound selected from the group consisting of indirubin-3'-monooxime, 5-iodo-indirubin-3'-monooxime and 5-SO₃Na-indirubin-3'-monooxime, or a pharmacologically acceptable salt thereof.

36. The agent for the promotion of neuropoiesis according to claim 20 wherein the substance that inhibits GSK-3 is indirubin-3'-monooxime or a pharmacologically acceptable salt thereof.

37. A neuron obtained by culturing a neural stem cell in the presence of the agent for the promotion of neuropoiesis according to any one of claims 20 to 36.

38. A method of the manufacture of a neuron which comprises culturing a neural stem cell in the presence of the agent for the promotion of neuropoiesis according to any one of claims 20 to 36 to allow neogenesis of the neuron, and collecting the neuron from the culture.

39. A method of the regeneration of a nerve which comprises administering a substance that inhibits GSK-3.

40. Use of a substance that inhibits GSK-3 for the manufacture of a nerve regenerating drug.

41. Use of a substance that inhibits GSK-3 for the manufacture of an agent for the promotion of neuropoiesis of a neural stem cell.


SEQUENCE LISTING

<110> KYOWA HAKKO KOGYO CO., LTD.

<120>

<130> 11562WO1

<140>

<141>

<150> JP2003-114579

<151> 2003-04-18

<160> 17

<170> PatentIn Ver. 2.1

<210> 1

<211> 420

<212> PRT

<213> Homo sapiens

<400> 1

Met Ser Gly Arg Pro Arg Thr Thr Ser Phe Ala Glu Ser Cys Lys

Pro

1                5                   10                  15

Val Gln Gln Pro Ser Ala Phe Gly Ser Met Lys Val Ser Arg Asp
Lys

            20                  25                  30

Asp Gly Ser Lys Val Thr Thr Val Val Ala Thr Pro Gly Gln Gly
Pro

        35                  40                  45

Asp Arg Pro Gln Glu Val Ser Tyr Thr Asp Thr Lys Val Ile Gly
Asn

    50                  55                  60

Gly Ser Phe Gly Val Val Tyr Gln Ala Lys Leu Cys Asp Ser Gly
Glu

65                  70                  75                  80

Leu Val Ala Ile Lys Lys Val Leu Gln Asp Lys Arg Phe Lys Asn
Arg

                85                  90                  95

Glu Leu Gln Ile Met Arg Lys Leu Asp His Cys Asn Ile Val Arg
Leu

            100                 105                 110

Arg Tyr Phe Phe Tyr Ser Ser Gly Glu Lys Lys Asp Glu Val Tyr
Leu
115 120 125

Asn Leu Val Leu Asp Tyr Val Pro Glu Thr Val Tyr Arg Val Ala
Arg
130 135 140

His Tyr Ser Arg Ala Lys Gln Thr Leu Pro Val Ile Tyr Val Lys
Leu
145 150 155 160

Tyr Met Tyr Gln Leu Phe Arg Ser Leu Ala Tyr Ile His Ser Phe
Gly
165 170 175

Ile Cys His Arg Asp Ile Lys Pro Gln Asn Leu Leu Leu Asp Pro
Asp
180 185 190

Thr Ala Val Leu Lys Leu Cys Asp Phe Gly Ser Ala Lys Gln Leu
Val
195 200 205

Arg Gly Glu Pro Asn Val Ser Tyr Ile Cys Ser Arg Tyr Tyr Arg
Ala
210 215 220

Pro Glu Leu Ile Phe Gly Ala Thr Asp Tyr Thr Ser Ser Ile Asp
Val
225 230 235 240

Trp Ser Ala Gly Cys Val Leu Ala Glu Leu Leu Leu Gly Gln Pro
Ile
245 250 255

Phe Pro Gly Asp Ser Gly Val Asp Gln Leu Val Glu Ile Ile Lys
Val
260 265 270

Leu Gly Thr Pro Thr Arg Glu Gln Ile Arg Glu Met Asn Pro Asn
Tyr
275 280 285

Thr Glu Phe Lys Phe Pro Gln Ile Lys Ala His Pro Trp Thr Lys
Val
290 295 300

Phe Arg Pro Arg Thr Pro Pro Glu Ala Ile Ala Leu Cys Ser Arg

Leu

305 310 315 320

Leu Glu Tyr Thr Pro Thr Ala Arg Leu Thr Pro Leu Glu Ala Cys

Ala

325 330 335

His Ser Phe Phe Asp Glu Leu Arg Asp Pro Asn Val Lys His Pro

Asn

340 345 350

Gly Arg Asp Thr Pro Ala Leu Phe Asn Phe Thr Thr Gln Glu Leu

Ser

355 360 365

Ser Asn Pro Pro Leu Ala Thr Ile Leu Ile Pro Pro His Ala Arg

Ile

370 375 380

Gln Ala Ala Ala Ser Thr Pro Thr Asn Ala Thr Ala Ala Ser Asp

Ala

385 390 395 400

Asn Thr Gly Asp Arg Gly Gln Thr Asn Asn Ala Ala Ser Ala Ser

Ala

405 410 415

Ser Asn Ser Thr

            420

<210> 2

<211> 1260

<212> DNA

<213> Homo sapiens

<400> 2

atg tca ggg cgg ccc aga acc acc tcc ttt gcg gag agc tgc aag

ccg 48

Met Ser Gly Arg Pro Arg Thr Thr Ser Phe Ala Glu Ser Cys Lys

Pro

1 5 10 15

gtg cag cag cct tca gct ttt ggc agc atg aaa gtt agc aga gac

aag 96

Val Gln Gln Pro Ser Ala Phe Gly Ser Met Lys Val Ser Arg Asp

Lys

20 25 30

gac ggc agc aag gtg aca aca gtg gtg gca act cct ggg cag ggt

cca 144

Asp Gly Ser Lys Val Thr Thr Val Val Ala Thr Pro Gly Gln Gly
Pro
35 40 45

gac agg cca caa gaa gtc agc tat aca gac act aaa gtg att gga
aat 192
Asp Arg Pro Gln Glu Val Ser Tyr Thr Asp Thr Lys Val Ile Gly
Asn
50 55 60

gga tca ttt ggt gtg gta tat caa gcc aaa ctt tgt gat tca gga
gaa 240
Gly Ser Phe Gly Val Val Tyr Gln Ala Lys Leu Cys Asp Ser Gly
Glu
65 70 75 80

ctg gtc gcc atc aag aaa gta ttg cag gac aag aga ttt aag aat
cga 288
Leu Val Ala Ile Lys Lys Val Leu Gln Asp Lys Arg Phe Lys Asn
Arg
85 90 95

gag ctc cag atc atg aga aag cta gat cac tgt aac ata gtc cga
ttg 336
Glu Leu Gln Ile Met Arg Lys Leu Asp His Cys Asn Ile Val Arg

Leu

100 105 110

cgt tat ttc ttc tac tcc agt ggt gag aag aaa gat gag gtc tat

ctt 384

Arg Tyr Phe Phe Tyr Ser Ser Gly Glu Lys Lys Asp Glu Val Tyr

Leu

115 120 125

aat ctg gtg ctg gac tat gtt ccg gaa aca gta tac aga gtt gcc

aga 432

Asn Leu Val Leu Asp Tyr Val Pro Glu Thr Val Tyr Arg Val Ala

Arg

130 135 140

cac tat agt cga gcc aaa cag acg ctc cct gtg att tat gtc aag

ttg 480

His Tyr Ser Arg Ala Lys Gln Thr Leu Pro Val Ile Tyr Val Lys

Leu

145 150 155 160

tat atg tat cag ctg ttc cga agt tta gcc tat atc cat tcc ttt

gga 528

Tyr Met Tyr Gln Leu Phe Arg Ser Leu Ala Tyr Ile His Ser Phe

Gly

165 170 175

atc tgc cat cgg gat att aaa ccg cag aac ctc ttg ttg gat cct

gat 576

Ile Cys His Arg Asp Ile Lys Pro Gln Asn Leu Leu Leu Asp Pro

Asp

180 185 190

act gct gta tta aaa ctc tgt gac ttt gga agt gca aag cag ctg

gtc 624

Thr Ala Val Leu Lys Leu Cys Asp Phe Gly Ser Ala Lys Gln Leu

Val

195 200 205

cga gga gaa ccc aat gtt tcg tat atc tgt tct cgg tac tat agg

gca 672

Arg Gly Glu Pro Asn Val Ser Tyr Ile Cys Ser Arg Tyr Tyr Arg

Ala

210 215 220

cca gag ttg atc ttt gga gcc act gat tat acc tct agt ata gat

gta 720

Pro Glu Leu Ile Phe Gly Ala Thr Asp Tyr Thr Ser Ser Ile Asp

Val

225 230 235 240

tgg tct gct ggc tgt gtg ttg gct gag ctg tta cta gga caa cca
ata   768
Trp Ser Ala Gly Cys Val Leu Ala Glu Leu Leu Leu Gly Gln Pro
Ile
                245                 250                 255

ttt cca ggg gat agt ggt gtg gat cag ttg gta gaa ata atc aag
gtc   816
Phe Pro Gly Asp Ser Gly Val Asp Gln Leu Val Glu Ile Ile Lys
Val
            260                 265                 270

ctg gga act cca aca agg gag caa atc aga gaa atg aac cca aac
tac   864
Leu Gly Thr Pro Thr Arg Glu Gln Ile Arg Glu Met Asn Pro Asn
Tyr
        275                 280                 285

aca gaa ttt aaa ttc cct caa att aag gca cat cct tgg act aag
gtc   912
Thr Glu Phe Lys Phe Pro Gln Ile Lys Ala His Pro Trp Thr Lys
Val
    290                 295                 300

ttc cga ccc cga act cca ccg gag gca att gca ctg tgt agc cgt
ctg 960
Phe Arg Pro Arg Thr Pro Pro Glu Ala Ile Ala Leu Cys Ser Arg
Leu
305 310 315 320

ctg gag tat aca cca act gcc cga cta aca cca ctg gaa gct tgt
gca 1008
Leu Glu Tyr Thr Pro Thr Ala Arg Leu Thr Pro Leu Glu Ala Cys
Ala
325 330 335

cat tca ttt ttt gat gaa tta cgg gac cca aat gtc aaa cat cca
aat 1056
His Ser Phe Phe Asp Glu Leu Arg Asp Pro Asn Val Lys His Pro
Asn
340 345 350

ggg cga gac aca cct gca ctc ttc aac ttc acc act caa gaa ctg
tca 1104
Gly Arg Asp Thr Pro Ala Leu Phe Asn Phe Thr Thr Gln Glu Leu
Ser
355 360 365

agt aat cca cct ctg gct acc atc ctt att cct cct cat gct cgg

att 1152

Ser Asn Pro Pro Leu Ala Thr Ile Leu Ile Pro Pro His Ala Arg

Ile

370 375 380

caa gca gct gct tca acc ccc aca aat gcc aca gca gcg tca gat

gct 1200

Gln Ala Ala Ala Ser Thr Pro Thr Asn Ala Thr Ala Ala Ser Asp

Ala

385 390 395 400

aat act gga gac cgt gga cag acc aat aat gct gct tct gca tca

gct 1248

Asn Thr Gly Asp Arg Gly Gln Thr Asn Asn Ala Ala Ser Ala Ser

Ala

405 410 415

tcc aac tcc acc

1260

Ser Asn Ser Thr

            420

<210> 3

<211> 737

<212> PRT

<213> Homo sapiens

<400> 3

Met Pro Leu Asn Arg Thr Leu Ser Met Ser Ser Leu Pro Gly Leu
Glu
1               5                   10                  15

Asp Trp Glu Asp Glu Phe Asp Leu Glu Asn Ala Val Leu Phe Glu
Val
            20                  25                  30

Ala Trp Glu Val Ala Asn Lys Val Gly Gly Ile Tyr Thr Val Leu
Gln
        35                  40                  45

Thr Lys Ala Lys Val Thr Gly Asp Glu Trp Gly Asp Asn Tyr Phe
Leu
    50                  55                  60

Val Gly Pro Tyr Thr Glu Gln Gly Val Arg Thr Gln Val Glu Leu
Leu
65                  70                  75                  80

Glu Ala Pro Thr Pro Ala Leu Lys Arg Thr Leu Asp Ser Met Asn
Ser

85 90 95

Lys Gly Cys Lys Val Tyr Phe Gly Arg Trp Leu Ile Glu Gly Gly
Pro

100 105 110

Leu Val Val Leu Leu Asp Val Gly Ala Ser Ala Trp Ala Leu Glu
Arg

115 120 125

Trp Lys Gly Glu Leu Trp Asp Ile Cys Asn Ile Gly Val Pro Trp
Tyr

130 135 140

Asp Arg Glu Ala Asn Asp Ala Val Leu Phe Gly Phe Leu Thr Thr
Trp

145 150 155 160

Phe Leu Gly Glu Phe Leu Ala Gln Ser Glu Glu Lys Pro His Val
Val

165 170 175

Ala His Phe His Glu Trp Leu Ala Gly Val Gly Leu Cys Leu Cys
Arg

180 185 190

Ala Arg Arg Leu Pro Val Ala Thr Ile Phe Thr Thr His Ala Thr
Leu
195 200 205

Leu Gly Arg Tyr Leu Cys Ala Gly Ala Val Asp Phe Tyr Asn Asn
Leu
210 215 220

Glu Asn Phe Asn Val Asp Lys Glu Ala Gly Glu Arg Gln Ile Tyr
His
225 230 235 240

Arg Tyr Cys Met Glu Arg Ala Ala Ala His Cys Ala His Val Phe
Thr
245 250 255

Thr Val Ser Gln Ile Thr Ala Ile Glu Ala Gln His Leu Leu Lys
Arg
260 265 270

Lys Pro Asp Ile Val Thr Pro Asn Gly Leu Asn Val Lys Lys Phe
Ser
275 280 285

Ala Met His Glu Phe Gln Asn Leu His Ala Gln Ser Lys Ala Arg
Ile
290 295 300
Gln Glu Phe Val Arg Gly His Phe Tyr Gly His Leu Asp Phe Asn
Leu
305 310 315 320
Asp Lys Thr Leu Tyr Phe Phe Ile Ala Gly Arg Tyr Glu Phe Ser
Asn
325 330 335
Lys Gly Ala Asp Val Phe Leu Glu Ala Leu Ala Arg Leu Asn Tyr
Leu
340 345 350
Leu Arg Val Asn Gly Ser Glu Gln Thr Val Val Ala Phe Phe Ile
Met
355 360 365
Pro Ala Arg Thr Asn Asn Phe Asn Val Glu Thr Leu Lys Gly Gln
Ala
370 375 380
Val Arg Lys Gln Leu Trp Asp Thr Ala Asn Thr Val Lys Glu Lys

Phe

385                 390                 395                 400

Gly Arg Lys Leu Tyr Glu Ser Leu Leu Val Gly Ser Leu Pro Asp

Met

                405                 410                 415

Asn Lys Met Leu Asp Lys Glu Asp Phe Thr Met Met Lys Arg Ala

Ile

            420                 425                 430

Phe Ala Thr Gln Arg Gln Ser Phe Pro Pro Val Cys Thr His Asn

Met

        435                 440                 445

Leu Asp Asp Ser Ser Asp Pro Ile Leu Thr Thr Ile Arg Arg Ile

Gly

    450                 455                 460

Leu Phe Asn Ser Ser Ala Asp Arg Val Lys Val Ile Phe His Pro

Glu

465                 470                 475                 480

Phe Leu Ser Ser Thr Ser Pro Leu Leu Pro Val Asp Tyr Glu Glu

Phe

485 490 495

Val Arg Gly Cys His Leu Gly Val Phe Pro Ser Tyr Tyr Glu Pro
Trp

500 505 510

Gly Tyr Thr Pro Ala Glu Cys Thr Val Met Gly Ile Pro Ser Ile
Ser

515 520 525

Thr Asn Leu Ser Gly Phe Gly Cys Phe Met Glu Glu His Ile Ala
Asp

530 535 540

Pro Ser Ala Tyr Gly Ile Tyr Ile Leu Asp Arg Arg Phe Arg Ser
Leu

545 550 555 560

Asp Asp Ser Cys Ser Gln Leu Thr Ser Phe Leu Tyr Ser Phe Cys
Gln

565 570 575

Gln Ser Arg Arg Gln Arg Ile Ile Gln Arg Asn Arg Thr Glu Arg
Leu

580 585 590

Ser Asp Leu Leu Asp Trp Lys Tyr Leu Gly Arg Tyr Tyr Met Ser
Ala
595 600 605
Arg His Met Ala Leu Ser Lys Ala Phe Pro Glu His Phe Thr Tyr
Glu
610 615 620
Pro Asn Glu Ala Asp Ala Ala Gln Gly Tyr Arg Tyr Pro Arg Pro
Ala
625 630 635 640
Ser Val Pro Pro Ser Pro Ser Leu Ser Arg His Ser Ser Pro His
Gln
645 650 655
Ser Glu Asp Glu Glu Asp Pro Arg Asn Gly Pro Leu Glu Glu Asp
Gly
660 665 670
Glu Arg Tyr Asp Glu Asp Glu Glu Ala Ala Lys Asp Arg Arg Asn
Ile
675 680 685

Arg Ala Pro Glu Trp Pro Arg Arg Ala Ser Cys Thr Ser Ser Thr
Ser
690 695 700

Gly Arg Lys Arg Asn Ser Val Asp Thr Ala Thr Ser Ser Ser Leu
Ser
705 710 715 720

Thr Pro Ser Glu Pro Leu Ser Pro Thr Ser Ser Leu Gly Glu Glu
Arg
725 730 735

Asn

<210> 4
<211> 2211
<212> DNA
<213> Homo sapiens

<400> 4
atg cct tta aac cgc act ttg tcc atg tcc tca ctg cca gga ctg
gag 48
Met Pro Leu Asn Arg Thr Leu Ser Met Ser Ser Leu Pro Gly Leu
Glu
1 5 10 15

gac tgg gag gat gaa ttc gac ctg gag aac gca gtg ctc ttc gaa
gtg   96
Asp Trp Glu Asp Glu Phe Asp Leu Glu Asn Ala Val Leu Phe Glu
Val
            20                  25                  30

gcc tgg gag gtg gct aac aag gtg ggt ggc atc tac acg gtg ctg
cag   144
Ala Trp Glu Val Ala Asn Lys Val Gly Gly Ile Tyr Thr Val Leu
Gln
        35                  40                  45

acg aag gcg aag gtg aca ggg gac gaa tgg ggc gac aac tac ttc
ctg   192
Thr Lys Ala Lys Val Thr Gly Asp Glu Trp Gly Asp Asn Tyr Phe
Leu
    50                  55                  60

gtg ggg ccg tac acg gag cag ggc gtc agg acc cag gtg gaa ctg
ctg   240
Val Gly Pro Tyr Thr Glu Gln Gly Val Arg Thr Gln Val Glu Leu
Leu
65                  70                  75                  80

gag gcc ccc acc ccg gcc ctg aag agg aca ctg gat tcc atg aac
agc   288
Glu Ala Pro Thr Pro Ala Leu Lys Arg Thr Leu Asp Ser Met Asn
Ser
                85                  90                  95

aag ggc tgc aag gtg tat ttc ggg cgc tgg ctg atc gag gga ggc
cct   336
Lys Gly Cys Lys Val Tyr Phe Gly Arg Trp Leu Ile Glu Gly Gly
Pro
            100                 105                 110

ctg gtg gtg ctc ctg gac gtg ggt gcc tca gct tgg gcc ctg gag
cgc   384
Leu Val Val Leu Leu Asp Val Gly Ala Ser Ala Trp Ala Leu Glu
Arg
        115                 120                 125

tgg aag gga gag ctc tgg gat atc tgc aac atc gga gtg ccg tgg
tac   432
Trp Lys Gly Glu Leu Trp Asp Ile Cys Asn Ile Gly Val Pro Trp
Tyr
    130                 135                 140

gac cgc gag gcc aac gac gct gtc ctc ttt ggc ttt ctg acc acc

tgg 480

Asp Arg Glu Ala Asn Asp Ala Val Leu Phe Gly Phe Leu Thr Thr

Trp

145 150 155 160

ttc ctg ggt gag ttc ctg gca cag agt gag gag aag cca cat gtg

gtt 528

Phe Leu Gly Glu Phe Leu Ala Gln Ser Glu Glu Lys Pro His Val

Val

165 170 175

gct cac ttc cat gag tgg ttg gca ggc gtt gga ctc tgc ctg tgt

cgt 576

Ala His Phe His Glu Trp Leu Ala Gly Val Gly Leu Cys Leu Cys

Arg

180 185 190

gcc cgg cga ctg cct gta gca acc atc ttc acc acc cat gcc acg

ctg 624

Ala Arg Arg Leu Pro Val Ala Thr Ile Phe Thr Thr His Ala Thr

Leu

195 200 205

ctg ggg cgc tac ctg tgt gcc ggt gcc gtg gac ttc tac aac aac

ctg 672

Leu Gly Arg Tyr Leu Cys Ala Gly Ala Val Asp Phe Tyr Asn Asn
Leu
210 215 220

gag aac ttc aac gtg gac aag gaa gca ggg gag agg cag atc tac
cac 720
Glu Asn Phe Asn Val Asp Lys Glu Ala Gly Glu Arg Gln Ile Tyr
His
225 230 235 240

cga tac tgc atg gaa agg gcg gca gcc cac tgc gct cac gtc ttc
act 768
Arg Tyr Cys Met Glu Arg Ala Ala Ala His Cys Ala His Val Phe
Thr
245 250 255

act gtg tcc cag atc acc gcc atc gag gca cag cac ttg ctc aag
agg 816
Thr Val Ser Gln Ile Thr Ala Ile Glu Ala Gln His Leu Leu Lys
Arg
260 265 270

aaa cca gat att gtg acc ccc aat ggg ctg aat gtg aag aag ttt
tct 864
Lys Pro Asp Ile Val Thr Pro Asn Gly Leu Asn Val Lys Lys Phe

Ser

275 280 285

gcc atg cat gag ttc cag aac ctc cat gct cag agc aag gct cga

atc 912

Ala Met His Glu Phe Gln Asn Leu His Ala Gln Ser Lys Ala Arg

Ile

290 295 300

cag gag ttt gtg cgg ggc cat ttt tat ggg cat ctg gac ttc aac

ttg 960

Gln Glu Phe Val Arg Gly His Phe Tyr Gly His Leu Asp Phe Asn

Leu

305 310 315 320

gac aag acc tta tac ttc ttt atc gcc ggc cgc tat gag ttc tcc

aac 1008

Asp Lys Thr Leu Tyr Phe Phe Ile Ala Gly Arg Tyr Glu Phe Ser

Asn

325 330 335

aag ggt gct gac gtc ttt ctg gag gca ttg gct cgg ctc aac tat

ctg 1056

Lys Gly Ala Asp Val Phe Leu Glu Ala Leu Ala Arg Leu Asn Tyr

Leu

340 345 350

ctc aga gtg aac ggc agc gag cag aca gtg gtt gcc ttc ttc atc
atg 1104
Leu Arg Val Asn Gly Ser Glu Gln Thr Val Val Ala Phe Phe Ile
Met
355 360 365

cca gcg cgg acc aac aat ttc aac gtg gaa acc ctc aaa ggc caa
gct 1152
Pro Ala Arg Thr Asn Asn Phe Asn Val Glu Thr Leu Lys Gly Gln
Ala
370 375 380

gtg cgc aaa cag ctt tgg gac acg gcc aac acg gtg aag gaa aag
ttc 1200
Val Arg Lys Gln Leu Trp Asp Thr Ala Asn Thr Val Lys Glu Lys
Phe
385 390 395 400

ggg agg aag ctt tat gaa tcc tta ctg gtt ggg agc ctt ccc gac
atg 1248
Gly Arg Lys Leu Tyr Glu Ser Leu Leu Val Gly Ser Leu Pro Asp
Met
405 410 415

aac aag atg ctg gat aag gaa gac ttc act atg atg aag aga gcc
atc   1296
Asn Lys Met Leu Asp Lys Glu Asp Phe Thr Met Met Lys Arg Ala
Ile
            420                 425                 430

ttt gca acg cag cgg cag tct ttc ccc cct gtg tgc acc cac aat
atg   1344
Phe Ala Thr Gln Arg Gln Ser Phe Pro Pro Val Cys Thr His Asn
Met
        435                 440                 445

ctg gat gac tcc tca gac ccc atc ctg acc acc atc cgc cga atc
ggc   1392
Leu Asp Asp Ser Ser Asp Pro Ile Leu Thr Thr Ile Arg Arg Ile
Gly
    450                 455                 460

ctc ttc aat agc agt gcc gac agg gtg aag gtg att ttc cac ccg
gag   1440
Leu Phe Asn Ser Ser Ala Asp Arg Val Lys Val Ile Phe His Pro
Glu
465                 470                 475                 480

ttc ctc tcc tcc aca agc ccc ctg ctc cct gtg gac tat gag gag
ttt 1488
Phe Leu Ser Ser Thr Ser Pro Leu Leu Pro Val Asp Tyr Glu Glu
Phe
485 490 495

gtc cgt ggc tgt cac ctt gga gtc ttc ccc tcc tac tat gag cct
tgg 1536
Val Arg Gly Cys His Leu Gly Val Phe Pro Ser Tyr Tyr Glu Pro
Trp
500 505 510

ggc tac aca ccg gct gag tgc acg gtt atg gga atc ccc agt atc
tcc 1584
Gly Tyr Thr Pro Ala Glu Cys Thr Val Met Gly Ile Pro Ser Ile
Ser
515 520 525

acc aat ctc tcc ggc ttc ggc tgc ttc atg gag gaa cac atc gca
gac 1632
Thr Asn Leu Ser Gly Phe Gly Cys Phe Met Glu Glu His Ile Ala
Asp
530 535 540

ccc tca gct tac ggt atc tac att ctt gac cgg cgg ttc cgc agc

ctg 1680

Pro Ser Ala Tyr Gly Ile Tyr Ile Leu Asp Arg Arg Phe Arg Ser

Leu

545 550 555 560

gat gat tcc tgc tcg cag ctc acc tcc ttc ctc tac agt ttc tgt

cag 1728

Asp Asp Ser Cys Ser Gln Leu Thr Ser Phe Leu Tyr Ser Phe Cys

Gln

565 570 575

cag agc cgg cgg cag cgt atc atc cag cgg aac cgc acg gag cgc

ctc 1776

Gln Ser Arg Arg Gln Arg Ile Ile Gln Arg Asn Arg Thr Glu Arg

Leu

580 585 590

tcc gac ctt ctg gac tgg aaa tac cta ggc cgg tac tat atg tct

gcg 1824

Ser Asp Leu Leu Asp Trp Lys Tyr Leu Gly Arg Tyr Tyr Met Ser

Ala

595 600 605

cgc cac atg gcg ctg tcc aag gcc ttt cca gag cac ttc acc tac

gag 1872

Arg His Met Ala Leu Ser Lys Ala Phe Pro Glu His Phe Thr Tyr
Glu
610 615 620

ccc aac gag gcg gat gcg gcc cag ggg tac cgc tac cca cgg cca
gcc 1920
Pro Asn Glu Ala Asp Ala Ala Gln Gly Tyr Arg Tyr Pro Arg Pro
Ala
625 630 635 640

tcg gtg cca ccg tcg ccc tcg ctg tca cga cac tcc agc ccg cac
cag 1968
Ser Val Pro Pro Ser Pro Ser Leu Ser Arg His Ser Ser Pro His
Gln
645 650 655

agt gag gac gag gag gat ccc cgg aac ggg ccg ctg gag gaa gac
ggc 2016
Ser Glu Asp Glu Glu Asp Pro Arg Asn Gly Pro Leu Glu Glu Asp
Gly
660 665 670

gag cgc tac gat gag gac gag gag gcc gcc aag gac cgg cgc aac
atc 2064
Glu Arg Tyr Asp Glu Asp Glu Glu Ala Ala Lys Asp Arg Arg Asn

Ile

675 680 685

cgt gca cca gag tgg ccg cgc cga gcg tcc tgc acc tcc tcc acc

agc 2112

Arg Ala Pro Glu Trp Pro Arg Arg Ala Ser Cys Thr Ser Ser Thr

Ser

690 695 700

ggc cgc aag cgc aac tct gtg gac acg gcc acc tcc agc tca ctc

agc 2160

Gly Arg Lys Arg Asn Ser Val Asp Thr Ala Thr Ser Ser Ser Leu

Ser

705 710 715 720

acc ccg agc gag ccc ctc agc ccc acc agc tcc ctg ggc gag gag

cgt 2208

Thr Pro Ser Glu Pro Leu Ser Pro Thr Ser Ser Leu Gly Glu Glu

Arg

725 730 735

aac

2211

Asn

<210> 5

<211> 60

<212> PRT

<213> Artificial Sequence

<223> Description of Artificial Sequence: Synthetic PRT

<400> 5

Tyr Arg Arg Ala Ala Val Pro Pro Ser Pro Ser Leu Ser Arg His

Ser

1 5 10 15

Ser Pro His Gln Ser Glu Asp Glu Glu Glu

            20                  25

<210> 6

<211> 24

<212> DNA

<213> Artificial Sequence

<223> Description of Artificial Sequence: synthetic DNA

<400> 6

agggtatgat aaccgggaga tcgt

24

<210> 7

<211> 24

<212> DNA

<213> Artificial Sequence

<223> Description of Artificial Sequence: synthetic DNA

<400> 7

gggccatata gttccacaaa ggca

24

<210> 8

<211> 24

<212> DNA

<213> Artificial Sequence

<223> Description of Artificial Sequence: synthetic DNA

<400> 8

caaaaggcac tggaactcgc aatg

24

<210> 9

<211> 24

<212> DNA
<213> Artificial Sequence
<223> Description of Artificial Sequence:synthetic DNA
<400> 9
ttcttggcaa cggcaacaaa ccac
24
<210> 10
<211> 24
<212> DNA
<213> Artificial Sequence
<223> Description of Artificial Sequence: synthetic DNA
<400> 10
ggtgaatcga gaagagccat catg
24
<210> 11
<211> 24
<212> DNA
<213> Artificial Sequence

<223> Description of Artificial Sequence: synthetic DNA

<400> 11
ttcaggtaga gttggaggct gatg
24

<210> 12
<211> 25
<212> DNA
<213> Artificial Sequence

<223> Description of Artificial Sequence: synthetic DNA

<400> 12
gaagaacttt cctgatggcc accag
25

<210> 13
<211> 25
<212> DNA
<213> Artificial Sequence

<223> Description of Artificial Sequence: synthetic DNA

<400> 13

ctggtggcca tcaggaaagt tcttc
25

<210> 14
<211> 21
<212> RNA
<213> Artificial Sequence

<223> Description of Artificial Sequence: synthetic RNA

<400> 14
gucaguuaca cagacacuat t 21

<210> 15
<211> 21
<212> RNA
<213> Artificial Sequence

<223> Description of Artificial Sequence: synthetic RNA

<400> 15
uagugucugu guaacugact t 21

<210> 16
<211> 21

<212> RNA
<213> Artificial Sequence

<223> Description of Artificial Sequence: synthetic RNA

<400> 16
gucuagccua uauccauuct t                                                21

<210> 17
<211> 21
<212> RNA
<213> Artificial Sequence

<223> Description of Artificial Sequence: synthetic RNA

<400> 17
gaauggauau aggcuagact t                                                21